(19) Europäisches Patentamt
European Patent Office
Office européen des brevets

(11) Veröffentlichungsnummer : **0 094 080**
**B1**

(12) # EUROPÄISCHE PATENTSCHRIFT

(45) Veröffentlichungstag der Patentschrift : **09.09.87**

(51) Int. Cl.⁴ : **C 07 D471/04**, A 61 K 31/505 // (C07D471/04, 239:00, 221:00)

(21) Anmeldenummer : **83104559.6**

(22) Anmeldetag : **09.05.83**

(54) **Pyrido(2,1-b)chinazolinderivate.**

(30) Priorität : **10.05.82 US 376324**
**02.03.83 US 471382**

(43) Veröffentlichungstag der Anmeldung : **16.11.83 Patentblatt 83/46**

(45) Bekanntmachung des Hinweises auf die Patenterteilung : **09.09.87 Patentblatt 87/37**

(84) Benannte Vertragsstaaten : **AT BE CH DE FR GB IT LI LU NL SE**

(56) Entgegenhaltungen :
**FR-A- 2 384 771**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**

**CH-4002 Basel (CH)**

(72) Erfinder : **Tilley, Jefferson Wright**
**19 Evergreen Drive**
**North Caldwell N.J. 07006 (US)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 094 080 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Verbindungen der allgemeinen Formel

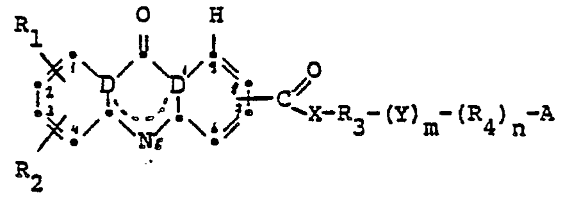 (I)

worin eines von D un D' ein Stickstoffatom und das andere ein Kohlenstoffatom, die gestrichelte Linie zwei zusätzliche Bindungen, $R_1$ und $R_2$ unabhängig von einander Wasserstoff, $(C_{1-7})$-Alkyl, $(C_{1-7})$-Alkoxy, Hydroxy oder Halogen, X —O— oder —$N(R_5)$—, $R_5$ Wasserstoff oder $(C_{1-7})$-Alkyl, $R_3$ $(C_{2-7})$-Alkylen, $R_4$ $(C_{1-7})$-Alkylen, Y —O— oder —S—, m 0 oder 1, n 0 oder 1 und A eine unsubstituierte oder durch $(C_{1-7})$-Alkyl, $(C_{1-7})$-Alkoxy, Halogen oder Nitro substituierte, aromatische, 5- oder 6-gliedrige heterocyclische Gruppe bedeuten, mit der Massgabe, dass m und n beide 0 sind, wenn A über ein heterocyclisches Stickstoffatom gebunden ist, und pharmazeutisch annehmbare Säureadditionssalze davon.

Die Verbindungen der Formel I und ihre pharmazeutisch annehmbaren Säureadditionssalze besitzen wertvolle pharmakologische Eigenschaften und können insbesondere für die Behandlung von Allergien und thrombotischen Gefässerkrankungen verwendet werden.

Gegenstand der vorliegenden Erfindung sind die Verbindungen der Formel I und ihre pharmazeutisch annehmbaren Säureadditionssalze als solche und als pharmazeutische Wirkstoffe ; ein Verfahren zur Herstellung dieser Produkte und Zwischenprodukte zu deren Herstellung ; pharmazeutische Präparate, enthaltend diese Stoffe, und ein Verfahren zur Herstellung solcher Präparate ; sowie die Verwendung besagter Produkte bei der Behandlung oder Verhütung von Krankheiten.

Aus FR-A-2384 770 und FR-A-2384 771 sind Pyrido [2,1-b] chinazolinderivate sowie deren antiallergische Eigenschaften bekannt. Diese Derivate besitzen in dei Seitenkette keinen über eine Alkylenkette gebundenen aromatischen Heterocyclus.

Der Ausdruck « $(C_{1-7})$-Alkyl », wie er hier verwendet wird, bezeichnet geradkettige oder verzweigte, gesättigte Kohlenwasserstoffreste mit 1-7 Kohlenstoffatomen, z. B. Methyl, Aethyl, Propyl, Isopropyl, Butyl, t-Butyl, Neopentyl, Pentyl, Heptyl und dergleichen. Der Ausdruck « $(C_{1-7})$-bzw. $(C_{2-7})$-Alkylen » bezeichnet geradkettige oder verzweigte, gesättigte Kohlenwasserstoffreste mit bis zu 7, vorzugsweise 4-6 Kohlenstoffatomen, wie Aethylen, Propylen, Butylen, Pentylen, 1-Methylpropylen, 1-Methylbutylen oder dergleichen. Der Ausdruck « $(C_{1-7})$-Alkoxy » bezeichnet einen Alkylätherrest, in welcher die $(C_{1-7})$-Alkylgruppe wie oben beschrieben ist, z. B. Methoxy, Aethoxy, Propoxy, Pentoxy und dergleichen. Der Ausdruck « Halogen » bezeichnet die Halogene Brom, Chlor, Fluor und Jod.

Der Ausdruck « heterocyclische Gruppe » bezeichnet eine heterocyclische Gruppe, welche gegebenenfalls mit einem anderen 6-gliedrigen heterocyclischen oder nicht-heterocyclischen Ring kondensiert ist, im speziellen eine heterocyclische Gruppe, welche 1-3, oder insbesondere 1 oder 2 Heteroatome, welche gleich oder verschieden sein können, enthält. Bevorzugte Heteroatome sind Stickstoff, Sauerstoff und Schwefel. Eine substituierte heterocyclische Gruppe, kann durch eine oder mehrere, vorzugsweise durch eine oder zwei Substituenten, welche gleich oder verschieden sein können, substituiert sein. Die Substituenten sind geradkettiges oder verzweigtes niederes Alkyl, im speziellen solche mit 1 oder 2 Kohlenstoffatomen ; geradkettiges oder verzweigtes niederes Alkoxy, im speziellen solche mit 1 oder 2 Kohlenstoffatomen ; Halogen und Nitro.

Bevorzugte heterocyclische Gruppen sind Pyridyl, Pyrimidinyl, Imidazolyl, Pyrrolyl, Furyl, Thienyl, Thiazolyl, Oxazolyl, Isoxazolyl, Pyrazinyl, Chinolinyl, Isochinolinyl, Chinazolinyl, 1,2,4-Triazinyl, Benzimidazolyl und Pyridazinyl. Diese Ringe können, wie unten dargestellt, über ein Kohlenstoff- oder Stickstoffatom gebunden sein :

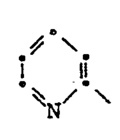 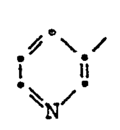 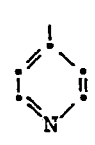 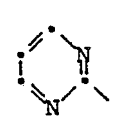 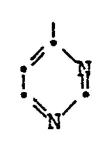 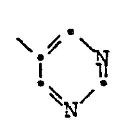

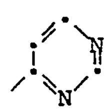 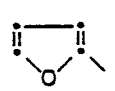 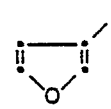 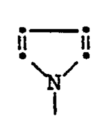 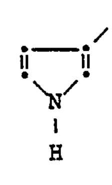 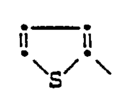

(Fortsetzung)

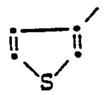 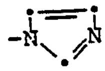 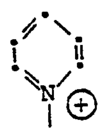 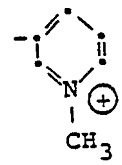

In einem Aspekt betrifft die vorliegende Erfindung Verbindungen der Formel I, worin D ein Kohlenstoffatom, D' ein Stickstoffatom und die gestrichelte Linie zwei zusätzliche Bindungen bedeuten, nämlich Verbindungen der allgemeinen Formel

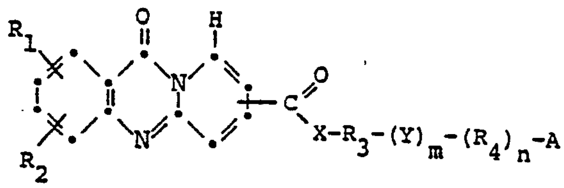
(Ia)

worin $R_1$, $R_2$, $R_3$, $R_4$, A, X, Y, m und n obige Bedeutung besitzen, und pharmazeutisch annehmbare Säureadditionssalze davon.

In einem anderen Aspekt betrifft die vorliegende Erfindung Verbindungen der Formel I, worin D ein Stickstoffatom, D' ein Kohlenstoffatom und die gestrichelte Linie zwei zusätzliche Bindungen bedeuten, nämlich Verbindungen der allgemeinen Formel

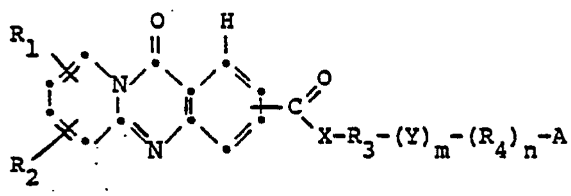
(Ib)

worin $R_1$, $R_2$, $R_3$, $R_4$, A, X, Y, m und n obige Bedeutung besitzen, und pharmazeutisch annehmbare Säureadditionssalze davon.

Bevorzugte Verbindungen der Formel I sind solche, worin sich $R_1$ und $R_2$ in der 2- bzw. 3-Stellung und die Gruppe $—CO—X—R_3\text{-}(Y)_m\text{-}(R_4)_n\text{-}A$ sich in der 8-Stellung befinden. Die bevorzugte Bedeutung von X ist $—N(R_5)—$. Vorzugsweise bedeutet $R_3$ $(C_{4\text{-}6})$-Alkylen und m und n sind 0. A ist vorzugsweise 3-Pyridyl, 4-Pyridyl oder Imidazol-1-yl. Vorzugsweise ist $R_1$ $(C_{1\text{-}7})$-Alkyl und $R_2$ Wasserstoff.

In einem weiteren Aspekt betrifft die vorliegende Erfindung Verbindungen der Formel Ia, worin $R_1$ Hydroxy bedeutet.

Die am meisten bevorzugten Verbindungen der Formel Ia sind :

N-[4-(1H-Imidazol-1-yl) butyl]-2-(1-methyläthyl)-11-oxo-11H-pyrido [2,1-b] chinazolin-8-carboxamid,
2-(1-Methyläthyl)-N-[6-(3-pyridyl) hexyl]-11-oxo-11H-pyrido [2,1-b] chinazolin-8-carboxamid und
2-(1-Methyläthyl)-N-[4-(3-pyridyl) butyl]-11-oxo-11H-pyrido [2,1-b] chinazolin-8-carboxamid.

Die am meisten bevorzugte Verbindung der Formel Ib ist 8-(1-Methyläthyl)-N-[4-(3-pyridyl)-butyl]-11-oxo-11H-pyrido [2,1-b] chinazolin-2-carboxamid.

Beispiele von Verbindungen der Formel Ia gemäss vorliegender Erfindung sind :

N-[4-(4-Pyridyl) butyl-2-(1-methyläthyl)-11-oxo-11H-pyrido [2,1-b] chinazolin-8-carboxamid,
N-[2-(2-Pyridyl) äthyl]-2-(1-methyläthyl)-11-oxo-11H-pyrido [2,1-b] chinazolin-8-carboxamid,
N-[2-(3-Pyridyl) äthyl]-2-(1-methyläthyl)-11-oxo-11H-pyrido [2,1-b] chinazolin-8-carboxamid,
N-[3-(3-Pyridyl) propyl]-2-(1-methyläthyl)-11-oxo-11H-pyrido [2,1-b] chinazolin-8-carboxamid,
N-[5-(1H-Imidazol-1-yl) pentyl]-2-(1-methyläthyl)-11-oxo-11H-pyrido [2,1-b] chinazolin-8-carboxamid,
N-[3-(1H-Imidazol-1-yl) propyl]-2-(1-methyläthyl)-11-oxo-11H-pyrido [2,1-b] chinazolin-8-carboxamid,
N-Methyl-2-(1-methyläthyl)-N-[4-(3-pyridyl) butyl]-11-oxo-11H-pyrido [2,1-b] chinazolin-8-carboxamid,
2-(1-Methyläthyl)-11-oxo-11H-pyrido [2,1-b] chinazolin-8-carbonsäure-3-(3-pyridinyl) propylester,
N-[2-(4-Pyridylthio) äthyl]-2-(1-methyläthyl)-11-oxo-11H-pyrido [2,1-b] chinazolin-8-carboxamid,
2-(1-Methyläthyl)-N-[4-(3-pyridyloxy) butyl]-11-oxo-11H-pyrido [2,1-b] chinazolin-8-carboxamid,
N-[5-(3-Pyridyl) pentyl]-2-(1-methyläthyl)-11-oxo-11H-pyrido [2,1-b] chinazolin-8-carboxamid,
2-(1-Methyläthyl)-N-[4-(4-pyridylthio) butyl]-11-oxo-11H-pyrido [2,1-b] chinazolin-8-carboxamid,
2-Methoxy-N-[4-(3-pyridyl) butyl]-11-oxo-11H-pyrido [2,1-b] chinazolin-8-carboxamid,

2-(1-Methyläthyl)-N-[4-(3-pyridyl) butyl]-11-oxo-11H-pyrido [2,1-b] chinazolin-8-carboxamid,
2-Methyl-N-[4-(3-pyridyl) butyl]-11-oxo-11H-pyrido [2,1-b] chinazolin-8-carboxamid,
N-[5-(4-Pyridyl) pentyl]-2-(1-methyläthyl)-11-oxo-11H-pyrido [2,1-b] chinazolin-8-carboxamid,
2-(1-Methyläthyl)-N-[4-(2-pyridyl) butyl]-11-oxo-11H-pyrido [2,1-b] chinazolin-8-carboxamid,
2-(1-Methyläthyl)-N-[7-(3-pyridyl) heptyl]-11-oxo-11H-pyrido [2,1-b] chinazolin-8-carboxamid,
2-(1-Methyläthyl)-N-[2-[[(4-pyridyl) methyl] thio] äthyl]-11-oxo-11H-pyrido [2,1-b] chinazolin-8-carboxamid,
2-(1-Methyläthyl)-N-[2-[[(3-pyridyl) methyl] thio] äthyl]-11-oxo-11H-pyrido [2,1-b] chinazolin-8-carboxamid,
N-[6-(1H-Imidazol-1-yl) hexyl]-2-(1-methyläthyl)-11-oxo-11H-pyrido [2,1-b] chinazolin-8-carboxamid,
N-[4-(2-Methyl-1H-imidazol-1-yl) butyl]-2-(1-methyläthyl)-11-oxo-11H-pyrido [2,1-b] chinazolin-8-carboxamid,
N-[4-(3-Pyridyl) butyl]-11-oxo-11H-pyrido [2,1-b] chinazolin-8-carboxamid,
2-(1-Methyläthyl)-N-[4-(3-pyridyl) butyl]-11-oxo-11H-pyrido [2,1-b] chinazolin-6-carboxamid,
2-Hydroxy-N-[4-(3-pyridyl) butyl]-11-oxo-11H-pyrido [2,1-b] chinazolin-8-carboxamid,
2-Brom-N-[4-(3-pyridyl) butyl]-11-oxo-11H-pyrido [2,1-b] chinazolin-8-carboxamid,
3-Chlor-N-[4-(3-pyridyl) butyl]-11-oxo-11H-pyrido [2,1-b] chinazolin-8-carboxamid,
3-Methoxy-N-[4-(3-pyridyl) butyl]-11-oxo-11H-pyrido [2,1-b] chinazolin-8-carboxamid,
2,3-Dimethyl-N-[4-(3-pyridyl) butyl]-11-oxo-11H-pyrido [2,1-b] chinazolin-8-carboxamid,
2-(1-Methyläthyl)-N-[1-methyl-3-(3-pyridyl) propyl]-11-oxo-11H-pyrido [2,1-b] chinazolin-8-carboxamid,
2-(1-Methyläthyl)-N-[4-(5-pyrimidinyl) butyl]-11-oxo-11H-pyrido [2,1-b] chinazolin-8-carboxamid,
2-(1-Methyläthyl)-N-[2-(2-pyridylthio) äthyl]-11-oxo-11H-pyrido [2,1-b] chinazolin-8-carboxamid,
2-(1-Methyläthyl)-N-[4-(4-isochinolyl) butyl]-11-oxo-11H-pyrido [2,1-b] chinazolin-8-carboxamid,
2-(1-Methyläthyl)-N-[6-(3-chinolyl) hexyl]-11-oxo-11H-pyrido [2,1-b] chinazolin-8-carboxamid,
2-(1-Methyläthyl)-N-[6-(3-thienyl) hexyl]-11-oxo-11H-pyrido [2,1-b] chinazolin-8-carboxamid,
2-(1-Methyläthyl)-N-[4-(5-oxazolyl) butyl]-11-oxo-11H-pyrido [2,1-b] chinazolin-8-carboxamid,
2-(1-Methyläthyl)-N-[4-(4-thiazolyl) butyl]-11-oxo-11H-pyrido [2,1-b] chinazolin-8-carboxamid,
2-Methoxy-N-[6-(3-pyridyl) hexyl]-11-oxo-11H-pyrido [2,1-b] chinazolin-6-carboxamid,
2-Methoxy-N-[4-(3-pyridyl) butyl]-11-oxo-11H-pyrido [2,1-b] chinazolin-6-carboxamid,
2-(1-Methyläthyl)-N-[4-(3-pyridyl) butyl]-11-oxo-11H-pyrido [2,1-b] chinazolin-7-carboxamid,
2-(1-Methyläthyl)-N-[4-(1H-imidazol-1-yl) butyl]-11-oxo-11H-pyrido [2,1-b] chinazolin-7-carboxamid,
2-Methoxy-N-[4-(1H-imidazol-1-yl) butyl]-11-oxo-11H-pyrido [2,1-b] chinazolin-7-carboxamid,
2-(1-Methyläthyl)-N-[4-(1H-1,2,4-triazol-1-yl) butyl]-11-oxo-11H-pyrido [2,1-b] chinazolin-8-carboxamid,
2-(1-Methyläthyl)-N-[2-(4-pyrimidinylthio) äthyl]-11-oxo-11H-pyrido [2,1-b] chinazolin-8-carboxamid,
2-Hydroxy-N-[6-(3-pyridyl) hexyl]-11-oxo-11H-pyrido [2,1-b] chinazolin-8-carboxamid,
2-Hydroxy-N-[4-(1H-imidazol-1-yl) butyl]-11-oxo-11H-pyrido [2,1-b] chinazolin-8-carboxamid,
4-Methoxy-N-[4-(1H-imidazol-1-yl) butyl]-11-oxo-11H-pyrido [2,1-b] chinazolin-8-carboxamid,
4-Chlor-N-[4-(1H-imidazol-1-yl) butyl]-11-oxo-11H-pyrido [2,1-b] chinazolin-8-carboxamid,
4-Methyl-N-[4-(1H-imidazol-1-yl) butyl]-11-oxo-11H-pyrido [2,1-b] chinazolin-8-carboxamid,
2-Butyl-N-[4-(1H-imidazol-1-yl) butyl]-11-oxo-11H-pyrido [2,1-b] chinazolin-8-carboxamid,
2-(1-Methyläthyl)-11-oxo-11H-pyrido [2,1-b] chinazolin-8-carbonsäure-4-(3-pyridinyl) butylester,
2-(1-Methyläthoxy)-N-[6-(3-pyridyl) hexyl]-11-oxo-11H-pyrido [2,1-b] chinazolin-8-carboxamid,
2-(1-Methyläthoxy)-N-[6-(5-pyrimidinyl) hexyl]-11-oxo-11H-pyrido [2,1-b] chinazolin-8-carboxamid,
2,3-Dimethyl-N-[6-(3-pyridyl) hexyl]-11-oxo-11H-pyrido [2,1-b] chinazolin-8-carboxamid,
2,3-Dimethyl-N-[4-(5-pyrimidinyl) butyl]-11-oxo-11H-pyrido [2,1-b] chinazolin-8-carboxamid,
2-(1-Methyläthyl)-N-[1-methyl-3-(5-pyrimidinyl) propyl]-11-oxo-11H-pyrido [2,1-b] chinazolin-8-carboxamid,
2-(1-Methyläthyl)-N-[1-methyl-4-(3-pyridyl) butyl]-11-oxo-11H-pyrido [2,1-b] chinazolin-8-carboxamid,
2-(1-Methyläthyl)-N-[1-methyl-3-(3-pyridyl) propyl]-11-oxo-11H-pyrido [2,1-b] chinazolin-8-carboxamid,
2-(1-Methyläthyl)-N-methyl-N-[4-(1H-imidazolyl-1-yl)-butyl]-11-oxo-11H-pyrido [2,1-b] chinazolin-8-carboxamid,
2-(1-Methyläthyl)-N-[1-methyl-4-(1H-imidazol-1-yl)-butyl]-11-oxo-11H-pyrido [2,1-b] chinazolin-8-carboxamid,
2-(1-Methyläthyl)-N-methyl-N-[1-methyl-4-(1H-imidazol-1-yl) butyl]-11-oxo-11H-pyrido [2,1-b] chinazolin-8-carboxamid,
1-[3-[[(2,3-Dimethyl-11-oxo-11H-pyrido [2,1-b] chinazolin-8-yl) carbonyl] amino] propyl] pyridiniumchlorid,
1-[4-[[(2,3-Dimethyl-11-oxo-11H-pyrido [2,1-b] chinazolin-8-yl) carbonyl] amino] butyl] pyridiniumchlorid und
3-[4-[[(2,3-Dimethyl-11-oxo-11H-pyrido [2,1-b] chinazolin-8-yl) carbonyl] amino] butyl]-1-methyl-pyridiniumjodid.

Beispiele von erfindungsgemässen Verbindungen der Formel Ib sind :

8-Methyl-N-[6-(4-pyridyl) hexy]-11-oxo-11H-pyrido [2,1-b] chinazolin-2-carboxamid,
8-(1-Methyläthyl)-11-oxo-11H-pyrido [2,1-b] chinazolin-2-carbonsäure-3-(3-pyridyl) propylester,
8-Chlor-N-[4-(1H-imidazol-1-yl) butyl]-11-oxo-11H-pyrido [2,1-b] chinazolin-2-carboxamid,
8-Chlor-11-oxo-11H-pyrido [2,1-b] chinazolin-2-carbonsäure-3-(3-pyridyl) propylester,
N-[4-(1H-Imidazol-1-yl) butyl]-8-(1-methyläthyl)-11-oxo-11H-pyrido [2,1-b] chinazolin-2-carboxamid und
8-(1-Methyläthyl)-N-[6-(3-pyridyl) hexyl]-11-oxo-11H-pyrido [2,1-b] chinazolin-2-carboxamid.

Erfindungsgemäss können die verbindungen der obigen Formel I und ihre pharmazeutisch annehmbaren Säureadditionssalze dadurch hergestellt werden, dass man eine Carbonsäure der allgemeinen Formel

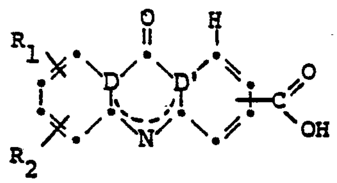

(II)

worin $R_1$, $R_2$, D, D' und die gestrichelte linie obige Bedeutung besitzen,
oder ein reaktives Derivat davon mit einer Verbindung der allgemeinen Formel

$$W—R_3—(Y)_m—(R_4)_n—A \qquad (VI)$$

worin W die Gruppe HX— oder, falls man die freie Carbonsäure der Formel II verwendet, auch eine Abgangsgruppe bedeutet, und $R_3$, $R_4$, X, Y, A, m und n obige Bedeutung besitzen, umsetzt und erwünschtenfalls eine erhaltene Verbindung der Formel I in ein pharmazeutisch annehmbares Säureadditionssalz überführt.

Die Amide und Ester der Formel I können nach an sich bekannten Methoden hergestellt werden.

Im speziellen können die Amide und Ester der Formel I hergestellt werden, indem man eine Verbindung der allgemeinen Formel

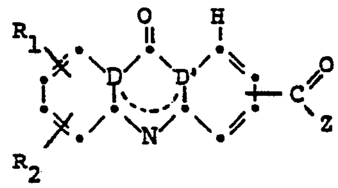

(III)

worin $R_1$, $R_2$, D, D' und die gestrichelte Linie obige Bedeutung besitzen, und Z eine Abgangsgruppe bedeutet,
mit einer Verbindung der allgemeinen Formel

$$HX—R_3—(Y)_m—(R_4)_n—A \qquad (IV)$$

worin $R_3$, $R_4$, $R_5$, A, X, Y, m und n obige Bedeutung besitzen, umsetzt. Die Abgangsgruppe Z kann ein Halogenatom oder, wenn X —$N(R_5)$— bedeutet, auch Cyanomethoxy, Phenyloxy, substituiertes Phenyloxy oder dergleichen sein.

Ist Z ein Halogenatom, so kann die Reaktion mit oder ohne Lösungsmittel durchgeführt werden. Aprotische lösungsmittel, wie Dimethylformamid, Hexamethylphosphoramid, N-Methylpyrrolidinon, Acetonitril, Toluol oder dergleichen, sind Beispiele von Lösungsmitteln welche verwendet werden können. Die Reaktion wird vorzugsweise in einem Temperaturbereich von 0 °C bis zur Rückflusstemperatur der Reaktionsmischung durchgeführt.

Bedeutet Z Cyanomethoxy, Phenyloxy oder dergleichen, so kann die Reaktion in einem polaren, aprotischen Lösungsmittel, wie Dimethylformamid, Hexamethylphosphoramid, N-Methylpyrrolidinon oder dergleichen, bei einer Reaktionstemperatur im Bereich von Raumtemperatur bis 100 °C durchgeführt werden.

Die erhaltenen Verbindungen der Formel I können unter Verwendung von herkömmlichen Methoden isoliert werden, beispielsweise durch Kristallisieren, Chromatographieren oder dergleichen.

Die Verbindungen der Formel I können erfindungsgemäss auch dadurch hergestellt werden, dass man eine Verbindung der Formel II in Gegenwart eines Kondensationsmittels, wie Diphenylphosphorylazid, mit einer Verbindung der Formel IV umsetzt.

Verwendet man Diphenylphosphorylazid, so kann die Reaktion in einem polaren, aprotischen Lösungsmittel, wie Dimethylformamid, Hexamethylphosphoramid, N-Methylpyrrolidinon oder dergleichen, bei einer Temperatur in einem Bereich von 0 °C bis Raumtemperatur durchgeführt werden. Die Reaktion wird dabei in Gegenwart eines Protonen-Akkzeptors, d. h. einer base, wie z. B. einem Trialkylamin, wie Triäthylamin oder dergleichen, durchgeführt. Die erhaltene Verbindung der Formel I kann mittels herkömmlicher methoden isoliert werden, beispielsweise durch Kristallisieren, Chromatographieren oder dergleichen.

Die Ester der Formel I, d. h. Verbindungen der Formel I, worin X —O— bedeutet, können auch hergestellt werden, indem man eine Verbindung der Formel II in einem polaren, aprotischen Lösungsmittel, wie Dimethylformamid, Hexamethylphosphoramid, Acetonitril oder dergleichen, bei einer Reaktionstemperatur in einem Bereich von 0 °C bis 100 °C und in Gegenwart einer Base, wie einem Alkalimetallcarbonat, mit einer Verbindung der allgemeinen Formel

$$Z'—R_3—(Y)_m—(R_4)_n—A \qquad (V)$$

worin $R_3$, $R_4$, A, Y, m und n obige Bedeutung besitzen und Z' eine Abgangsgruppe bedeutet, umsetzt. Die erhaltene Verbindung der Formel I kann mittels herkömmlicher Methoden isoliert werden, beispielsweise durch Kristallisieren, Chromatographieren oder dergleichen.

Die Abgangsgruppe Z' kann ein Halogenatom, eine p-Toluolsulfonylgruppe, eine Methylsulfonylgruppe oder dergleichen sein.

Die als Ausgangsstoffe verwendeten Verbindungen der Formel II sind bekannt oder können nach an sich bekannten Methoden hergestellt werden. Beispiele von Verbindungen der Formel II sind :

2-Methyl-11-oxo-11H-pyrido [2,1-b] chinazolin-8-carbonsäure,
2-(1-Methyläthyl)-11-oxo-11H-pyrido [2,1-b] chinazolin-8-carbonsäure,
2-Methoxy-11-oxo-11H-pyrido [2,1-b] chinazolin-8-carbonsäure,
2-(1-Methyläthyl)-11-oxo-11H-pyrido [2,1-b] chinazolin-6-carbonsäure und
2-(1-Methyläthyl)-11-oxo-11H-pyrido [2,1-b] chinazolin-7-carbonsaüre.

Die Ausgangsstoffe der Formel III, worin Z ein Halogenatom bedeutet, sind bekannte Verbindungen oder können nach an sich bekannten methoden hergestellt werden. Beispiele solcher Verbindungen sind :

2-(1-Methyläthyl)-11-oxo-11H-pyrido [2,1-b] chinazolin-8-chlorcarbonyl,
2-Methoxy-11-oxo-11H-pyrido [2,1-b] chinazolin-8-chlorcarbonyl,
2-Brom-11-oxo-11H-pyrido [2,1-b] chinazolin-8-chlorcarbonyl und
2-Methyl-11-oxo-11H-pyrido [2,1-b] chinazolin-8-chlorcarbonyl.

Die Ausgangsstoffe der Formel III, worin Z Cyanomethoxy bedeutet, können hergestellt werden, indem man eine Verbindung der Formel II mit einem Haloacetonitril, wie Chloracetonitril, in einem polaren, aprotischen Lösungsmittel, wie Dimethylformamid, Hexamethylphosphoramid, N-Methylpyrrolidinon oder dergleichen, in Gegenwart einer Base, wie Kaliumcarbonat, Natriumcarbonat oder dergleichen, bei einer Temperatur zwischen 0 °C und 50 °C umsetzt.

Die Ausgangsstoffe der Formel III, worin Z phenoxy oder substituiertes Phenoxy bedeutet, können hergestellt werden, indem man eine Verbindung der Formel II in Gegenwart eines Säurehalogenides, wie Toluolsulfonylchlorid, in einem polaren, aprotischen Lösungsmittel, wie Dimethylformamid oder dergleichen, und bei einer Temperatur zwischen Raumtemperatur und 70 °C mit einem Phenol umsetzt.

Die Ausgangsstoffe der Formel IV sind bekannte Verbindungen oder können nach an sich bekannten Methoden hergestellt werden. Beispiele von Verbindungen der Formel IV sind :

3-(3-Hydroxypropyl) pyridin, 1-(4-Hydroxybutyl)-1H-imidazol, 4-(3-Hydroxypropyl) pyridin, 3-(6-Hydroxyhexyl) pyridin, 3-Pyridin-N-methylbutanamin, 1-Methyl-3-(3-pyridinyl) propanamin, 1-Methyl-3-(5-pyrimidinyl) propanamin, 5-(4-Hydroxybutyl) pyrimidin, 3-Pyridinbutanamin, 6-Pyridinhexanamin, 2-[[(3-Pyridinyl) methyl] thio] äthanamin, 4-(3-Pyridinyloxy) butanamin, 4-Pyridinbutanamin, 4-(1H-Imidazol-1-yl) butanamin, 4-(2-Methyl-1H-imidazol-1-yl) butanamin und 5-Pyrimidinbutanamin.

Die Ausgangsstoffe der Formel V sind bekannte Verbindungen oder können nach an sich bekannten Methoden hergestellt werden. Beispiele von Verbindungen der Formel V sind :

3-(3-Brompropyl) pyridin, 4-(3-Brompropyl) pyridin, 3-(6-Bromhexyl) pyridin und 3-(3-Methansulfonyloxypropyl) pyridin.

Die Verbindungen der obigen Formel I sind basische Verbindungen und können mit anorganischen oder organischen Säuren Säureadditionssalze bilden. Im speziellen können die Verbindungen der Formel I Säureadditionssalze mit pharmazeutisch annehmbaren organischen oder anorganischen Säuren

bilden, beispielsweise Hydrohalogenide, wie Hydrochloride, Hydrobromide oder Hydrojodide, andere Mineralsäuresalze, wie Sulfate, Nitrate, Phosphate oder dergleichen, Alkyl- und mono-Arylsulfonate, wie Aethansulfonate, Toluolsulfonate, Benzolsulfonate oder dergleichen, oder andere organische Säureadditionssalze, wie Acetate, Tartrate, Maleate, Citrate, Benzoate, Salicylate, Ascorbate oder dergleichen. Pharmazeutisch nicht-annehmbare Säureadditionssalze von Verbindungen der Formel I können mittels herkömmlichen Austauschreaktionen in pharmazeutisch annehmbare Säureadditionssalze übergeführt werden, wobei das pharmazeutisch nicht-annehmbare Anion durch ein pharmazeutisch annehmbares Anion ersetzt wird. Andererseits kann man ein pharmazeutisch nicht-annehmbares Säureadditionssalz neutralisieren und dann die so erhaltene freie Base mit einem ein pharmazeutisch annehmbares Anion liefernden Mittel umsetzen.

Die Verbindungen der Formel I und ihre Salze antagonisieren die durch SRS-A (Slow Reacting Substance of Anaphylaxis) und andere, bei allergischen Reaktionen freigesetzten Substanzen, wie Histamin und PAF (Platelet activating factor), bewirkte Bronchokonstriktion. Darüberhinaus hemmen die Verbindungen der Formel I und ihre Salze die Freisetzung von Allergievermittler-Substanzen und die Produktion von Thromboxan-$A_2$ durch die Hemmung der Thromboxan-Synthase. Dementsprechend können die Verbindungen der Formel I und ihre Salze bei der Behandlung von allergischen Erkrankungen, welche Hautleiden, Heuschnupfen, chronische Bronchitis, obstruktive Erkrankungen der Atemwege, wie Asthma, allergische Erkrankungen des Auges und allergische Erkrankungen des gastrointestinalen Traktes, wie Lebensmittelallergien, sowie bei der Behandlung von thrombotischen Gefässerkrankungen (Blutplättchen-Aggregation), wie ischämische Herzkrankheiten, pulmonale Hypertension oder dergleichen, verwendet werden.

Die nützlichen antiallergischen und antithrombotischen Eigenschaften der Verbindungen der Formel I und ihrer Salze können mittels Standard-Tests in vitro und in Warmblütern ermittelt werden. Beispiele solcher Tests sind :

a) Hemmung der durch LTE-bewirkten Bildung von Hautschwellungen in Ratten

Es wird das Vermögen eines Wirkstoffes bestimmt, die durch Leukotrien-E (LTE) bewirkte Bildung von Hautschwellungen in Ratten zu hemmen. In diesem Modellversuch verabreicht man anästhetisierten Ratten, welche man während 30 Minuten mit 50 mg/kg Pyrilamin-Maleat und 4 mg/kg Methylsergid-Maleat intraperitoneal vorbehandelt hat, eine Dosis von LTE, welche eine maximale Antwort bewirkt, in 0,5 ml Kochsalzlösung intradermal. Das Versuchstier wird dann sofort mit 10 mg/kg i. v. der Testsubstanz behandelt, worauf man 0,5-proz. Evans-Blau intravenös in die Schwanzvene des versuchstieres injiziert. 30 Minuten später werden die Versuchestiere getötet. Die durch LTE bewirkte Zunahme der kapillaren Permeabilität verursacht eine Wanderung des Farbstoffes zum Injektionspunkt in der Haut. Die erhaltene Antwort wird durch Messung der Grösse (in mm) des Farbfleckens auf der Haut quantifiziert. Die durchschnittlich im mit Testsubstanz behandelten Versuchstieren erhaltene Antwort (5 Versuchstiere, 4 intradermale Injektionen von LTE pro Versuchstier) wird mit der in Kontrolltieren erhaltenen Antwort verglichen. Fur die Ermittlung der oralen Aktivität werden die Versuchstiere während 2 Stunden vor der intradermalen Injektion mit LTE vorbehandelt. In diesem Modellversuch bewirkt 7-[3-(4-Acetyl-3-hydroxy-2-propylphenoxy)-2-hydroxypropoxy]-4-oxo-8-propyl-4H-1-benzopyran-2-carbonsäure eine Hemmung von 88 % bei 10 mg/kg i. v., ist jedoch oral inaktiv.

Für N-[4-(1H-Imidazol-1-yl) butyl]-2-(1-methyläthyl)-11-oxo-11H-pyrido [2,1-b] chinazolin-8-carboxamid wurde eine $ID_{50}$ von 46 mg/kg p. o. und eine $ID_{50}$ von 30 mg/kg i. v. ermittelt. Diese Substanz zeigte eine $LD_{50}$ von 775 mg/kg p. o. und 220 mg/kg i. p. in Mäusen.

Für 2-(1-Methyläthyl)-N-[4-(3-pyridyl) butyl]-11-oxo-11H-pyrido [2,1-b] chinazolin-8-carboxamid wurde eine $ID_{50}$ von 70 mg/kg p. o. und eine $ID_{50}$ von 15 mg/kg i. v. ermittelt. Diese Substanz zeigte eine $LD_{50}$ von 1 000 mg/kg p. o.

Für 2-(1-Methyläthyl)-N-[4-(4-pyridyl) butyl]-11-oxo-11H-pyrido [2,1-b] chinazolin-8-carboxamid wurde eine $ID_{50}$ von 90 mg/kg p. o. und eine $ID_{50}$ von 10 mg/kg i. v. ermittelt. Diese Substanz zeigte in Mäusen eine $LD_{50}$ von 1 000 mg/kg p. o.

b) Hemmung der durch LTE bewirkten Bronchokonstriktion in Meerschweinchen (in vivo)

Man anästhetisiert männliche, 300-450 g schwere Meerschweinchen intraperitoneal mit 2 g/kg Urethan und führt zwecks intravenöser Verabreichung von Substanzen eine Polyäthylenkanüle in die Vena jugularis ein. Mittels einer in die Trachea eingeführten Kanüle wird der tracheale Druck aufgezeichnet. Man paralysiert die Atmung mit 1,2 mg/kg i. v. Succinylcholin und beatmet die Versuchstiere mechanisch bei 40 Atemzüge/Minute und einem Atemvolumen von 2,5 $cm^3$. Zwei Minuten später verabreicht man 0,1 mg/kg i. v. Propranolol. Fünf Minuten später werden die Versuchstiere während 30 Sekunden mit 10 mg/kg der zu testenden Verbindung bzw. mit einem Kontrollvehikel vorbehandelt. Anschliessend behandelt man die Versuchstiere mit einer maximal konstringierenden Dosis von Leukotrien-E, und zwar ebenfalls intravenös. Die abgelesene Differenz (in cm $H_2O$) zwischen Vor- und Spitzen-Ventilationsdruck wird gemittelt, und zwar über drei Werte bei Kontrolltieren und über fünf Werte bei Tieren, welche mit Testsubstanz behandelt worden sind. Die prozentuale Hemmung wird

mittels der folgenden Formel berechnet :

$$\frac{\text{Kontrollwert-Testsubstanzwert}}{\text{Kontrollwert}} \times 100.$$

Zur Ermittlung der oralen Aktivität werden spontan atmende Versuchstiere während 2 Stunden mit 100 mg/kg der Testverbindung oral vorbehandelt, bevor sie mit Leukotrien-E behandelt werden. 7-[3-(4-Acetyl-3-hydroxy-2-propylphenoxy)-2-hydroxypropoxy]-4-oxo-8-propyl-4H-1-benzopyran-2-carbonsäure bewirkt eine 98-proz. Hemmung bei 10 mg/kg i. v., ist jedoch oral inaktiv in diesem Test.

Für N-[4-(1H-Imidazol-1-yl) butyl]-2-(1-methyläthyl)-11-oxo-11H-pyrido [2,1-b] chinazolin-8-carboxamid wurde eine $ID_{50}$ von 47 mg/kg p. o. und eine $ID_{50}$ von 0,9 mg/kg i. v. ermittelt.

Für 2-(1-Methyläthyl)-N-[4-(3-pyridyl) butyl]-11-oxo-11H-pyrido [2,1-b] chinazolin-8-carboxamid wurde eine $ID_{50}$ von 60 mg/kg p. o. und eine $ID_{50}$ von 0,3 mg/kg i. v. ermittelt.

Für 2-(1-Methyläthyl)-N-[4-[4-pyridyl) butyl]-11-oxo-11H-pyrido [2,1-b] chinazolin-8-carboxamid wurde eine $ID_{50}$ von 57 mg/kg p. o. und eine $ID_{50}$ von 1,9 mg/kg i. v. ermittelt.

c) Hemmung der durch LTE bewirkten Bronchokonstriktion in Meerschweinchen (in vivo, Aerosol)

Man anästhetisiert männliche, 300-500 g schwere Meerschweinchen intraperitoneal mit 2 g/kg Urethan und führt eine Polyäthylenkanüle in die Vena jugularis ein, durch welche die Wirkstoffe verabreicht werden können. Mittels einer in die Trachea eingeführten Kanüle wird der tracheale Druck aufgezeichnet. Nach chirurgischer Präparierung der Versuchstiere wartet man eine gewisse Zeit, damit die pulmonalen Funktionen sich stabilisieren können. Die zu testende Verbindung wird anschliessend gemäss dem nachfolgenden Protokoll verabreicht. Man verabreicht den Versuchstieren, welche spontan atmen, 0,1 mg/kg Propranolol intravenös. Fünf Minuten später werden die Versuchstiere während 5 Minuten einer 1-proz. Aerosollösung der zu testenden Verbindung (Gew/Vol) auf einen alkalischen pH eingestellt, sofern für die Lösung des Wirkstoffes notwendig, oder destilliertem Wasser mit einem geeigneten pH (zu Kontrollzwecken) ausgesetzt. Man verwendet ein Ultraschall-Sprühgerät, um alle Testsubstanzen durch Inhalation zu verabreichen. Die Ultraschallfrequenz des Sprühgerätes wird so eingestellt, dass der Durchmesser der produzierten Partikel 1-8 μ, durchschnittlich 3 μ beträgt. Wässrige Lösungen werden jeweils frisch hergestellt und in die Kammer des Sprühgerätes eingeführt. Der produzierte Sprühnebel wird den Versuchstieren verabreicht, indem man einen schräg gerichteten Strahl des Aerosols durch einen mit der Trachealkanüle verbundenem Y-Rohr leitet. Nach Ablauf der Behandlungszeit werden die Versuchstiere mit 1,2 mg/kg i. v. Succinylcholin paralysiert und bei 40 Atemzügen/Minute und einem Atemvolumen von 2,5 $cm^3$ mechanisch beatmet. 30 Sekunden nach der Verabreichung des Succinylcholins werden die Versuchstiere mit einer maximal konstringierenden Dosis von Leukotrien-E intravenöls behandelt.

Die abgelesene Differenz (in cm $H_2O$) zwischen Vor- und Spitzenventilationsdruck wird gemittelt, und zwar über drei Werte bei Kontrolltieren und über fünf Werte bei Versuchstieren, welche mit Testsubstanz behandelt worden sind. Die prozentuale Hemmung wird mittels der folgenden Formel berechnet :

$$\frac{\text{Kontrollwert-Testsubstanzwert}}{\text{Kontrollwert}} \times 100.$$

Werden verschiedene Wirkstoffkonzentrationen untersucht, so wird die prozentuale Hemmung für jede Konzentration aufgezeichnet, und zwar der Log Konzentration auf der Abszisse gegen die prozentuale Hemmung auf der Ordinate aufgetragen. Die $IC_{50}$ wird dann durch lineare Regressionsanalyse ermittelt.

Um die Wirkungsdauer zu ermitteln, werden die Versuchstiere wie oben beschrieben behandelt, mit der Ausnahme, dass die Zeit zwischen Behandlung mit Aerosol und LTE variliert wird. Sämtliche Testverbindungen werden bei derjenigen Konzentration verabreicht, welche notwendig ist um die durch Leukotrien bewirkte Bronchokonstriktion um 80 % zu hemmen. Die Wirkungsdauer wird aus der graphischen Aufzeichnung der Zeit (Abszisse) gegen die Hemmung in Prozent (Ordinate) berechnet.

In der nachfolgenden Tabelle I sind die Resultate aufgeführt, welche mit erfindungsgemässen Substanzen erhalten worden sind :

(Siehe Tabelle I Seite 9 f.)

Tabelle I

| Testsubstanz | $ID_{50}$ (% Aerosol) | Biologische Halbwertszeit (Min.) |
|---|---|---|
| N-[4-(1H-Imidazol-1-yl)-butyl]-2-(2-methyläthyl)-11-oxo-11H-pyrido[2,1-b]-chinazolin-8-carboxamid | 0,25 | 56 |
| 2-(1-Methyläthyl)-N-[4-(3-pyridyl)butyl]-11-oxo-11H-pyrido[2,1-b]chinazolin-8-carboxamid | 0,30 | 7 |
| Isoproternol | 0,00014 | 19,4 |
| $PGE_2$ | 0,00011 | 2,1 |
| 7-[3-(4-Acetyl-3-hydroxy-2-propylphenoxy)-2-hydroxy-propoxy]-4-oxo-8-propyl-4H-1-benzopyran-2-carbonsäure | 0,75 | 77 |

d) Hemmung der Thromboxan-$A_2$-Synthase ($TXA_2$-Syn.)

Die $TXA_2$-Synthase-Aktivität wird ermittelt, indem man die Umwandlung von $^{14}C$-Prostaglandin-endoperoxid ($PGH_2$) zu $^{14}C$-Thromboxan-$A_2$ ($TXA_2$) verfolgt, wobei man Mikrosomenfraktionen von menschlichen Blutplättchen als Enzymquelle verwendet. Im wässrigen Inkubationsmedium zersetzt sich das $TXA_2$ rasch zu $TXB_2$. Die Menge an $TXA_2$-Synthase wird so eingestellt, dass in Kontrollversuchen unter den angewendeten Versuchsbedingungen ungefähr 80-90 % des Substrates $PGH_2$ in Produkt umgewandelt wird. Um das $^{14}C$-$PGH_2$ herzustellen, wird $^{14}C$-Arachidonsäure ($^{14}C$-AA, 50-60 mCi/mMol) mit Mikrosomen von Samenbläschendrüsen von Schafen während 1,5 Minuten bei 37 °C inkubiert, worauf man das gebildete $^{14}C$-$PGH_2$ mit Diäthyläther extrahiert, an einer mit Sephadex LH-20 oder Kieselgel beladenen Kolonne reinigt und in Aceton bei — 70 °C aufbewahrt. Die Inkubationen werden wie folgt durchgeführt. Genügend $^{14}C$-$PGH_2$, um eine Endkonzentration des Substrates von 10 µM (30 000 cpm) zu erhalten, wird in die Inkubationsröhrchen gegeben, worauf man das Aceton unter Stickstoff entfernt. Man stellt die Röhrchen in ein Eisbad und versetzt nacheinander mit 250 µl eiskalter, phosphatgepufferter Kochsalzlösung, 10 µl Aethanol (zu Kontrollzwecken) oder Testsubstanz in Aethanol und 25 µl Mikrosomensuspension, wobei man jeweils so rasch als möglich mischt. Man inkubiert dann während 2 Minuten bei 22 °C, stoppt die Reaktion, extrahiert dann die radioaktiven Produkte und das nicht-umgesetzte $PGH_2$ und analysiert diese mittels Dünnschichtchromatographie. Die Menge an umgesetztem $^{14}C$-$PGH_2$ wird mittels Dünnschichtchromatographie ermittelt. Die Menge an umgesetztem $^{14}PGH_2$ wurde dann als Mass für die $TXA_2$-Synthase-Aktivität verwendet. Inhibitoren wurden anfänglich bei einer Endkonzentration von 100 µM getestet.

e) Antagonisierung der durch Acetylcholin-bewirkten Konstriktion in Meerschweinchen-Ilea

Man bestimmt die anticholinergische Aktivität an einem Meerschweinchen-Ileum-Segment, das in einem bei 37 °C gehaltenen Bad suspendiert wird, das mit Sauerstoff belüftete Tyrodes-Lösung enthält. In Abständen von 4 Minuten wird das Ileum mit $5 \times 10^{-7}$ M Acetylcholin genügend lange angeregt, um eine Spitzenkontraktion zu erzielen, und dann mit frischer Tyrodes-Lösung gewaschen. Nachdem sich die Reaktion des Gewebes auf die Behandlung mit Acetylcholin stabilisiert hat, wird das Ileum während 1 Minuten vor der erneuten Anregung mit Acetylcholin mit dem Standard-Anticholinergikum Atropinsulfat vorbehandelt. Die erzielten Kontraktionen werden aufgezeichnet und das Gewebe wird dann mit Tyrodes-Lösung gewaschen. Man wiederholt diese Versuche mit erhöhten Konzentrationen an Antropinsulfat, und zwar bis die Reaktion auf die Behandlung mit Acetylcholin maximal gehemmt wird. Für Atropin wurde eine $IC_{50}$ von $1 \times 10^{-8}$ M ermittelt. Die $IC_{50}$-Werte für die Testverbindungen wurden auf die gleiche Weise ermittelt.

f) Antagonisierung der durch Histamin-bewirkten Konstriktion in Meerschweinchen-Ilea

Die Antihistaminwirkung wird an einem Meerschweinchen-Ileum-Segment, das in einem bei 37 °C gehaltenen Bad von mit Sauerstoff belüfteter Tyrodes-Lösung besteht, bestimmt. In Abständen von 4

Minuten wird das Ileum mit 1 × $10^{-7}$ M Histamin genügend lange behandelt, um eine Spitzenkontraktion zu bewirken, und dann mit frischer Tyrodes-Lösung gewaschen. Nachdem sich die Reaktion des Gewebes auf die Behandlung mit Histamin stabilisiert hat, wird das Ileum während 1 Minute vor der erneuten Anregung mit Histamin,mit dem Standard-Antihistaminikum Pyrilaminmaleat vorbehandelt. Man zeichnet die Kontraktionen auf und wäscht das Gewebe jeweils mit Tyrodes-Lösung. Man verwendet dann steigende Konzentrationen an Pyrilaminmaleat, bis die Reaktion auf die Behandlung mit Histamin maximal gehemmt wird. Für Pyrilaminmaleat wurde in diesme Testsystem eine $IC_{50}$ von 1 × $10^{-8}$ M ermittelt. Die $IC_{50}$-Werte für fie Testverbindungen wurden auf die gleiche Weise ermittelt.

g) Antagonisierung der durch PAF (Platelet Activating Factor) und Histamin-bewirkten Bronchokonstriktion in Meerschweinchen

Spontan atmende, männliche, 300-450 g schwere Meerschweinchen werden 2 Stunden vor der Behandlung mit dem Agonisten mit der Testsubstanz oder einem Kontrollvehikel oral vorbehandelt. Die Versuchstiere werden dann mit 2 mg/kg Urethan intraperitoneal anästhetisiert, worauf man die Vena jugularis und die Trachea kanüliert. Nach 2 Stunden paralysiert man die Atmund mit 1,2 mg/kg i. v. Succinylcholin und beatmet die Tiere bei 40 Atemzügen/Minute und einem Atemvolumen von 2,5 $cm^3$ mechanisch. Man verabreicht 0,1 mg/kg i. v. Propranolol und 5 Minuten später eine konstringierende Dosis von PAF (10 µg/kg i. v.) oder Histamin-dihydrochlorid (20 µg/kg i. v.). Die abgelesene Differenz (in cm $H_2O$) zwischen Vor- und Spitzenventilationsdruck wird gemittelt, und zwar über drei Werte bei Kontrolltieren und über fünf Werte bei Tieren, welche mit Testsubstanz behandelt worden sind. Die prozentuale Hemmung wird mittels der folgenden Formel berechnet :

$$\frac{\text{Kontrollwert-Testsubstanzwert}}{\text{Kontrollwert}} \times 100.$$

h) Antagonisierung der durch Kaliumchlorid bewirkten Konstriktion in Meerschweinchen-Ilea

Ein 1-1,5 cm grosses Meerschweinchen-Ileum-Segment aus einem 200-250 g schweren Versuchstier wird in einem Organbad, enthaltend 10 ml Tyrodes-Lösung, $10^{-6}$ M Atropinsulfat und $10^{-6}$ M Pyrilaminmaleat, suspendiert. Das Bad wird unter Einleiten eines Gasgemisches, bestehend aus 95 % Sauerstoff und 5 % Kohlendioxid, bei 37 °C gehalten. Kaliumchlorid bewirkt bei Konzentrationen zwischen 10-30 mM isometrische Konstriktionen des Ileums. Die Kontraktionen bestehen aus zwei Phasen, nämlich einer anfänglichen schnellen Kontraktion (phasischer Teil) und einer folgenden, durch weniger intensive jedoch längere Kontraktionen gekennzeichneten Phase (tonische Phase). Man inkubiert das Ileum während 3 Minuten vor der Behandlung mit Kaliumchlorid bei verschiedenen Konzentrationen an Testsubstanz. Man ermittelt dann diejenigen Konzentrationen der Testsubstanz, welche den phasischen und tonischen Teil der durch Kaliumchlorid bewirkten Konstriktionen um 50 % hemmt ($IC_{50}$-Werte).

i) Hemmung der Hautanaphylaxie in passiv sensibilisierten Ratten

1. Herstellung des Antiserums

IgE-enthaltendes Ratten-Antiserum (Reagin-Antiserum) wurde gemäss folgenden Verfahren hergestellt. Normale, männliche, 150-200 g schwere Sprague-Dawley-Ratten wurden durch eine intraperitoneale Injektion von 0,5 ml Bordetella pertussis-Vakzin (20 ou/ml), enthaltend 100 µg Eieralbumin, immunisiert. Nach 16 Tagen verabreicht man subkutan Nippostrongylus brasiliensis (3 000 Larven/0,1 ml) und nach 21 Tagen 10 µg Eieralbumin intraperitoneal in 0,5 ml gewöhnlicher Kochsalzlösung. Nach 30 Tagen entnimmt man Blut mittels Herzpunktion, trennt das Serum durch Zentrifugation ab und bewahrt es über Nacht bei 5 °C auf. Um das Serum auf Antikörper-Aktivität zu untersuchen, verwendet man den PCA-Test (passive cutaneous anaphylaxis test) nach einer Sensibilierungs-Periode von 24 Stunden (siehe weiter unten). Man verwendete nur diejenigen Sera, welche im PCA-Test nach einer intradermalen Injektion von 0,05 ml einer verdünnten Lösung des Serums in normaler Kochsalzlösung (1 : 50) einen durchschnittlichen Schwellungs-Durchmesser von 3 mm oder mehr verursachten. Diese Sera werden gepoolt, in Aliquote aufgeteilt und bis zur Verwendung eingefroren. Die Reagin-Natur des Antiserums zeigt sich dadurch, dass nach Erwärmen auf 56 °C während 4 Stunden die Aktivität verloren geht. Dies wurde durch Inkubieren des Serums in vitro mit Ratten-Anti-IgE, -Anti-IgG, -Anti-IgA und -Anti-IgM bestätigt. Nach Inkubieren dieser Proben während 1 Stunde bei 20 °C und während 16 Stunden bei 4 °C wurden sie zentrifugiert, worauf die Ueberstände im PCA-Test auf Aktivität hin untersucht wurden. Nur die Ueberstände von Inkubationsversuchen mit Anti-IgE waren inaktiv.

2. Tierversuch

Der PCA-Test wurde mit 190-220 g schweren, männlichen Sprague-Dawley-Ratten und auf ähnliche

Weise durchgeführt, wie durch J. Goose und A.M.J.N. Blair, Immunology 16, 749 (1969) beschrieben. Man stellt eine verdünnte (1 : 512) Lösung des Serums in Kochsalzlösung her und injiziert zwei Aliquote (0,05 ml) dieses Titers intradermal in das rasierte Fell am Rücken der Versuchstiere (diese Menge des Antikörpers verursachte einen mittleren Schwellungsdurchmesser von etwa 7 mm). Nach einer Sensibilisierungsperiode von 24 Stunden verabreicht man jeder Ratte intravenös durch die Schwanzvene 1 ml einer wässrigen Lösung von 8 mg Eieralbumin und 5 mg Evans-Blau. Nach 40 Minuten werden die Versuchstiere mittels Genickbruch getötet, worauf man das dorsale Fell für die Untersuchung entfernt. Mit einer Schublehre mit Nonius misst man die lange und die kurze Achse jeder schwellung und bestimmt den jeweiligen durchschnittlichen Durchmesser. Die Testsubstanzen werden entweder intravenös und gleichzeitig wie das Antigen oder oral 5, 10, 20, 30 oder 60 Minuten vorher verabreicht. Für die intravenöse Verabreichung werden die Testsubstanzen in Dimethylsulfoxid (DMSO) gelöst, wobei die Konzentrationen so eingestellt wurden, dass jeweils 0,1 ml/100 g Körpergewicht der DMSO-Lösung verabreicht werden konnten. Für die orale Verabreichung wurden die Testsubstanzen in einem wässrigen Vehikel (0,15 M-Natriumchloridlösung, enthaltend 0,5 % Carboxymethylcellulose, 0,4 % Tween 80 und 0,9 %Benzylalkohol) suspendiert, wobei die Konzentration jeweils so eingestellt wurde, dass 0,5 ml/100 g Körpergewicht der Suspension verabreicht werden konnten. Für die Bestimmung der oralen $ID_{50}$-Werte verwendete man logarithmisch eingeteilte Dosen an Testsubstanz und verabreichte sie zum Zeitpunkt der Spitzenaktivität. Man verwendete dabei jeweils fünf Versuchstiere aus der Kontroll- und der Behandlungsgruppe. Die statistische Analyse der Resultate erfolgte mittels des t-Testes (Student). Für die Berechnung der $ID_{50}$-Werte verwendete man die Methode von J. Berkson, J. Am. Stat. Assoc. 48, 565 (1953).

j) Antagonisierung von SRS-A in vitro

Für die Bestimmung von SRS-A verwendete man eine modifizierte Version des Meerschweinchen-Ileum-Testes, der durch Orange et al., Adv. Immunol. 10, 105 (1969) beschrieben worden ist. Ein 1,5 cm grosses Ileum-Segment aus einem 175-200 g schweren Meerschweinchen wird in einem Organbad, das Tyrodes-Lösung, $10^{-6}$ M Atropinsulfat und $10^{-6}$ M Pyrilaminmaleat enthält, suspendiert. Das Bad wird bei 37 °C gehalten und mit einer Mischung von 95 % Sauerstoff und 5 % Kohlendioxid belüftet. Das Ileum-Segment wird nun mit einem Messgerät verbunden, das die isotonischen Kontraktionen registriert und aufzeichnet. Man stellt auf eine Restspannung von 0,5 g ein und lässt das Gewebe dann während 1 Stunde ins Gleichgewicht kommen.

Das in diesem Versuch verwendete SRS-A erhält man, indem man eine grosse Menge zerkleinerter Lungenstücke von aktiv sensibilisierten Meerschweinchen in vitro mit Eieralbumin anregt. Die Versuchstiere werden gegenüber Eieralbumin sensibilisiert, indem man ihnen 28-45 Tage vor Versuchsbeginn 10 mg Antigen in 1 ml Kochsalzlösung intraperitoneal injiziert. Der in vitro-Versuch wird durchgeführt, indem man zuerst das Lungengewebe (40 mg/ml) bei 37 °C in Tyrodes-Lösung während 5 Minuten vorinkubiert, das Eieralbumin dazugibt (40 g/ml Endkonzentration) und nach 10 Minuten das SRS-A-enthaltende Medium durch Filtration durch Papierfilter (Whatman n° 1) vom Lungengewebe abtrennt. Durch Vergleich der Kontraktions-Amplituden, welche mit einem vorgegebenen Volumen des Filtrates erhalten werden, mit denjenigen, welche durch verschiedene Konzentrationen von Histamin bewirkt werden, ermittelt man die Konzentration von SRS-A im Filtrat. Eine Einheit SRS-A entspricht derjenigen Menge, welche notwendig ist, um die gleiche Anzahl Kontraktionen zu bewirken wie 5 ng Histamin. Nach Standardisierung wird dieses Material in kleinen Aliquoten bei — 80 °C aufbewahrt. Man lässt die durch SRS-A bewirkten Kontraktionen während 5 Minuten andauern, bevor man das Gewebe mit Tyrodes-Lösung wäscht. SRS-A-Antagonismus wird bestimmt, indem man das Ileum während 30 Sekunden vor der Zugabe einer Menge an SRS-A, welche ein submaximale Reaktion bewirkt, mit der zu testenden Verbindung vorbehandelt, die Amplitude der Kontraktionen in Gegenwart der Testsubstanz bestimmt und dann das Ileum mit Tyrodes-Lösung wäscht. In diesem Versuch wurde für die Referenzverbindung 7-[3-(4-Acetyl-3-hydroxy-2-ropylphenoxy)-2-hydroxy-propoxy]-4-oxo-8-propyl-4H-1-benzopyran-2-carbonsäure eine $IC_{50}$ von $3 \times 10^{-8}$ M ermittelt.

Die mit erfindungsgemässen Substanzen in den Versuchen d) - j) erhaltenen Resultate sind in der Tabelle II zusammengestellt.

(Siehe Tabelle II Seite 12 f.)

k) Schutz vor der durch PAF bewirkten Mortalität

Männliche, 20-30 g schwere Mäuse werden ad libitum gefüttert. Man stellt eine Lösung von 1 mg/ml des Hemihydrates des inneren Salzes von racemischem 2-[[[2-(Acetyloxy)-3-(octadecyloxy) propoxy] hydroxyphosphinyl] oxy]-N,N,N-trimethyläthanaminiumhydroxid in Aethanol her. Für den Versuch wird das PAF mit 0,1-proz. BSA (Rinderserumalbumin) verdünnt. Man verabreicht diese Lösung bei einer Dosis von 0,1 mg/kg 1 Stunde nachdem 10 Mäuse entweder mit Vehikel (BSA) oder Testsubstanz behandelt worden sind. Die mit PAF behandelten Mäuse sterben innerhalb von 30 Minuten nach i. v.-Verabreichung. Die Mortalität in Mäusen, welche nur mit PAF behandelt wurden, beträgt mindestens 80 %.

Wurde 2-(1-Methyläthyl-N-[6-(3-pyridyl) hexyl]-11-oxo-11H-pyrido [2,1-b] chinazolin-8-carboxamid als Testsubstanz verwendet, so wurden die Versuchstiere von der durch PAF bewirkten Mortalität geschützt.

Tabelle II

| Versuch d) - j) | Verbindung A[1] | $IC_{50}$ ($ID_{50}$) Verbindung B[2] | Verbindung C[3] |
|---|---|---|---|
| (d) Hemmung der Thromboxan-$A_2$-Synthase | $10^{-7}$ - $10^{-6}$M | $10^{-7}$ - $10^{-6}$M | $10^{-7}$M |
| (e) Antagonisierung der durch Acetylcholin bewirkten Konstriktionen in Meerschweinchen-Ilea | 5 x $10^{-6}$M | 7 x $10^{-6}$ | - |
| (f) Antagonisierung der durch Histamin bewirkten Konstriktionen in Meerschweinchen-Ilea | 1 x $10^{-6}$M | 2 x $10^{-6}$M | - |
| (g) Antagonisierung der durch PAF bewirkten Bronchokonstriktionen in Meerschweinchen | 13 mg/kg (p.o., 2 Stunden Vorbehandlung) | 100 mg/kg | 50 mg/kg |
| (g) Antagonisierung der durch Histamin bewirkten Bronchokonstriktionen in Meerschweinchen | 39 mg/kg (p.o., 2 Stunden Vorbehandlung) | - | - |
| (h) Antagonisierung der durch Kaliumchlorid bewirkten Konstriktionen in Meerschweinchen-Ilea | 1,7 x $10^{-5}$M (phasisch) 1 x $10^{-5}$M (tonisch) | 1,3 x $10^{-5}$M (phasisch) 8 x $10^{-6}$M (tonisch) | 5,4 x $10^{-6}$M (phasisch) 2,4 x $10^{-6}$ (tonisch) |
| (i) Hemmung der Hautanaphylaxis in passiv sensibilisierten Ratten | 20 mg/kg (p.o., 10 Minuten Vorbehandlung) | 21 mg/kg (p.o., 5 Minuten Vorbehandlung) | - |
| (j) Antagonisierung von SRS-A in vitro | 1 x $10^{-6}$M | 1 x $10^{-6}$M | |

(1) Verbindung A N-[4-(1H-Imidazol-1-yl) butyl]-2-(1-methyläthyl)-11-oxo-11H-pyrido [2,1-b]-chinazolin-8-carboxamid

(2) Verbindung B 2-(1-Methyläthyl)-N-[4-(3-pyridyl) butyl]-11-oxo-11H-pyrido [2,1-b]-chinazolin-8-carboxamid

(3) Verbindung C 2-(1-Methyläthyl)-N-[6-(3-pyridylhexyl]-11-oxo-11H-pyrido [2,1-b]-chinazolin-8-carboxamid

Für diese Verbindung wurde eine orale $ID_{50}$ von 13 mg/kg ermittelt.

Für 2-(1-Methyläthyl)-N-[6-(1H-imidazol-1-yl) hexyl]-11-oxo-11H-pyrido [2,1-b] chinazolin-8-carboxamid wurde eine $ID_{50}$ von 17 mg/kg ermittelt.

1) Hemmung der Blutplättchen-Aggregation in vitro

Man verwendet die turbidometrische Methode von G.V.R. Born, Nature 194, 927 (1962). Mit 0,38-proz. Natriumcitrat-Blut stellt man Plasma, das reich and Blutplättchen ist (PRP), und Plasma, das arm an Blutplättchen ist (PPP), her. Je nach Spezies variierte die Geschwindigkeit und die Dauer der Zentrifugation ; menschliches Blut : 160 g während 20 Minuten, Hundeblut : 160 g während 15 Minuten, Meerschweinchen- und Kaninchenblut : 160 g während 15 Minuten, Rattenblut : 230 g während 10 Minuten. Die Aggregation wird mit einem Payton-Dual-Kanal-Gerät oder, wenn die Preisetzung von ATP gleichzeitig gemessen wird, mit einem Chronolog-Lumiaggregometer verfolgt. Man gibt 0,45 ml PRP in eine Küvette und wärmt auf 37 °C vor. Das Stimulans (Arachidonsäure oder synthetisches PAF) wird in Volumina von 50 µl zugegeben ; alle anderen Reagenzien in 5 µl. Zu Proben, welche kein Agens enthalten, gibt man ein Vehikel. Mit PRP und PPP werden die Werte für 0 und 100 % Lichttransmission festgelegt. Die prozentuale Aggregation wird aus der prozentualen Zunahme der Lichttransmission 5 Minuten nach der Zugabe des Stimulans berechnet. Für die Untersuchung der Hemmung der Aggregation wird der Inhibitor zuerst zugegeben und mit den Blutplättchen während 1 Minute unter Rühren vorinkubiert, worauf man das Stimulans zugibt. Die prozentuale Hemmung wird aus der prozentualen Aggregation mit und ohne Testverbindung berechnet.

$$\% \text{ Hemmung} = \frac{(\% \text{ Aggregation} + \text{Verbindung})}{(1\text{-}\% \text{ Aggregation} + \text{Vehikel})} \times 100.$$

Die mit erfindungsgemässen Substanzen erhaltenen Resultate sind in der Tabelle III zusammengestellt.

Tabelle III

Hemmung der durch Arachidonsäure und PAF[(1)] bewirkten Blutplättchenaggregation

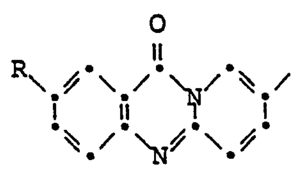 $CO\text{-}X\text{-}(CH_2)_n\text{-}Het$

| R | X | N | Het | AA[(2)] | PAF[(2)] |
|---|---|---|---|---|---|
| $(CH_3)_2CH$ | NH | 4 | 3-Pyridyl | + | + |
| HO | NH | 4 | 3-Pyridyl | + | + |
| $CH_3$ | NH | 4 | 3-Pyridyl | + | - |
| $(CH_3)_2CH\text{-}O$ | NH | 4 | 3-Pyridyl | + | + |
| $(CH_3)_2CH$ | NH | 6 | 3-Pyridyl | + | + |
| $(CH_3)_2CH$ | NH | 4 | 4-Pyridyl | - | + |
| $(CH_3)_2CH$ | NH | 5 | 4-Pyridyl | + | + |
| $(CH_3)_2CH$ | -O- | 3 | 3-Pyridyl | - | + |
| $(CH_3)_2CH$ | NH | 2 | 4-Thiopyridyl | + | + |
| $(CH_3)_2CH$ | NH | 4 | 1-Imidazolyl | + | - |
| $(CH_3)_2CH$ | NH | 5 | 1-Imidazolyl | + | - |

(1) PAF ist das Hemihydrat des inneren Salzes von racemischem 2-[[[2-(Acetyloxy)-3-(octadecyloxy) propoxy]-hydroxyphosphinyl] oxy]-N,N,N-trimethyläthanaminiumhydroxid

(2) $IC_{50} < 10$ µM werden mit (+) bezeichnet

m) Radiorezeptor-Bindungs-Versuch mit PAF

Plasma, das reich an Blutplättchen ist, wird hergestellt, indem man Citratblut von Hunden zentrifugiert. Man stellt dann mit 0,15M-Zitronensäure auf pH 6,5 ein und zentrifugiert während 10 Minuten bei 1 000 g, worauf man die erhaltene Blutplättchenmasse durch Suspendieren in mit EDTA-Phosphat gepufferter Kochsalzlösung (PBS) und erneutes Zentrifugieren wäscht. Das gewaschene Blutplättchen-Präparat wird in BSA-PBS aufgenommen ($2 \times 10^{-7}$ Blutplättchen/50 µl).

In ein 400 µl Mikrozentrifugierröhrchen, das 50 µl Silikonöl (spezifisches Gewicht 1,023) enthält, gibt man Puffer, PAF-Standard oder Analoges oder einen Extrakt, um das Volumen auf 150 µl zu bringen. Man versetzt dann mit 50 µl $^3$H-PAF (10 000 Cpm, 45 Ci/mM) und anschliessend mit $2 \times 10^{-7}$ Hunde-Blutplättchen. Nach Mischen, Inkubieren während 10 Minuten bei Raumtemperatur und Zentrifugieren während 1 Minute bei 8 000 g wird das Material durch Aufbrechen des Röhrchenendes entfernt und in 200 µl 50-proz. Methanol aufgenommen. Man zählt dann in 10 ml Aquasol. Innerhalb einer Inkubationszeit von 10 Minuten erhält man eine Kurve von 50-2 500 pg/Röhrchen, was zeigt, dass eine hohe Spezifizität und Korrelation mit der biologischen Aktivität von PAF und dessen Analogen besteht.

Für 2-(1-Methyläthyl)-N-[4-(3-pyridinyl) butyl]-11-oxo-11H-pyrido [2,1-b] chinazolin-8-carboxamid wurde eine $IC_{50}$ von 0,45 µM ermittelt.

Für 2-(1-Methyläthyl)-N-[6-(3-pyridinyl) hexyl]-11-oxo-11H-pyrido [2,1-b] chinazolin-8-carboxamid wurde eine $IC_{50}$ von 1,0 µM ermittelt.

Die Dosierung der zu verabreichenden Verbindung der Formel I und ihrer Salze und die Häufigkeit der Verabreichung hängen ab von der Wirksamkeit und der Wirkungsdauer dieser Verbindung, von der Art der Verabreichung, sowie von der Stärke der zu behandelnden Krankheit, vom Alter des zu behandelnden Säugers usw. Im allgemeinen dürfte die tägliche Dosis einer Verbindung der Formel I oder eines Salzes davon in einem Bereich von etwa 100 bis etwa 1 500 mg liegen, wobei die Verabreichung in einer einzigen Dosierung oder in mehreren Einzeldosen erfolgen kann.

Eine Verbindung der Formel I oder ein Salz davon oder ein pharmazeutisches Präparat, enthaltend eine therapeutisch wirksame Menge einer Verbindung der Formel I oder eines Salzes davon, kann nach an sich bekannten Methoden verabreicht werden. Eine Verbindung der Formel I oder ein Salz davon kann als Einzelsubstanz verabreicht werden oder zusammen mit anderen Wirkstoffen, z. B. Antihistaminika, Inhibitoren von freigesetzten Vermittlersubstanzen, Methylxanthine, $B_2$-Agonisten oder antiasthmatischen Steroiden, wie Prednison und Prednisolon. Diese Substanzen können oral, parenteral, rektal oder durch Inhalation, z. B. in Form eines Aerosols, mikropulverisiertem Pulvers oder in Form einer versprühten Lösung, verabreicht werden. Für die orale Verabreichung eignen sich Tabletten, Kapseln, z. B. in Mischung mit Talk, Stärke, Milchzucker oder anderen inerten Hilfsstoffen, d. h. pharmazeutisch annehmbare Träger, oder wässrige Lösungen, Suspensionen, Elixire oder wässrige alkoholische Lösungen, z. B. in Mischungen mit Zucker oder anderen Süssstofen, Aromastoffen, Färbemitteln, Verdickungsmitteln und anderen herkömmlichen pharmazeutischen Hilfsstoffen. Für die parenterale Verabreichung eignen sich Lösungen oder Suspensionen, z. B. wässrige Lösungen oder Erdnussöl-Lösungen oder -suspensionen unter Verwendung von üblichen Hilfsstoffen und Trägermaterialien. Für die Verabreichung in Form von Aerosolen können diese Substanzen in einem geeigneten, pharmazeutisch annehmbaren Lösungsmittel gelöst werden, z. B. in Aethanol oder Wasser oder in Kombinationen von mischbaren Lösungsmitteln, und mit einem pharmazeutisch annehmbaren Treibgas vermischt werden. Solche Aerosole werden in geeignete Druckflaschen abgefüllt, welche mit einem für solche Zwecke geeigneten Ventil versehen sind. Dieses Ventil ist vorzugsweise ein Dosierungsventil, d. h. ein Ventril, das bei Betätigung eine vorbestimmte Menge einer wirksamen Dosis des Aerosols freigibt.

Die folgenden Beispiele 1-50, 72, 74 und 75 illustrieren die vorliegende Erfindung, wahrend die Beispiele 51-71, 73 und 76-117 die Herstellung von Ausgangsstoffen beschreiben. Alle Temperaturen sind in Celsiusgraden angegeben, sofern nichts anderes erwähnt ist.

Beispiel 1 (Methode A)

Eine Mischung aus 5,0 g 2-(1-Methyläthyl)-11-oxo-11H-pyrido [2,1-b] chinazolin-8-carbonsäure, 5,2 g 3-(3-Pyridin)-propyl] bromid-hydrobromid und 5,2 g Kaliumcarbonat in 200 ml Dimethylformamid wird während 45 Minuten in einem 70° warmen Bad erwärmt. Man verteilt das Reaktionsgemisch zwischen 100 ml Wasser und 100 ml Methylenchlorid und extrahiert die wässrige Phase dreimal mit je 100 ml Methylenchlorid. Man wäscht die vereinigten Auszüge mit 100 ml Portionen 10-proz. Natronlauge und Wasser, trocknet über Kaliumcarbonat und dampft ein. Man erhält 6,3 g eines Oeles, das man mittels präparativer Flüssigkeitschromatographie an Kieselgel unter Eluieren mit Essigester/Hexan (3 : 1) reinigt. Die Hauptfraktion enthält 4,6 g eines orange-farbenen Oels, das man aus Essigester/Hexan kristallisiert. Man erhält 2,7 g (38 %) 2-(1-Methyläthyl)-11-oxo-11H-pyrido [2,1-b] chinazolin-8-carbonsäure-3-(3-pyridyl) propylester vom Schmelzpunkt 108-109°.

Das aus wässrigem Isopropanol/Aether erhaltene Hydrochlorid schmilzt bei 226-227°.

Beispiel 2 (Methode B)

Eine Mischung aus 20,84 g 2-(1-Methyläthyl)-11-oxo-11H-pyrido [2,1-b] chinazolin-8-carbonsäure und 400 ml Thionylchlorid wird solange unter Rückfluss zum Sieden erhitzt, bis vollständige Lösung eintritt.

Man entfernt das Thionylchlorid unter vermindertem Druck und verdünnt den Rückstand viermal mit je 100 ml Toluol, wobei man jeweils wieder eindampft. Man nimmt den Rückstand in Toluol auf, versetzt mit 9 g 2-(3-Pyridyl) äthanamin und erhitzt die erhaltene Lösung während 6 Stunden unter Rückfluss zum Sieden. Man konzentriert die erhaltene Mischung verbeilt den Rückstand zwischen Methylenchlorid und Wasser, trocknet die organische Phase über Magnesiumsulfat, dampft ein und stellt den Rückstand mit Salzsäure sauer. Durch Kristallisieren aus Aethanol erhält man 11,5 g N-[2-(3-Pyridyl) äthyl]-2-(1-methyläthyl)-11-oxo-11H-pyrido [2,1-b] chinazolin-8-carboxamid vom Schmelzpunkt 283-292°. Nach dreimaligem Umkristallisieren aus Aethanol/Aether erhält man 2,75 g (8 %) des Produktes vom Schmelzpunkt 293-296°.

Beispiel 3 (Methode C)

Man versetzt eine eiskalte Suspension von 16,15 g 2-(1-Methyläthyl)-11-oxo-11H-pyrido [2,1-b] chinazolin-8-carbonsäure, 15,75 g Diphenylphosphorylazid und 10,2 g 6-(3-Pyridyl) hexanamin in 100 ml trockenem Dimethylformamid mit 8 ml Triäthylamin. Man hält die Reaktionsmischung während 2 Stunden bei 0° und lässt dann über Nacht auf Raumtemperatur erwärmen. Die erhaltene klare, gelbe Lösung wird mit Wasser und 5M-Natronlauge verdünnt und dreimal mit je 100 ml Methylenchlorid extrahiert. Die vereinigten organischen Auszüge werden über Kaliumcarbonat getrocknet und zu einem gelben halbfesten Körper eingedampft, den man aus Essigester/Hexan umkristallisiert. Man erhält 21,22 g (84 %) N-[6-(3-Pyridyl) hexyl]-2-(1-methyläthyl)-11-oxo-11H-pyrido [2,1-b] chinazolin-8-carboxamid vom Schmelzpunkt 94-96°. Durch erneutes Umkristallisieren erhält man Material vom Schmelzpunkt 100-103°. Man überführt dieses Material in das Dihydrochlorid und erhält 22,12 g Material vom Schmelzpunkt 230-236° (Zersetzung). Durch Umkristallisieren aus Aethanol/Aether erhält man 21,06 g Dihydrochlorid vom Schmelzpunkt 234-240° (Zersetzung).

Beispiel 4 (Methode D)

Eine Lösung von 1,30 g Cyanomethyl-2-(1-methyläthyl)-11-oxo-11H-pyrido [2,1-b] chinazolin-8-carboxylat und 1,20 g (1H-Imidazol-1-yl) butanamin in 15 ml Dimethylformamid wird während 18 Stunden in einem 35 °C warmen Bad gerührt. Man verdünnt die Reaktionsmischung mit Wasser und kristallisiert dann das ausgefallene Rohmaterial aus Acetonitril um. Man erhält 1,41 g (85 %) N-[4-(1H-Imidazol-1-yl) butyl]-2-(1-methyläthyl)-11-oxo-11H-pyrido [2,1-b] chinazolin-8-carboxamid vom Schmelzpunkt 165-167°.

(Siehe Tabellen Seite 16 ff.)

Beispiele 5-48

Pyrido [2,1-b] chinazolin-8-carbonsäurederivate der Formel Ic, hergestellt gemäss einem der in den Beispielen 1, 2, 3 und 4 beschriebenen Verfahren.

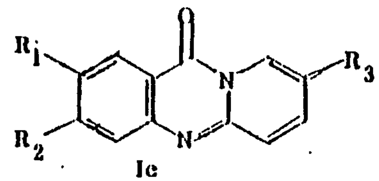

Ic

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 5 | $(CH_3)_2CH-$ | H | 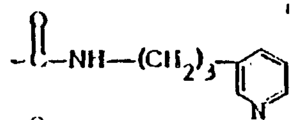 | C | 52 | 221-222 | $EtOH-Et_2O$ | $C_{24}H_{24}N_4O_2 \cdot 2HCl \cdot H_2O$ |
| 6 | $(CH_3)_2CH-$ | H | 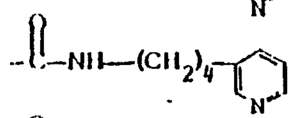 | C | 48 | 247-252 | $EtOH-Et_2O$ | $C_{25}H_{26}N_4O_2 \cdot 2HCl$ |
| 7 | $(CH_3)_2CH-$ | H | 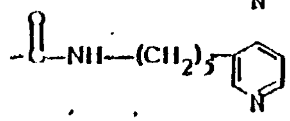 | C | 60 | 227-230 | $EtOH-Et_2O$ | $C_{26}H_{28}N_4O_2 \cdot 2HCl$ |

Beispiele 5-48 (Fortsetzung)

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 8 | $(CH_3)_2CH-$ | H | 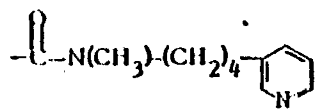 | C | 65 | 216-218 | $EtOH-Et_2O$ | $C_{26}H_{28}N_4O_2 \cdot 2HCl$ |
| 9 | $(CH_3)_2CH-$ | H | 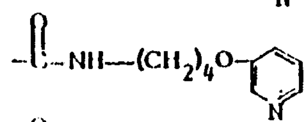 | C | 65 | 200-204 | $PrOH-Et_2O$ | $C_{25}H_{26}N_4O_3 \cdot 2HCl \cdot H_2O$ |
| 10 | $(CH_3)_2CH-$ | H | 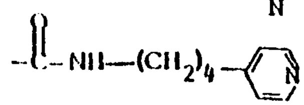 | C | 50 | 259-261 | EtOH | $C_{25}H_{26}N_4O_2 \cdot 2HCl$ |
| 11 | $(CH_3)_2CH-$ | H | 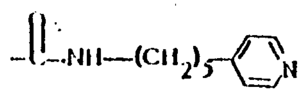 | C | 38 | 158-159 | $CH_2Cl_2$-Hex | $C_{26}H_{28}N_4O_2$ |
| 12 | $(CH_3)_2CH-$ | H | 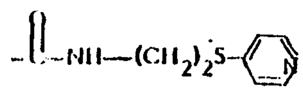 | C | 68 | 156-159, 190 | $CH_2Cl_2$-Hex | $C_{23}H_{22}N_4O_2S$ |
| 13 | $(CH_3)_2CH-$ | H | 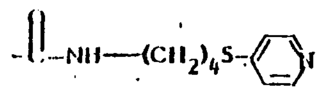 | C | 55 | 169-171 | $CH_2Cl_2$-Hex | $C_{25}H_{26}N_4O_2S$ |
| 14 | $(CH_3)_2CH-$ | H | 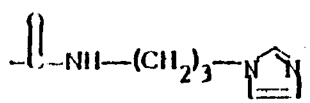 | C | 47 | 265-267 | EtOH | $C_{22}H_{23}N_5O_2 \cdot 2HCl$ 0,58$H_2O$ |

Beispiele 5-48 (Fortsetzung)

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 15 | $(CH_3)_2CH-$ | H | 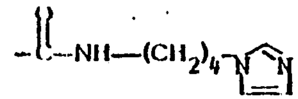 | C | 42 | 261-263 | $EtOH-Et_2O$ | $C_{23}H_{25}N_5O_2 \cdot 2HCl$ |
| 16 | $(CH_3)_2CH-$ | H | 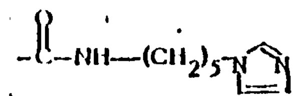 | C | 13 | 232-234 | EtOH | $C_{24}H_{27}N_5O_2 \cdot 2HCl$ |
| 17 | $CH_3-$ | H | 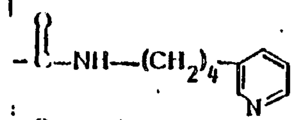 | C | 66 | 289-290 | $EtOH-Et_2O$ | $C_{23}H_{22}N_4O_2 \cdot 2HCl$ |
| 18 | $CH_3O-$ | H | 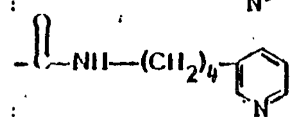 | C | 75 | 172-173 | THF-Hex | $C_{23}H_{22}N_4O_3$ |
| 19 | $(CH_3)_2CHO-$ | H | 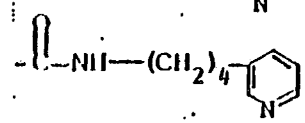 | C | 81 | 253-255 | $EtOH-Et_2O$ | $C_{25}H_{26}N_4O_3 \cdot 2HCl$ $0.66\ H_2O$ |
| 20 | H | H | 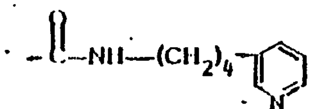 | C | 53 | 179-183 | $IProh-CH_2Cl_2-Hex$ | $C_{22}H_{20}N_4O_2$ |
| 21 | Br- | H | 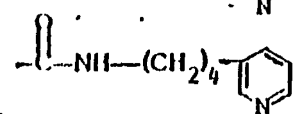 | C | 50 | 197-199 | $CH_2Cl_2-CH_3CN$ | $C_{22}H_{19}BrN_4O_2$ |
| 22 | HO- | H | 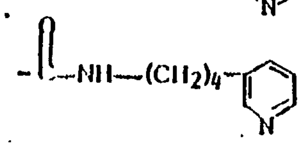 | C | 11 | 244 | $DMF-H_2O$ | $C_{22}H_{20}N_4O_2\ {}^1/_3\ H$ |

Beispiele 5-48 (Fortsetzung)

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 23 | H | $CH_3O$- | 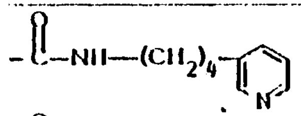 | C | 48 | 184-186 | $CH_2Cl_2$-$CH_3CN$ | $C_{23}H_{22}N_4O_3$ |
| 24 | H | Cl- | 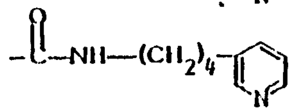 | C | 23 | 175-178 | $CH_2Cl_2$-$CH_3CN$ | $C_{22}H_{19}ClN_4O_2$ |
| 25 | $CH_3$- | $CH_3$- | 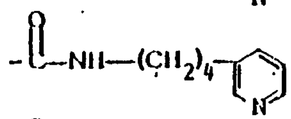 | C | 53 | 188-189.5 | $CH_2Cl_2$-$CH_3CN$ | $C_{24}H_{24}N_4O_2$ |
| 26 | $(CH_3)_2CH$- | H | 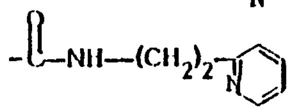 | B | 35 | 248-250 | $CH_3OH$-$Et_2O$ | $C_{23}H_{22}N_4O_2 \cdot 2HCl$ |
| 27 | $(CH_3)_2CH$- | H | 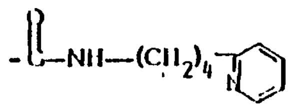 | C | 49 | 118-120 | EtOAc-Hex | $C_{25}H_{26}N_4O_2$ |
| 28 | $(CH_3)_2CH$- | H | 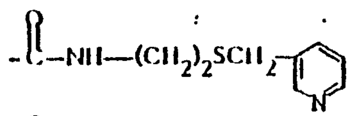 | C | 36 | 204-210 | aq.EtOH-$Et_2O$ | $C_{24}H_{24}N_4O_2S \cdot HCl$ |
| 29 | $(CH_3)_2CH$- | H | 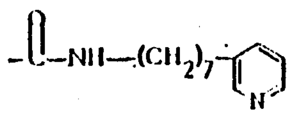 | C | 75 | 103-105 | EtOAc-Hex | $C_{28}H_{32}N_4O_2$ |
| 30 | $(CH_3)_2CH$- | H | 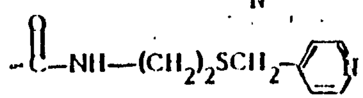 | C | 53 | 140-142 | EtOAc | $C_{24}H_{24}N_4O_2S$ |

Beispiele 5-48 (Fortsetzung)

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 31 | $(CH_3)_2CH-$ | H | 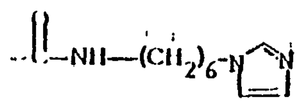 | C | 48 | 135-136 | EtOAc | $C_{25}H_{29}N_5O_2$ |
| 32 | $(CH_3)_2CH-$ | H | 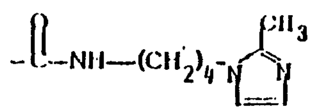 | C | 4 | 270-272 | $CH_3OH$-$CH_3CN$ | $C_{24}H_{27}N_5O_2 \cdot 2\,HCl$ |
| 33 | $(CH_3)_2CH-$ | H | 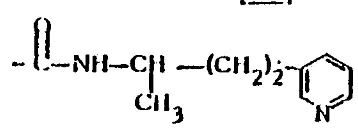 | C | 44 | 156-159 | $CH_3CN$ | $C_{25}H_{26}N_4O_2$ |
| 34 | $(CH_3)_2CH-$ | H | 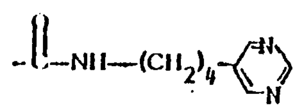 | D | 64 | 170-171 | $CH_3CN$-$CH_3OH$ | $C_{24}H_{25}N_5O_2$ |
| 35 | $(CH_3)_2CH-$ | H | 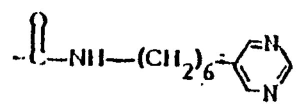 | D | 63 | 132-134 | $CH_3CN$ | $C_{26}H_{29}N_5O_2$ |

Beispiele 5-48 (Fortsetzung)

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 36 | $CH_3$- | $CH_3$- | 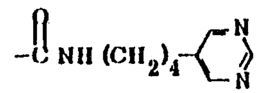 | E | 55 | 205-207 | $CHCl_3$ | $C_{23}H_{23}N_5O_2$ |
| 37 | $(CH_3)_2CH$- | H | 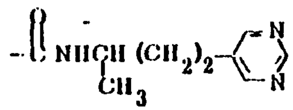 | C | 33 | 204-206 | EtOH-$Et_2O$ | $C_{24}H_{25}N_5O_2$ |
| 38 | HO- | H | 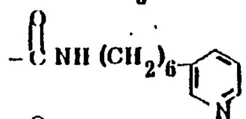 | D | 43 | 211-212 | DMF | $C_{24}H_{24}N_4O_3$ |
| 39 | HO- | H | 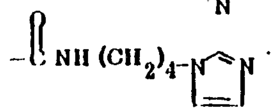 | D | 58 | 320-322 | DMF | $C_{20}H_{19}N_5O_3$ |
| 40 | $(CH_3)_2CH$- | H | 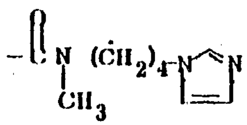 | C | 32 | 202-204 | EtOH-$Et_2O$ | $C_{24}H_{27}N_5O_2 \cdot 2\ HCl$ 0.5 $H_2O$ |
| 41 | $(CH_3)_2CH$- | H | 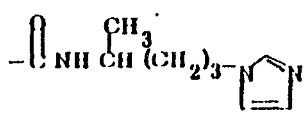 | C | 41 | 226-228 | MeOH $CH_3CN$ | $C_{24}H_{27}N_5O_2 \cdot 2\ HCl$ |
| 42 | $(CH_3)_2CH$- | H | 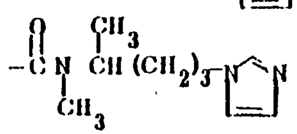 | C | 9 | 235-239 | MeOH-$Et_2O$ | $C_{25}H_{29}N_5O_2 \cdot 2\ HCl$ |

Beispiele 5-48 (Fortsetzung)

| Beispiel | $R_1$ | $R_2$ | $R_3$ | Verfahren | Ausbeute in % | Smp. in °C | Umkristallisiert aus | Summenformel |
|---|---|---|---|---|---|---|---|---|
| 43 | $(CH_3)_2CH-$ | H | 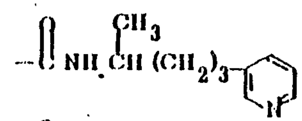 | C | 21 | 95-96 | EtOAC-Hex | $C_{26}H_{28}N_4O_2$ |
| 44 | $(CH_3)_2CH-$ | H | 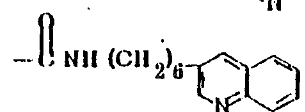 | D | 71 | 140-141.5 | $CH_3CN$ | $C_{31}H_{32}N_4O_2$ |
| 45 | $(CH_3)_2CH-$ | H | 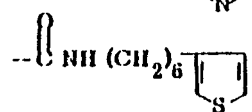 | D | | | | $C_{26}H_{29}N_3O_2S$ |
| 46 | $CH_3-$ | $CH_3-$ | 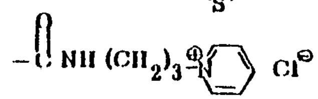 | B | 58 | 253-257 dec. | EtOH | $C_{23}H_{23}Cl\,N_4O_2 \cdot 0.5\,H$ |
| 47 | $CH_3-$ | $CH_3-$ | 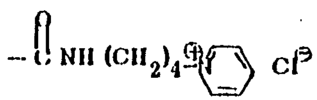 | B | 26 | 245-247 dec. | EtOH | $C_{24}H_{25}Cl\,N_4O_2 \cdot 2\,H_2O$ 1.2 HCl |
| 48 | $CH_3-$ | $CH_3-$ | 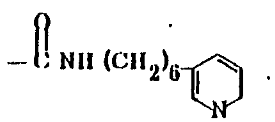 | B | 58 | 253-257 dec. | EtOH | $C_{26}H_{28}N_4O_2 \cdot 2\,HCl$ |

## Beispiel 49

Eine Suspension von 0,7 g 2-(1-Methyläthyl)-11-oxo-11H-pyrido [2,1-b] chinazolin-8-carbonsäure, 0,5 g 4-(3-Pyridyl)-butylchlorid, 0,4 g Kaliumjodid und 0,39 g Kaliumcarbonat in 12 ml Dimethylformamid wird über Nacht in einem 50° warmen Bad gerührt. Man verdünnt das Reaktionsgemisch mit Essigester, wäscht mit Wasser, trocknet über Kaliumcarbonat und konzentriert. Den Rückstand chromatographiert man an 75 g Kieselgel unter Eluieren mit Essigester. Man erhält 0,46 g 2-(1-Methyläthyl)-11-oxo-11H-pyrido [2,1-b] chinazolin-8-carbonsäure-4-(3-pyridyl) butylester als gelben Festkörper. Nach Umkristallisieren aus Essigester/Hexan erhält man 0,33 g (32 %) des Produktes vom Schmelzpunkt 95-96°.

## Beispiel 50

Eine Mischung aus 2,2 g 2,3-Dimethyl-11-oxo-N-[4-(3-pyridinyl) butyl]-11H-pyrido [2,1-b] chinazolin-8-carboxamid und 10 ml Jodmethan wird während 3 Tagen unter Argon und bei Raumtemperatur gerührt. Man entfernt das überschüssige Jodmethan und erhält ein orange-farbenes, gummiartiges Produkt, das man aus Methanol kristallisiert. Man erhält 2,3 g (77,2 %) 3-[4-[[(2,3-Dimethyl-11-oxo-11H-pyrido [2,1-b]-chinazolin-8-yl) carbonyl] amino] butyl]-1-methylpyridinium-jodid als orange-farbene Kristalle vom Schmelzpunkt 129° (Zersetzung).

## Beispiel 51

Man verteilt insgesamt 76,4 g 3-(3-Pyridyl) propyl-bromid-hydrochlorid zwischen wässriger Natronlauge und 200 ml Aether. Man trocknet die Aetherphase über Kalium-carbonat, verdünnt mit 250 ml Dimethylformamid, das 60 g Kaliumphthalimid enthält, und erhöht schrittweise die Temperatur, so dass der Aether abdestilliert. Die Badtemperatur wird schliesslich auf 130° erhöht und über Nacht bei dieser Temperatur gehalten. Man versetzt dann mit einer zusätzlichen Portion von 24 g Kaliumphthalimid und erhitzt noch während 18 Stunden. Man verteilt das erhaltene Gemisch zwischen Aether und Wasser, trocknet die ätherische Phase über kaliumcarbonat und dampft zu einem Festkörper ein, den man auf Methylenchlorid/Hexan kristallisiert. Man erhält 22 g (30 %) 2-[3-(3-Pyridyl) propyl]-1H-isoindol-1,3 (2H)-dion vom Schmelzpunkt 88-89°. Auf Methylenchlorid/Hexan erhält man eine analytische Probe vom Schmelzpunkt 89-91°.

## Beispiel 52

Man erhitzt eine Mischung aus 11,3 g 2-[3-(3-Pyridyl)-propyl]-1H-isoindol-1,3 (2H)-dion und 10,1 g Hydrazinhydrat in 200 ml Aethanol während 6 Stunden unter Rückfluss zum Sieden, lässt abkühlen und filtriert. Das Filtrat wird konzentriert, worauf man den Rückstand destilliert. Man erhält 4,77 g (70 %) 3-Pyridinpropanamin vom Siedepunkt 84-86°/0,35 mm.

## Beispiel 53

Man überschichtet eine gerührte Lösung von 1 040 g 3-(3-Pyridyl) propylbromid-hydrochlorid in 1 l Eis und Wasser mit 2 l Aether und neutralisiert durch langsame Zugabe von 5M Kalilauge auf pH 7. Während der Neutralisierung hält man die Temperatur durch portionenweise Zugabe von Eis bei 0°. Man trennt die Schichten, extrahiert die wässrige Phase zweimal mit je 2 l Aether und trocknet die vereinigten Aetherauszüge kurz über natriumsulfat. Man konzentriert unter vermindertem Druck bei einer Temperatur von 30° und erhält 760 g der freien Base 3-(3-Pyridyl)-propylbromid.

Man löst das obige Rohprodukt in 3 l Aethanol und versetzt in einer Portion mit einer Lösung von 361 g Kaliumcyanid in 2 l Wasser. Die erhaltene Mischung wird während 8 Stunden unter Rückfluss zum Sieden erhitzt, abgekühlt und konzentriert, um überschüssiges Aethanol zu entfernen. Den wässrigen Rückstand sättigt man mit Natriumchlorid und extrahiert viermal mit je 4 l Methylenchlorid. Die vereinigten Auszüge werden mit halbgesättigter Kochsalzlösung gewaschen, über Natriumsulfat getrocknet und eingedampft. Man erhält 385 g eines schwarzen Oeles, das man destilliert. Man erhält 335 g (63 %) 3-Pyridinbutannitrile vom Siedepunkt 110-115°/0,3 mm.

## Beispiel 54

Durch Hydrierung von 78,6 g 3-Pyridinbutannitril, das man in 850 ml Methanol und 20 ml Methanol und 20 ml Triäthylamin gelöst hat, über 20 g Raney-Cobalt bei einem Wasserstoffdruck von 1 000 psi und bei einer Temperatur von 100° während 2,5 Stunden erhält man 3-Pyridinbutanamin. Das nach Filtrieren und Konzentrieren erhaltene Produkt wird destilliert, wobei man 69,8 g (86 %) 3-Pyridinbutanamin vom Siedepunkt 118-120°/1 mm erhält. Das entsprechende Dihydrochlorid kristallisiert aus Aethanol'Aether und hat einen Schmelzpunkt von 133-134°.

Beispiel 55

Man versetzt eine Lösung von Natriumäthoxid, erhalten aus 9,2 g Natrium in 200 ml Aethanol, mit 32 g Diäthylmalonat und 56 g 3-(3-Pyridyl) propylbromid-hydrobromid in 30 ml Aethanol. Man erhitzt das erhaltene Gemisch während 1 Stunde unter Rückfluss zum Sieden, kühlt ab und konzentriert. Den Rückstand behandelt man mit Aether, dampft den Aether ab und reinigt das erhaltene Rohprodukt (53,2 g) mittels präparativer Flüssigkeitschromatographie über Kieselgel unter Eluieren mit Essigester/Hexan (1 : 1). Man erhält 36 g (65 %) des entsprechenden Malonesters. Dieses Material wird mit 200 ml 20-proz. Salzsäure gelöst, worauf man über Nacht unter Rückfluss zum Sieden erhitzt. Man entfernt das Lösungsmittel unter vermindertem Druck und kristallisiert den Rückstand aus Isopropanol. Man erhält 17,7 g (41 %) 3-Pyridinvaleriansäure-hydrochlorid vom Schmelzpunkt 144-146°.

Beispiel 56

3-Pyridinvaleramid wurde wie weiter unten für 3-Pyridinhexanamid beschrieben durch Umsetzen des Säurechlorides von 3-Pyridinvaleriansäure mit Ammoniumhydroxid hergestellt. Aus 15 g des Säurechlorides von 3-Pyridinvaleriansäure erhält man 8,85 g (70 %) 3-Pyridinvaleramid vom Schmelzpunkt 72-75°. Eine analytische Probe erhält man aus Essigester/Hexan, Schmelzpunkt 77-78°.

Beispiel 57

3-Pyridinpentanamin erhält man wie weiter unten für 3-Pyridinhexanamin beschrieben durch Boranreduktion von 3-Pyridinvaleramid. Aus 7,7 g 5-3-Pyridinvaleramid erhält man 5,6 g (80 %) 3-Pyridinpentanamin vom Siedepunkt 100-108°/0,2 mm.

Beispiel 58

Man versetzt eine Suspension von 150 g 4-(Carboxy)-butyltriphenylphosphoniumbromid in 400 ml Dimethylsulfat portionenweise mit 33,6 g einer 50-proz. Dispersion von Natriumhydrid in Mineralöl. Die Badtemperatur wurde dabei schrittweise auf 75-85° erhöht und während 30 Minuten bei dieser Temperatur gehalten, wobei die Mischung sich dunkelrot färbt. Die interne Temperatur wird auf 15-19° gesenkt, worauf 32,1 g Nicotinaldehyd über einen Zeitraum von 3 Minuten zugegeben werden. Man rührt das Reaktionsgemisch während 3 Stunden bei Raumtemperatur, verdünnt mit 1,5 l Wasser und wäscht dreimal mit je 300 ml Aether. Die wässrige Phase wird mit Salzsäure stark sauer gestellt, mit Methylenchlorid gewaschen und mit Natronlauge basisch gestellt. Man neutralisiert mit überschüssiger Essigsäure auf pH 5-6 und extrahiert viermal mit je 300 ml Methylenchlorid. Man trocknet die vereinigten Auszüge über Magnesiumsulfat und dampft ein, wobei man schliesslich noch im Hochvakuum bei einer Badtemperatur von 60° erwärmt, um das Dimethylsulfoxid und die Essigsäure zu entfernen. Der Rückstand wird aus Acetonitril/Aether umkristallisiert. Man erhält insgesamt 32,41 g (50 %) des gewünschten Olefins, das aus einer Mischung der Doppelbindungsisomeren besteht und zwischen 65 und 67° schmilzt.

Eine Lösung von 32,41 g des obigen Olefingemisches in 450 ml Isopropylalkohol wird sukzessive über 3,5 g, 4,0 g und 1,5 g 10-proz. Palladium auf Kohle hydriert, bis insgesamt 5 l Wasserstoff aufgenommen worden sind. Man filtriert die Reaktionsmischung und dampft ein. Man erhält 31,25 g (95 %) 3-Pyridinhexansäure vom Schmelzpunkt 100-105°. Durch Umkristallisieren aus Essigester/Hexan erhält man ein Produkt vom Schmelzpunkt 103-105°. Aus Isopropanol/Aether erhält man das Hydrochlorid, das einen Schmelzpunkt von 154-156° hat.

Beispiel 59

Man rührt eine Lösung von 14,19 g 3-Pyridinhexansäure in 42 ml Thionylchlorid bei Raumtemperatur über Nacht, dampft die Reaktionsmischung ein und versetzt zweimal mit je 50 ml Toluol, wobei man jeweils wieder eindampft. Den Rückstand gibt man tropfenweise zu 400 ml konzentriertem Ammoniak, das zwischen 0-5° gehalten wird. Man lässt die erhaltene Mischung während 4 Stunden auf Raumtemperatur erwärmen, verdünnt mit Wasser und extrahiert dreimal mit je 150 ml Methylenchlorid. Die vereinigten organischen Auszüge werden über Kaliumcarbonat getrocknet und konzentriert. Man erhält 11,75 g (83 %) 3-Pyridinhexanamid vom Schmelzpunkt 64-65°.

Beispiel 60

Man versetzt eine eiskalte Lösung von 16,27 g 3-Pyridinhexanamid in 250 ml trockenem Tetrahydrofuran mit 300 ml einer 1M-Lösung von Boran in Tetrahydrofuran. Man erhitzt die Mischung über Nacht unter Rückfluss zum Sieden, kühlt ab, versetzt mit Wasser und stellt mit 6N-Salzsäure stark sauer. Man lässt die saure Lösung über Nacht stehen, stellt mit Natronlauge basich und extrahiert dreimal mit je 150 ml Methylenchlorid. Die vereinigten organischen Auszüge werden über Kaliumcarbonat getrocknet und

eingedampt. Durch Destillieren des Rückstandes erhält man 10,2 g (68 %) 3-Pyridin-hexanamin vom Siedepunkt 140-150 %/0,2 mm.

Beispiel 61

4-Pyridinbutannitril wird wie für 3-Pyridinbutannitril beschrieben hergestellt. Aus 73,1 g des Hydrobromides von 3-(4-Pyridyl) propylbromid erhält man 19,35 g (51 %) 4-Pyridinbutannitril vom Siedepunkt 140-141 %/2,0 mm.

Beispiel 62

Eine Lösung von 30,0 g 4-Pyridinbutannitril in 200 ml Aethanol, das 7,5 ml Triäthylamin enthält, wird über 7,5 g Raney-Cobalt bei 100° und einem Wasserstoffdruck von 1 200 lbs. hydriert. Das nach Filtrieren und Konzentrieren erhaltene Rohprodukt wird destilliert, wobei man 9,7 g (31 %) 4-Pyridinbutanamin vom Siedepunkt 105-112 %/0,8 mm erhält. 15,3 g einer zweiten Fraktion (Siedepunkt 112-122°) bestand gemäss Dünnschichtchromatographie aus einer Mischung und wurde erneut destilliert. Man erhält dabei eine zweite Portion von 4,74 g (15 %) 4-Pyridinbutanamin vom Siedepunkt 95-98°/0,15 mm. Das aus Aethanol/Aether erhaltene Hydrochlorid schmilzt bei 124-125°.

Beispiel 63

4-Pyridinvaleriansäure wird wie für 3-Pyridinvaleriansäure beschrieben hergestellt. Aus 56 g des Hydrobromides von 3-(4-Pyridyl) propylbromid erhält man 10,4 g (24 %) 4-Pyridinvaleriansäure vom Schmelzpunkt 195-200°. Eine aus Isopropanol erhaltene analytische Probe hat einen Schmelzpunkt von 200-201°.

Beispiel 64

4-Pyridinvaleramid wird wie für 3-Pyridinvaleramid beschrieben hergestellt. Aus 9,3 g 4-Pyridinvaleriansäure erhält man 5,3 g (69 %) 4-Pyridinvaleramid vom Schmelzpunkt 123-125°. Eine aus Tetrahydrofuran/Hexan erhaltene analytische Probe hat einen Schmelzpunkt von 126-127°.

Beispiel 65

4-Pyridinpentanamid erhält man wie für 3-Pyridinhexanamin beschrieben aus 4-Pyridinvaleramid mittels Boranreduktion. Aus 5,2 g 4-Pyridinvaleramid erhält man 3,5 g (74 %) 4-Pyridinpentanamin vom Siedepunkt 140-150°/0,07 mm. Dieses Material wird ohne weitere Reinigung für die Herstellung von N-[5-(4-Pyridyl) pentyl]-2-(1-methyläthyl)-11-oxo-11H-pyrido [2,1-b] chinazolin-8-carboxamid verwendet.

Beispiel 66

Eine Lösung von 32, 72 g 4-Mercaptopyridin und 61 g 2-Bromäthanamin-hydrobromid in 200 ml absolutem Aethanol wird unter einer Argonatmosphäre während 2 Stunden unter Rückfluss zum Sieden erhitzt. Nach dem Abkühlen wird das ausgefallene Material abfiltriert und mit Aethanol gewaschen. Man erhält 76,3 g rohes 2-(4-Pyridylthio) äthanamindihydrobromid vom Schmelzpunkt 250-254°. Durch Umkristallisieren aus 1 500 ml Aethanol und 150 ml Wasser unter Behandlung mit Aktivkohle erhält man 63,09 g (68 %) 2-(4-Pyridylthio) äthanamin-dihydrobromid vom Schmelzpunkt 265-268°.

Beispiel 67

Eine Suspension von 16,0 g 4-Mercaptopyridin und 23,54 g 4-Brombutyronitril in 40 ml absolutem Alkohol wird unter einer Argonatmosphäre während 2 Stunden unter Rückfluss zum Sieden erhitzt. Man verdünnt die abgekühlte Reaktionsmischung mit 30 ml Aether, filtriert den ausgefallenen Festkörper ab und kristallisiert dieses aus Aethanol/Aether um. Man erhält 24,9 g (67 %) des Hydrobromides von 4-(4-Pyridylthio) butannitril vom Schmelzpunkt 150-154°. Durch Verteilen dieses Salzes zwischen Methylenchlorid und 3N-Natronlauge und Destillieren des durch Eindampfen der Methylenchloridlösung erhaltenen Rückstandes erhält man 16,16 g der freien Base vom Siedepunkt 185°/0,1 mm.

Beispiel 68

Eine Lösung von 14,35 g 4-(4-Pyridylthio) butannitril in 280 ml 1M-Boran in Tetrahydrofuran wird über Nacht bei Raumtemperatur gerührt. Überschüssiges Reagens wird dann durch vorsichtige Zugabe von 100 ml Methanol zersetzt. Man dampft das Reaktionsgemisch zur Trockene ein, verdünnt mit 200 ml Methanol und dampft ein. Man verdünnt mit 100 ml 6N-Salzsäure und lässt während 72 Stunden bei Raumtemperatur stehen. Das erhaltene Gemisch wird mit 50-proz. Natronlauge stark basisch gestellt und

viermal mit je 150 ml Methylenchlorid extrahiert. Die vereinigten organischen Auszüge werden über Kaliumcarbonat getrocknet und eingedampft. Durch Destillieren des Rückstandes erhält man 12,62 g (86 %) 4-(4-Pyridylthio) butanamin vom Siedepunkt 180°/0,1 mm.

Beispiel 69

Eine Suspension von 17,0 g Imidazol, 25,0 g 4-Brombutyrolnitrile und 34,5 g Kaliumcarbonat in 60 ml Methyläthylketon wird während 18 Stunden unter Rückfluss zum Sieden erhitzt. Man verdünnt die Reaktionsmischung mit wässriger Natronlauge und extrahiert dreimal mit je 100 ml Methylenchlorid. Die vereinigten Auszüge werden über Kaliumcarbonat getrocknet und eingedampft, worauf man den Rückstand destilliert. Nach einem Vorlauf von Imidazol erhält man 11,3 g (50 %) (1H-Imidazol-1-yl) butannitril vom Siedepunkt 170°/0,1 mm.

Beispiel 70

Eine Lösung von 10,8 g (1H-Imidazol-1-yl) butannitril in 225 ml Methanol und 2,5 ml Triäthylamin wird über 2,5 g Raney-Cobalt bei 90° und einem Wasserstoffdruck von 1 000 psi hydriert. Das nach Filtrieren und Konzentrieren erhaltene Rohprodukt wird destilliert. Man erhält 7,8 g (70 %) (1H-Imidazol-1-yl) butanamin vom Siedepunkt 100-103°/0,1 mm. Das entsprechende Dihydrochlorid kristallisiert aus Aethanol und hat einen Schmelzpunkt von 139-141°.

Beispiel 71

Eine Mischung aus 13 g 2-Chlornicotinsäure, 9,9 g 5-Isopropylanthranilsäure und 0,1 g Kaliumbromid in 10 ml Triglym wird schrittweise auf eine Badtemperatur von 140° erhitzt. Nach 2 Stunden ist ein orange-farbener Niederschlag entstanden. Man lässt die Reaktionsmischung abkühlen, versetzt mit 10 ml Aethanol und filtriert das Rohprodukt ab. Dieses Material wird aus Essigsäure/Wasser kristallisiert. Man erhält 9,3 g eines Festkörpers vom Schmelzpunkt 173-193°, das man in 100 ml Essigsäure aufnimmt. Man erhitzt während 1 Stunde unter Rückfluss zum Sieden, dampft das Lösungsmittel ab und kristallisiert den erhaltenen Rückstand aus Isopropylalkohol. Man erhält 5,8 g (37 %) 2-(1-Methyläthyl)-11-oxo-11H-pyrido [2,1-b] chinazolin-6-carbonsäure vom Schmelzpunkt 206-210°. Eine analytische Probe hat einen Schmelzpunkt von 221-223°.

Beispiel 72

Eine Lösung von 1,5 g 8-(1-Methyläthyl)-11-oxo-11H-pyrido [2,1-b] chinazolin-2-carbonsäure, 0,8 g 3-Pyridin-butanamin und 1,5 g Diphenylphosphorylazid in 22 ml trockenem Dimethylformamid wird in einem Eisbad gekühlt, worauf man die Reaktionsmischung in einer Portion mit 0,73 ml Triäthylamin versetzt. Man lässt die Reaktionsmischung auf Raumtemperatur erwärmen und über Nacht stehen, verdünnt mit Wasser und extrahiert mit Methylenchlorid. Die vereinigten organischen Auszüge werden über gesättigter Natriumbicarbonatlösung gewaschen, über Kaliumcarbonat getrocknet und eingedampft. Das erhaltene Oel chromatographiert man an 120 g Kieselgel unter Eluieren mit einem Lösungsmittelgemisch, bestehend aus 1 % Triäthylamin, 2 % Aethanol und 97 % Essigester. Das erhaltene Produkt wird mit äthanolischer Salzsäure sauer gestellt und aus Isopropanol/Aether umkristallisiert. Man erhält 1,87 g (72 %) 8-(1-Methyläthyl)-N-[4-(3-pyridyl) butyl]-11-oxo-11H-pyrido [2,1-b] chinazolin-2-carboxamid-dihydrochlorid vom Schmelzpunkt 247-249°.

Beispiel 73

Eine Suspension von 20 g 2-(1-Methyläthyl)-11-oxo-11H-pyrido [2,1-b] chinazolin-8-carbonsäure, 6,3 ml Chloracetonitril und 13,2 g Kaliumcarbonat in 100 ml Dimethylformamid wird während 18 Stunden bei Raumtemperatur gerührt. Man verdünnt die Reaktionsmischung mit 200 ml Wasser und filtriert das ausgefallene Produkt ab. Man erhält 21,63 g (95 %) 2-(1-Methyläthyl)-11-oxo-11H-pyrido [2,1-b] chinazolin-8-carbonsäure-cyanomethylester vom Schmelzpunkt 186-188°.

Beispiel 74

Eine Lösung von 2,82 g 2-(1-Methyläthyl)-11-oxo-11H-pyrido [2,1-b] chinazolin-6-carbonsäure, 2,75 g Diphenyl-phosphorylazid und 1,5 g 3-Pyridinbutanamin in 30 ml trockenem Dimethylformamid wird in einem Eisbad gekühlt, worauf man mit 1,0 g Triäthylamin versetzt. Man lässt die Reaktionsmischung auf Raumtemperatur erwärmen und während 18 Stunden stehen, verdünnt mit Wasser und extrahiert mit Methylenchlorid. Die vereinigten organischen Auszüge werden mit verdünnter Natriumcarbonatlösung gewaschen, über Kaliumcarbonat getrocknet und zur Trockene eingedampft. Der Rückstand wird wiederholt aus Essigester umkristallisiert, wobei man 0,25 g (6 %) 2-(1-Methyläthyl)-N-[4-(3-pyridyl)-butyl]-11-oxo-11H-pyrido [2,1-b] chinazolin-6-carboxamid vom Schmelzpunkt 129-130° erhält.

Beispiel 75

Eine Suspension von 0,6 g 8-(1-Methyläthyl)-11-oxo-11H-pyrido [2,1-b] chinazolin-2-carbonsäure, 0,63 g 3-(3-Pyridyl)-propylbromid-hydrobromid und 0,62 g Kaliumcarbonat in 50 ml Dimethylformamid wird während 3 Stunden bei einer Badtemperatur von 70° gerührt. Man verdünnt das Reaktionsgemisch mit Methylenchlorid und wäscht nacheinander mit Wasser, 10 % Natronlauge und Wasser und trocknet über Kaliumcarbonat. Nach Eindampfen erhält man 0,40 g 8-(1-Methyläthyl)-11-oxo-11H-pyrido [2,1-b] chinazolin-2-carbonsäure-3 (3-pyridyl) propylester vom Schmelzpunkt 111-112°.

Beispiel 76

Man löst 34,1 g Natriumhydroxid unter einer Argonatmosphäre in 500 ml Aethanol und versetzt unter Kühlen mit einem Eisbad mit 48,6 g 2-Mercaptoäthanamin-hydrochlorid und 35 g 4-Chlormethylpyridinhydrochlorid. Man lässt während 2 Stunden auf Raumtemperatur erwärmen, konzentriert, verdünnt mit 300 ml Wasser und extrahiert dreimal mit je 150 ml Methylenchlorid. Die vereinigten organischen Auszüge werden mit 100 ml gesättigter Kochsalzlösung gewaschen, über Kaliumcarbonat getrocknet und zu einem dunklen Oel eingedampft, den man destilliert. Die Fraktionen zwischen 120-130°/0,1 mm lieferten 30,68 g (85 %) 2-[[(4-Pyridyl)-methyl] thio] äthanamin.

Beispiel 77

Man löst 16,8 g Natriumhydroxid in 350 ml Aethanol und versetzt unter Kühlen in einem Eisbad mit 17,2 g 3-Chlor-methylpyridin-hydrochlorid und 23,7 g 2-Mercaptoäthanaminhydrochlorid. Man lässt während 2 Stunden auf Raumtemperatur erwärmen, konzentriert, verdünnt mit Wasser und extrahiert dreimal mit je 100 ml Methylenchlorid. Man wäscht die vereinigten organischen Auszüge mit gesättigter Kochsalzlösung, trocknet über Kaliumcarbonat und konzentriert. Durch Destillieren des Rückstandes erhält man 23,3 g (70 %) 2-[[(3-Pyridyl) methyl] thio] äthanamin vom Siedepunkt 115-125°/0,1 mm.

Beispiel 78

Man löst 12,8 g Natriumhydroxid in 12 ml Wasser und verdünnt mit 150 ml Dimethylformamid, das 20,3 g 2-Methylimidazol enthält. Dazu gibt man langsam eine Lösung von 50 g 4-Brombutyronitril in 100 ml Dimethylformamid, wobei man die Temperatur unterhalb 30° hält. Das erhaltene Gemisch wird über Nacht gerührt und dann konzentriert. Den Rückstand nimmt man in 150 ml Kochsalzlösung auf und extrahiert mit Methylenchlorid. Die vereinigten organischen Auszüge werden über Kaliumcarbonat getrocknet und eingedampft, worauf man den Rückstand destilliert. Die ersten Fraktionen bestehen vorwiegend aus 2-Methylimidazol und werden verworfen. Die Hauptfraktion wird zwischen 160 und 185°/2,5 mm gesammelt und liefert 23,2 g des gewünschten Produktes. Durch erneute Destillation erhält man 14,7 g (31 %) (2-Methyl-1H-imidazol-1-yl) butannitril vom Siedepunkt 140-147°/0,5 mm.

Beispiel 79

Eine Lösung von 14,7 g (2-Methyl-1H-imidazol-1-yl)-butannitril in 3 ml Triäthylamin und 200 ml Methanol wird über 3 g Raney-Cobalt bei 90° und einem Wasserstoffdruck von 1 000 lbs hydriert. Das nach Filtrieren und Eindampfen erhaltene Produkt wird destilliert. Man erhält 10,6 g (70 %) (2-Methyl-1H-imidazol-1-yl) butanamin vom Siedepunkt 105-110°/0,5 mm.

Beispiel 80

Eine Lösung von 12,0 g 4-(3-Pyridinyl)-2-butanon-hydrochlorid, 3,38 g Natriumcyanoborhydrid und 42,78 g Ammonium-acetat in 200 ml Methanol wird während 2,5 Tagen bei Raumtemperatur gerührt, mit verdünnter Salzsäure angesäuert und konzentriert. Den Rückstand nimmt man in wässriger Kaliumcarbonatlösung auf und extrahiert mit Methylenchlorid. Der nach Eindampfen der Methylenchloridlösung erhaltene Rückstand liefert nach Destillation 6,5 g (80 %) α-Methyl-3-pyridinpropanamin vom Siedepunkt 126-148°/0,3 mm. Das entsprechende Picrat kristallisiert aus Methanol/Aether und hat einen Schmelzpunkt von 146-148°.

Beispiel 81

4-(5-Pyrimidinyl)-3-butyn-1-ol wird hergestellt, indem man wie in Beispiel 70 für die Herstellung von 6-(5-Pyrimidinyl)-5-hexyn-1-ol verfährt. Aus 20 g 5-Brompyrimidin erhält man 20,3 g rohes 4-(5-Pyrimidinyl)-3-butyn-1-ol.

Beispiel 82

Eine Lösung von 18,5 g 4-(5-Pyrimidinyl)-3-butyn-1-ol in 300 ml Isopropanol wird über insgesamt 3,5 g

10-proz. Palladium auf Kohle, das man in drei Portionen hinzugibt, hydriert. Nach Eindampfen der Lösung und Destillation des Rückstandes erhält man 15,2 g (80 %) 5-Pyrimidinbutanol vom Siedepunkt 175-180°/0,5 mm.

Beispiel 83

2-[4-(5-Pyrimidinyl) butyl]-1H-isoindol-1,3 (2H)-dion wird hergestellt, indem man wie in Beispiel 87 für die Herstellung von 2-[6-(5-Pyrimidinyl) hexyl]-1H-isoindol-1,3-(2H)-dion verfährt. Aus 14,8 g 5-Pyrimidinbutanol erhält man 16,3 g (60 %) 2-[4-(5-Pyrimidinyl) butyl]-1H-1,3(2H)-dion vom Schmelzpunkt 148-149°.

Beispiel 84

5-Pyrimidinbutanamin wird wie in Beispiel 88 für die Herstellung von 5-Pyrimidinhexanamin beschrieben hergestellt. Aus 15,8 g 2-[4-(5-Pyrimidinyl) butyl]-1H-isoindol-1,3(2H)-dion erhält man 7,4 g (87 %) 5-Pyrimidinbutanamin vom Siedepunkt 140-150°/0,3 mm. Das entsprechende Picrat kristallisiert aus Aethanol und hat einen Schmelzpunkt von 183-184°.

Beispiel 85

Eine Lösung von 23,8 g 5-Brompyrimidin und 17,7 g 5-Hexyn-1-ol in 59,5 ml Triäthylamin und 240 ml Methylenchlorid wird mit Argon behandelt und dann mit 200 mg Kupfer (I) jodid und 2,1 g bis(Triphenylphosphin) palladiumchlorid versetzt. Man erhitzt die erhaltene Mischung während 3 Stunden unter Rückfluss zum Sieden, verdünnt mit Methylenchlorid, wäscht mit Wasser und dampft ein. Man erhält 31,7 g Rohprodukt, das man mittels Chromatographie an 400 g Kieselgel unter Eluieren mit Essigester reinigt. Man erhält 26,2 g 6-(5-Pyrimidinyl)-5-hexyn-1-ol.

Beispiel 86

Eine Lösung von 25,3 g 6-(5-Pyrimidinyl)-5-hexin-1-ol in 300 ml Isopropanol wird über insgesamt 2,1 g 10-proz. Palladium auf Kohle, das man in zwei Portionen dazugibt, hydriert. Das so erhaltene Material wird destilliert und liefert 22,8 g (88 %) 5-Pyrimidinhexanol vom Siedepunkt 185-196°/0,35 mm.

Beispiel 87

A. Eine Lösung von 20,6 g 5-Pyrimidinhexanol in 80 ml Methylenchlorid wird in einem Eisbad abgekühlt, worauf man tropfenweise eine Lösung von 12,3 ml Thionylchlorid in 40 ml Methylenchlorid dazugibt. Man rührt die Mischung während 1 Stunde bei Raumtemperatur und erhitzt während 1 Stunde unter Rückfluss zum Sieden. Anschliessend wird konzentriert, mit Toluol versetzt und erneut eingedampft. Man nimmt den Rückstand in Methylenchlorid auf, wäscht mit Wasser, gesättigter Natriumbicarbonatlösung und Kochsalzlösung und dampft ein. Man erhält 22,3 g 6-(5-Pyrimidinyl)-hexylchlorid.

B. Eine Lösung von 22,3 g 6-(5-Pyrimidinyl) hexylchlorid, 41,6 g Kaliumphthalimid und 1,8 g Kaliumjodid in 180 ml Dimethylformamid wird während 1 Stunde in einem 130° heissen Bad erhitzt. Man verdünnt die Mischung mit Wasser, extrahiert mit Methylenchlorid, wäscht die vereinigten organischen Auszüge mit Wasser und trocknet sie über Kaliumcarbonat. Der nach Eindampfen erhaltene Rückstand wird aus Essigester/Hexan kristallisiert und liefert 24,7 g (71 %) 2-[6-(5-Pyrimidinyl) hexyl]-1H-isoindol-1,3(2H)-dion vom Schmelzpunkt 124-126,5°.

Beispiel 88

Eine Lösung von 23,2 g 2-[6-(5-Pyrimidinyl) hexyl]-1H-isoindol-1,3(2H)-dion und 14,6 ml Hydrazinhydrat in 320 ml Aethanol wird während 3 Stunden unter Rückfluss zum Sieden erhitzt. Die abgekühlte Mischung wird filtriert, worauf man das Filtrat konzentriert. Man nimmt den Rückstand in Methylenchlorid auf, wäscht mit 5-proz. Natronlauge, trocknet und dampft ein. Das erhaltene Oel wird destilliert und liefert 12,0 g (90 %) 5-Pyrimidinhexanamin vom Siedepunkt 120-128°/0,6 mm.

Beispiel 89

A. Eine Mischung aus 50 g 6-Bromhexansäure, 88 g Benzylbromid und 69 g Kaliumcarbonat in 500 ml Dimethylformamid wird über Nacht bei Raumtemperatur gerührt. Man filtriert, konzentriert, nimmt den erhaltenen Rückstand in Aether auf, wäscht mit Wasser und trocknet über Natriumsulfat. Der nach Eindampfen erhaltene Rückstand wird destilliert und liefert 63 g (85 %) Benzyl-6-bromhexanoat.

B. Zu einer Suspension von 9,74 g 57% Natriumhydrid in 100 ml Dimethylformamid gibt man eine Lösung von 15,0 g Imidazol in 500 ml Dimethylformamid, und zwar bei einer Temperatur von 15-25°. Man erhitzt die erhaltene Mischung während 1 Stunde auf 60°, kühlt auf Raumtemperatur ab und versetzt mit

62,8 g Benzyl-6-bromhexanoat. Man erhitzt die Mischung während 2 Stunden auf 60°, lässt über Nacht bei Raumtemperatur stehen und dampft ein. Den Rückstand nimmt man in wässriger Salzsäure auf, wäscht mit Aether, stellt mit Natronlauge basisch und extrahiert mit Methylenchlorid. Die vereinigten organischen Auszüge werden getrocknet und eingedampft. Durch Destillieren des Rückstand erhält man 45,1 g (75 %) Benzyl-(1H-imidazol-1-yl) hexanoat.

Beispiel 90

Eine Lösung von 45,1 g Benzyl-(1H-imidazol-1-yl)-hexanoat in 200 ml Isopropanol wird über 10-proz. Palladium auf Kohle hydriert. Durch Filtrieren und Eindampfen erhält man 24,9 g (80 %) (1H-Imidazol-1-yl) hexansäure vom Schmelzpunkt 135-137°.

Beispiel 91

A. Eine Lösung von 10,4 g (1H-Imidazol-1-yl) hexansäure-hydrochlorid in 50 ml Thionylchlorid wird während 2 Stunden bei Raumtemperatur gerührt und dann eingedampft. Den Rückstand nimmt man in Toluol auf und dampft ein, worauf man diese Prozedur wiederholt. Man gibt den Rückstand zu eiskaltem Ammoniak. Nach 18 Stunden wird eingedampft, und der Rückstand in Wasser aufgenommen. Man extrahiert mit Methylenchlorid und erhält nach Eindampfen 6,0 g eines Festkörpers, den man aus Methylenchlorid/Hexan kristallisiert. Man erhält 3,9 g (45 %) (1H-Imidazol-1-yl) hexanamid vom Schmelzpunkt 90-91°.

B. Eine Lösung von 3,9 g (1H-Imidazol-1-yl) hexanamid in 75 ml Tetrahydrofuran wird in einem Eisbad abgekühlt, worauf man 75 ml 1M-Boran in Tetrahydrofuran dazugibt. Die erhaltene Mischung wird über Nacht unter Rückfluss zum Sieden erhitzt, mit Wasser und verdünnter Salzsäure verdünnt und über Nacht stehengelassen. Man stellt die Mischung mit 50-proz. Natronlauge basisch und extrahiert mit Methylenchlorid. Der nach Eindampfen erhaltene Rückstand wird destilliert und liefert 3,1 g (86 %) (1H-Imidazol-1-yl)-hexanamin vom Siedepunkt 145-155°/0,1 mm.

Beispiel 92

A. Eine Lösung von 26,6 g 3-Pyridinhexansäure in 200 ml trockenem Tetrahydrofuran wird in einem Eisbad abgekühlt und mit 488 ml einer 1M-Lösung von Boran in Tetrahydrofuran langsam versetzt. Die Reaktionsmischung wird schrittweise auf Rückflusstemperatur erhitzt und über Nacht unter Rückfluss zum Sieden erhitzt. Das überschüssige Reagens wird durch vorsichtige Zugabe von 100 ml Methanol zersetzt. Man konzentriert das Reaktionsgemisch, verdünnt den Rückstand mit 6N-Salzsäure und lässt über Nacht stehen. Man stellt dann mit 50-proz. Natronlauge basisch und extrahiert mit Methylenchlorid. Die vereinigten organischen Auszüge werden über Kaliumcarbonat getrocknet und zu einem Oel eingedampft, das man destilliert. Man erhält 22,6 g (92 %) 6-(3-Pyridyl)-hexanol vom Siedepunkt 180-200°/0,2 mm.

B. Eine Lösung von 22 g 6-(3-Pyridyl) hexanol in 100 ml Chloroform wird in einem Eisbad abgekühlt und tropfenweise mit einer Lösung von 21,9 g Thionylchlorid in 50 ml Chloroform behandelt. Man lässt die Reaktionsmischung auf Raumtemperatur erwärmen und erhitzt nach 1 Stunde während 1 Stunde in einem 60° warmen Bad. Man konzentriert, nimmt den Rückstand in Chloroform auf, wäscht mit gesättigter Kaliumcarbonatlösung, trocknet und dampft ein. Der Rückstand wird destilliert und liefert 20,5 g (84 %) 6-(3-Pyridyl) hexylchlorid vom Siedepunkt 180-190°/0,15 mm.

C. Eine Mischung aus 4,0 g Kaliumcyanid, 0,85 g Kaliumjodid und 10,1 g 6-(3-Pyridyl) hexylchlorid in 100 ml Dimethylformamid wird über Nacht auf 100° erhitzt. Man verdünnt die Reaktionsmischung mit 200 ml Wasser, extrahiert mit Methylenchlorid, wäscht die vereinigten organischen Auszüge mit Wasser, trocknet diese über Kaliumcarbonat und dampft ein. Der Rückstand wird destilliert und liefert 8,1 g (84 %) 3-Pyridinheptannitril.

D. Eine Lösung von 7,1 g 3-Pyridinheptannitril in 100 ml Tetrahydrofuran wird in einem Eisbad gekühlt und mit 113 ml einer 1M-Lösung von Boran in Tetrahydrofuran behandelt. Nach 30 Minuten erhitzt man die Reaktionsmischung unter Rückfluss zum Sieden, kühlt nach 18 Stunden ab, behandelt mit 100 ml Methanol und dampft ein. Den Rückstand behandelt man mit 6N-Salzsäure und lässt über Nacht bei Raumtemperatur stehen. Anschliessend erhitzt man während 15 Minuten auf 95°, verdünnt dann mit Wasser und Kaliumcarbonat und extrahiert mit Methylenchlorid. Die organische Phase wird über Kaliumcarbonat getrocknet und eingedampft, wobei man 5,6 g eines Oeles erhält. Dieses Oel wird über Nacht mit Essigsäureanhydrid behandelt, worauf man mit Wasser verdünnt und mit Methylenchlorid extrahiert. Die vereinigten organischen Auszüge werden über Kaliumcarbonat getrocknet und eingedampft. Der Rückstand wird destilliert und liefert 5,8 g N-[7-(3-Pyridyl) heptyl] acetamid vom Siedepunkt 145-155°/0,1 mm.

E. Eine Lösung von 5,1 g N-[7-(3-Pyridyl) heptyl] acetamid wird in verdünnter Salzsäure über Nacht unter Rückfluss zum Sieden erhitzt. Die Mischung wird abgekühlt, mit 50-proz. Natronlauge basisch gestellt und mit Methylenchlorid extrahiert. Die vereinigten organischen Auszüge werden über Kaliumcarbonat getrocknet und eingedampft. Der Rückstand wird destilliert und liefert 3,4 g 3-Pyridinheptanamin vom Siedepunkt 175-180°/0,4 mm.

Beispiel 93

Eine Mischung aus 15,0 g 2,3-Dimethyl-11-oxo-11H-pyrido [2,1-b] chinazolin-8-carbonsäure, 10,2 g Kaliumcarbonat, 4,9 ml (5,8 g) Chloracetonitril und 150 ml N,N-Dimethylformamid wird während 18 Stunden auf 65° erhitzt. Die abgekühlte Reaktionsmischung wird auf 800 ml Wasser gegossen, und der erhaltene Festkörper wird abfiltriert. Dieser wird mit Wasser gewaschen und getrocknet, wobei man 14,4 g (84 %) des gewünschten Rohproduktes in Form gelber Kristalle vom Schmelzpunkt 175-179° (Zersetzung) erhält. Durch Umkristallisieren aus Acetonitril erhält man 8,7 g (51 %) einer analytischen Probe von [(2,3-Dimethyl-11-oxo-11H-pyrido [2,1-b] chinazolin-8-carbonsäure-cyanomethylester vom Schmelzpunkt 182-184° (Zersetzung).

Beispiel 94

Eine Suspension von 5 g 2-Hydroxy-11-oxo-11H-pyrido-[2,1-b] chinazolin-8-carbonsäure, 1,7 ml Chloracetonitril und 2,3 g Natriumbicarbonat in 70 ml Dimethylformamid wird während 12 Tagen bei Raumtemperatur gerührt. Man verdünnt die Reaktionsmischung mit Wasser und filtriert das ausgefallene Produkt ab. Man erhält 5,4 g Material vom Schmelzpunkt 260-262°, das man aus Dimethylformamid/Wasser umkristallisiert. Man erhält schliesslich 4,0 g (68 %) 2-Hydroxy-11-oxo-11H-pyrido [2,1-b] chinazolin-8-carbonsäure-cyanomethylester vom Schmelzpunkt 265-267°.

Beispiel 95

Eine Lösung von 16,5 g 3-Brompropanamin-hydrobromid und 6 ml (5,9 g) Pyridin in 75 ml Aethanol wird während 18 Stunden unter Rückfluss zum Sieden erhitzt. Man kühlt dann ab, filtriert und wäscht den Festkörper mit Aethanol, wobei man 17,0 g (76 %) 1-(3-Aminopropyl) pyridiniumbromidhydrobromid als farblose Kristalle vom Schmelzpunkt 178-179° erhält. Durch Umkristallisieren aus Methanol erhält man eine analytische Probe vom Schmelzpunkt 176-179°.

Beispiel 96

Eine Lösung von 17,4 g 4-Brombutanamin-hydrobromid und 6,0 ml (5,9 g) Pyridin in 75 ml Aethanol wird während 18 Stunden unter Rückfluss zum Sieden erhitzt. Nach Abkühlen wird das ausgefallene Material abfiltriert und aus Aethanol umkristallisiert. Man erhält 3,4 g (15 %) 1-(4-Aminobutyl) pyridiniumbromid-hydrobromid als farblose Kristalle vom Schmelzpunkt 219° (Zersetzung).

Beispiel 97

Eine Suspension von 29 g Imidazol und 23,1 g Natriummethoxid in 385 ml Dimethylformamid wird während 30 Minuten gerührt, in einem Eisbad abgekühlt und tropfenweise mit 85,8 g 3-(2-Methyl-1,3-dioxolan-2-yl) propylbromid versetzt. Die erhaltene Mischung wird während 30 Minuten bei Raumtemperatur gehalten und dann während 1 Stunde unter Rückfluss zum Sieden erhitzt. Man entfernt das Lösungsmittel im Vakuum, verdünnt den Rückstand mit Aceton und filtriert. Das Filtrat wird konzentriert, und der erhaltene Rückstand wird destilliert. Man erhält 37,9 g (47 %) 1-[2-(2-Methyl-1,3-dioxolan-2-yl) propyl]-1H-imidazol als farbloses Oel vom Siedepunkt 102-108°/0,1 mm.

Beispiel 98

Eine Lösung von 48,4 g 1-[3-(2-Methyl-1,3-dioxolan-2-yl) propyl]-1H-imidazol in 615 ml 1M-Salzsäure wird über Nacht gerührt. Die Reaktionsmischung wird auf die Hälfte des Volumens eingedampft, und zwar unter vermindertem Druck, und dann mit Natronlauge basisch gestellt. Man extrahiert mit Methylenchlorid, trocknet die vereinigten organischen Auszüge über Kaliumcarbonat und destilliert den nach Eindampfen erhaltenen Rückstand. Man erhält 30,17 g (77 %) 5-(1H-Imidazol-1-yl)-2-pentanon vom Siedepunkt 110-121°/0,1 mm. Durch Chromatographie an einer präparativen Kieselgelplatte unter Verwendung von 9-proz. Methanol/Methylenchlorid als Fliessmittel erhält man eine analytisch reine Probe.

Beispiel 99

Eine Lösung von 8,3 g 5-(1H-Imidazol-1-yl)-2-pentanon, 44 g Ammoniumacetat und 3,5 g Natriumcyanoborhydrid in 200 ml Methanol wird während 2,5 Tagen bei Raumtemperatur gerührt. Man stellt die Mischung mit Salzsäure sauer und konzentriert zur Trockene. Man nimmt den Rückstand in Kaliumcarbonatlösung auf und extrahiert mit Methylenchlorid. Die vereinigten Auszüge werden über Kaliumcarbonat getrocknet und eingedampft. Man destilliert den Rückstand und erhält 4,6 g (55 %) α-Methyl-1H-

imidazol-1-butanamin vom Siedepunkt 110-117°/0,25 mm.

Beispiel 100

Eine Lösung von 10 g 5-(1H-Imidazol-1-yl)-2-pentanon, 13 g Methylaminhydrochlorid und 4,2 g Natriumcyanoborhydrid in 200 ml Methanol wird während 3 Tagen gerührt. Die Reaktion wird dann durch Zugabe von verdünnter Salzsäure abgestoppt. Man dampft dann ein, verdünnt den Rückstand mit wässriger Kaliumcarbonatlösung und extrahiert mit Methylenchlorid. Die vereinigten organischen Auszüge werden über Kaliumcarbonat getrocknet und eingedampft. Man erhält 10,2 g eines Oeles, das man destilliert. Man erhält 9,0 g (90 %) N-α-Dimethyl-1H-imidazol-1-butanamin vom Siedepunkt 110-120°/0,1 mm. Das entsprechende Picrat hat einen Schmelzpunkt von 112-114°.

Beispiel 101

Eine Lösung von 53,2 g (1H-Imidazol-1-yl) butanamin in 820 ml Ameisensäure wird in einem Eisbad abgekühlt und tropfenweise mit 266 ml Essigsäureanhydrid versetzt. Man rührt die Reaktionsmischung während 3 Tagen, konzentriert im Vakuum und verteilt den Rückstand zwischen wässrigem Kaliumcarbonat und Methylenchlorid. Die organische Phase wird über Kaliumcarbonat getrocknet und eingedampft, worauf man den Rückstand destilliert. Man erhält 58,8 g (87 %) N-[4-(1H-Imidazol-1-yl) butyl] formamid vom Siedepunkt 165-180°/0,3 mm, das man direkt in die nächste Stufe einsetzt.

Beispiel 102

Eine Lösung von 58,8 g N-[4-(1H-Imidazol-1-yl) butyl]-formamid in 480 ml trockenem Tetrahydrofuran wird mit 87 ml 10M-Lösung von Boranmethylsulfid-Komplex in Tetrahydrofuran behandelt. Die Reaktionsmischung wird während 18 Stunden unter Argon und unter Rückfluss zum Sieden erhitzt, in einem Eisbad abgekühlt und mit 400 ml Methanol behandelt. Man entfernt das Lösungsmittel, versetzt mit Methanol und dampft erneut ein. Den Rückstand stellt man mit Salzsäure sauer, worauf man über Nacht rührt. Die erhaltene Mischung wird mit Natronlauge basisch gestellt und mit Methylenchlorid extrahiert. Die vereinigten organischen Auszüge werden über Kaliumcarbonat getrocknet und zu einem Oel eingedampft, das man durch Destillation reinigt. Man erhält 35,9 g (68 %) 4-(1H-Imidazol-1-yl)-N-methylbutanamin vom Siedepunkt 130-135°/0,3 mm. Das entsprechende Picrat hat einen Schmelzpunkt von 111-113°.

Beispiel 103

Eine Lösung von 31,8 g 5-Brompyrimidin, 20 g Natrium-bicarbonat und 21,65 g 3-Buten-1-ol in 150 ml Dimethylformamid wird zuerst mit Argon behandelt und dann während 30 Minuten mit 0,75 g Palladiumacetat versetzt. Man evakuiert das Reaktionsgefäss dreimal und füllt es jeweils mit Argon auf. Anschliessend wird die Badtemperatur auf 120° erhöht und während 4 Stunden bei dieser Temperatur belassen. Man verdünnt das Reaktionsgemisch mit 400 ml Essigester, filtriert, konzentriert das Filtrat und destilliert schliesslich den Rückstand. Man erhält 27,36 g eines Oeles vom Siedepunkt 100-110°/3 mm. Das Rohprodukt wird mit Salzsäure angesäuert und aus Aethanol/Aether kristallisiert. Man erhält 19,22 g (51 %) 4-(5-Pyrimidinyl)-2-butanonhydrochlorid vom Schmelzpunkt 103-106°.

Beispiel 104

Eine Lösung von 10 g 4-(5-Pyrimidinyl)-2-butanon-hydrochlorid, 42,5 g Ammoniumacetat und 3,37 g Natriumcyanoborhydrid in 200 ml Methanol wird während 2,5 Tagen bei Raumtemperatur gerührt, anschliessend angesäuert und konzentriert, worauf man den Rückstand in Wasser aufnimmt, mit verdünnter Natronlauge basisch stellt und mit Methylenchlorid extrahiert. Die vereinigten organischen Auszüge werden über Kaliumcarbonat getrocknet und eingedampft. Man erhält 7,5 g rac-α-Methyl-5-pyrimidinpropanamin als Oel. Man stellt das entsprechende Hydrochlorid her, das aus Aethanol/Aether kristallisiert. Man erhält 2,47 g rac-α-Methyl-5-pyrimidinpropanamin-hydrochlorid vom Schmelzpunkt 163-165°.

Beispiel 105

Ein getrockneter 250 ml-Drei-Halskolben wird mit einem Gaseinleitungsrohr versehen, das bis fast auf den Boden des Gefässes reicht. Man legt dann 9,81 ml 3-Brompyridin, 11,8 g 5-Hexin-1-ol, 40 ml destilliertes Triäthylamin und 60 ml Methylenchlorid vor, begast die Mischung mit Argon während 15 Minuten und versetzt dann mit 0,7 g Bis(Triphenylphosphin) palladiumdichlorid und 0,13 g Kupfer (U)-jodid. Das Gefäss wird dann dreimal evakuiert und jeweils mit Argon aufgefüllt. Man erhitzt dann während 2 Stunden unter Rückfluss zum Sieden, kühlt ab, verdünnt mit Methylenchlorid und wäscht nacheinander mit Wasser und Kochsalzlösung. Man trocknet über Kaliumcarbonat und konzentriert, wobei man 20 g

eines dunkelroten Oels erhält, das man ohne weitere Reinigung in die nächste Stufe einsetzt. Eine Probe dieses Materials wird an 70 g Kieselgel unter Eluieren mit Essigester/Hexan (1 : 1), das 1-proz. Triäthylamin enthält, chromatographiert und liefert ein farbloses Oel, das man destilliert. Man erhält schliesslich eine analytische Probe von 6-(3-Pyridinyl)-5-hexin-1-ol vom Siedepunkt 145-150°/0,1 mm.

Beispiel 106

6-(3-Thiophen)-5-hexin-1-ol wird in Analogie zu den Angaben in Beispiel 105 hergestellt, und zwar ausgehend von 16,0 g 3-Bromthiophen, 11,8 g 5-Hexin-1-ol, 3,0 g Bis-(Triphenylphosphin) palladiumdichlorid und 0,29 g Kupfer (I)-chlorid in 165 ml Methylenchlorid und 41 ml Triäthylamin. Das erhaltene Rohprodukt wird mittels Hochdruck-Flüssigkeits-Chromatographie unter Eluieren mit 9,75-proz. Methanol in Methylenchlorid gereinigt. Man erhält 8,9 g (48 %) Produkt als farbloses Oel, das man direkt weiterverarbeitet. Eine Probe dieses Materials wird an präparativen Kieselgelplatten chromatographiert, wobei man eine analytische Probe an 6-(3-Thiophen)-5-hexin-1-ol erhält.

Beispiel 107

3-Thiophenhexanol wird durch Reduktion von 8,4 g 6-(3-Thiophenyl)-5-hexin-1-ol in 100 ml Aethanol über 1 g 10-proz. Palladium/Kohle unter Atmosphärendruck hergestellt. Nach 8 Stunden wird eine zweite Portion an Katalysator dazugegeben und die Reduktion wird über Nacht fortgesetzt. Das nach Filtrieren und Eindampfen erhaltene Rohprodukt wird destilliert und liefert 6,9 g (80 %) 3-Thiophenhexanol vom Siedepunkt 135-147°/0,2 mm.

Beispiel 108

3-Pyridinhexanol wird durch Hydrieren von 20 g rohem 6-(3-Pyridinyl)-5-hexin-1-ol in 200 ml Isopropylalkohol über 2 g 10-proz. Palladium auf Kohle bei einem Druck von 1 Atmosphäre hergestellt. Das nach Filtrieren und Eindampfen erhaltene Rohprodukt wird destilliert und liefert 15,8 g (89 %) 3-Pyridinhexanol als gelbes Oel vom Siedepunkt 120-150°/0,2 mm, das gemäss gaschromatographischer Analyse einen Reinheitsgrad von 90 % aufweist.

Beispiel 109

Eine Suspension von 9,8 g 3-Pyridinhexylchlorid und 18,4 g Kaliumphthalimid in 130 ml Dimethylformamid wird in Gegenwart von 0,83 g Kaliumjodid in einem 130° warmen Bad erhitzt. Nach 1 Stunde lässt man Abkühlen, verdünnt mit Wasser und extrahiert mit Aether. Die vereinigten organischen Auszüge werden über Natriumsulfat getrocknet und zu einem rosa-farbenen Festkörper eingedampft. Durch Umkristallisieren aus Aethanol erhält man 9,5 g (62 %) 2-[6-(3-Pyridinyl) hexyl]-1H-isoindol-1,3 (2H)-dion als rosafarbenen Festkörper vom Schmelzpunkt 90-92°. Aus den Mutterlaugen kann eine weitere Portion von 3,15 g (21 %) des gewünschten Produktes mit einem Schmelzpunkt von 89-92° isoliert werden.

Beispiel 110

Eine Lösung von 81,9 g 2-[6-(3-Pyridinyl) hexyl]-1H-isoindol-1,3 (2H)-dion in 1,1 l Aethanol und 51,7 ml Hydrazin-hydrat wird während 3 Stunden unter Rückfluss zum Sieden erhitzt und unter Waschen mit Aethanol filtriert.

Das Filtrat wird konzentriert, und der Rückstand in Methylenchlorid aufgenommen. Man wäscht mit 2,5 N Natronlauge und trocknet über Kaliumcarbonat. Der nach Eindampfen erhaltene Rückstand wird destilliert und liefert 42,4 g (90 %) 6-Pyridinhexanamin vom Siedepunkt 125-130°/0,3 mm. Der Reinheitsgrad dieses Materials betrug gemäss gaschromatographischer Analyse 98,5 %.

Beispiel 111

6-(3-Chinolinyl)-5-hexin-1-ol wird gemäss den Angaben in Beispiel 105 hergesrtellt, und zwar ausgehend von 15 g 3-Bromchinolin, 8,5 g 5-Hexin-1-ol, 1,05 g Bis(triphenylphosphin) palladiumdichlorid und 0,1 g Kupfer (I) jodid in 115 ml Methylenchlorid und 28,5 ml Triäthylamin. Das Rohprodukt wird mittels Hochdruck-Flüssigkeits-Chromatographie unter Eluieren mit 2,5-proz. Methanol in Methylenchlorid gereinigt und liefert 14,4 g (89 %) 6-(3-Chinolinyl)-5-hexin-1-ol als farbloses Oel. Eine Probe davon wird an einer präparativen Kieselgelplatte unter Verwendung von 10-proz. Methanol in Methylenchlorid als Fliessmittel chromatographiert und getrocknet. Man erhält das Produkt von einem Schmelzpunkt von 47-48°.

Beispiel 112

3-Chinolinhexanol wird hergestellt, indem man 13,5 g 6-(3-Chinolinyl)-5-hexin-1-ol über 1 g 10-proz.

Palladium auf Kohle unter einem Wasserstoffdruck von 1 Atmosphäre reduziert. Das nach Filtrieren und Eindampfen erhaltene Rohprodukt wird mittels Hochdruck-Flüssigkeits-Chromatographie unter Eluieren mit 2-proz. Methanol in Methylenchlorid gereinigt. Zuerst eluiert man 0,95 g eines Materials, das man destilliert und das 0,76 g (6 %) 6-[3-(1,2,3,4-Tetrahydro)-chinolinyl] hexanol vom Siedepunkt 180-185°/0,1 mm liefert. Anschliessend eluiert man 9,1 g eines Stoffes, den man ebenfalls destilliert. Man erhält 8,3 g (61 %) 3-Chinolinhexanol vom Siedepunkt 195-205°/0,25 mm.

Beispiel 113

Eine Lösung von 7,8 g 3-Chinolinhexanol in 35 ml trockenem Methylenchlorid wird tropfenweise mit einer Lösung von 6,07 g Thionylchlorid in 15 ml Methylenchlorid behandelt. Man erhitzt das Reaktionsgemisch während 1 Stunde unter Rückfluss zum Sieden, dampft zur Trockene ein und nimmt den Rückstand in Methylenchlorid auf. Man wäscht nacheinander mit Wasser, gesättigter Natriumbicarbonatlösung und Kochsalzlösung, trocknet über Kaliumcarbonat und dampft ein. Man erhält 8,3 g rohes 6-(3-Chinolinyl) hexylchlorid, das man zusammen mit 12,6 g Kaliumphthalimid und 0,5 g Kaliumjodid in 60 ml Dimethylformamid suspendiert. Man erhitzt während 1 Stunde in einem 130° warmen Bad, kühlt ab, verdünnt mit Wasser und extrahiert mit Methylenchlorid. Die vereinigten organischen Auszüge werden mit Wasser gewaschen, über Kaliumcarbonat getrocknet und eingedampft. Das erhaltene Rohprodukt kristallisiert man aus Essigester/Hexan und erhält 9,9 g (82 %) 2-[6-(3-Chinolinyl)hexyl]-1H-isoindol-1,3(2H)-dion vom Schmelzpunkt 90-91°. Aus Toluol erhält man eine analytische Probe mit einem Schmelzpunkt von 95,5-96,5°.

Beispiel 114

3-Chinolinhexanamin wird in Analogie zu den Angaben in Beispiel 110 hergestellt, und zwar ausgehend von 4,4 g 2-[6-(3-Chinolinyl)hexyl]-1H-isoindol-1,3(2H)-dion und 2,5 g Hydrazinhydrat in 60 ml Aethanol. Das erhaltene Rohprodukt wird destilliert und liefert 2,5 g (89 %) 3-Chinolinhexanamin vom Siedepunkt 144-155°/0,1 mm. Das entsprechende Bis-Picrat hat einen Schmelzpunkt von 225-226°.

Beispiel 115

Argon wird durch eine Lösung von 45 ml 3-Brompyridin und 49,61 g 5-Hexinnitril in 500 ml Methylenchlorid und 150 ml trockenem Triäthylamin durchgeleitet. Nach 15 Minuten versetzt man mit 3,0 g Bis(triphenylphosphin) palladiumdichlorid und 0,45 g Kupfer (I) jodid, evakuiert das Reaktionsgefäss und füllt es mit Argon und erhitzt während 12 Stunden unter Rückfluss zum Sieden. Man verdünnt die erhaltene Mischung mit 1 l Aethylenchlorid, wäscht zweimal mit je 300 ml Wasser, einmal mit 300 ml Kochsalzlösung und trocknet über Kaliumcarbonat. Das nach Eindampfen erhaltene Material wird destilliert und liefert 62, 82 g (79 %) 6-(3-Pyridinyl)-5-hexinnitril als gelbes Oel vom Siedepunkt 140-170°/0,2 mm. Dieses Material wird direkt in die nächste Stufe eingesetzt. Eine Probe wird durch Chromatographieren an Kieselgel und unter Eluieren mit Essigester/Hexan (1 : 1), das 1 % Triäthylamin enthält, und erneute Destillation gereinigt. Man erhält eine analytische Probe von 6-(3-Pyridinyl)-5-hexinnitril.

Beispiel 116

Eine Lösung von 62,82 g 6-(3-Pyridinyl)-5-hexinnitril in 500 ml 2-Propanol wird über 3,0 g 10-proz. Palladium auf Kohle und bei Atmosphärendruck hydriert. Zwei zusätzliche 3 g-Portionen des Katalysators werden jeweils dann zugegeben, wenn die Geschwindigkeit der Wasserstoffaufnahme abnimmt. Nach 2 Tagen wird das Reaktionsgemisch filtriert und konzentriert, worauf man den Rückstand destilliert und das Destillat in 300 ml 2-Propanol aufnimmt. Man reduziert erneut an 3,0 g 10-proz. Palladium auf Kohle. Nach Filtrieren und Eindampfen wird das erhaltene Material destilliert. Man erhält 52,15 g (82 %) 6-(3-Pyridinyl)hexannitril vom Siedepunkt 150°/0,3 mm, welches gemäss Gaschromatographie einen Reinheitsgehalt von 93 % aufweist. Eine Probe dieses Materials wird durch Chromatographieren an Kieselgel und unter Eluieren mit Essigester/Hexan (1 : 1), das 1 % Triäthylamin enthält, und erneute Destillation gereinigt. Man erhält auf diese Art eine analytisch reine Probe.

Beispiel 117

Eine Lösung von 52,15 g 3-Pyridinhexannitril in 600 ml Methanol und 13 ml Triäthylamin wird unter 13 g Raney-Cobalt hydriert, wobei man einen Anfangsdruck von 1 000 psi anlegt und bei 100° arbeitet. Die abgekühlte Mischung wird filtriert und eingedampft. Durch Destillieren des Rückstandes erhält man 46,5 g (87 %) 3-Pyridinhexanamin vom Siedepunkt 102-107°/0,2 mm. Dieses Material wird über das entsprechende Phthalimid, das man durch Reaktion mit 39,53 g Phthalsäureanhydrid in 300 ml Eisessig unter Erhitzen unter Rückfluss über Nacht erhält, gereinigt. Das nach dem Eindampfen erhaltene Material wird in 300 ml Essigester aufgenommen, worauf man mit verdünnter Natronlauge und Natriumbicarbonatlösung wäscht, über Kaliumcarbonat trocknet und eindampft. Der Rückstand wird aus Essigester/Hexan kristallisiert und liefert 62,87 g (77 %) 2-[6-(3-Pyridinyl)hexyl]-1H-isoindol-1,3(2H)-dion vom Schmelzpunkt 89-92°. Eine Lösung dieses Materials in 880 ml Aethanol und 33 ml Hydrazin-hydrat wird während 3 Stunden unter Rückfluss zum Sieden erhitzt, abgekühlt, filtriert und eingedampft. Den Rückstand nimmt man in 500 ml Methylenchlorid auf, wäscht mit 10-proz. Natronlauge und trocknet über Kaliumcarbonat. Das nach dem Eindampfen erhaltene Material wird destilliert und liefert 31,6 g (60 % bezogen auf das Ausgangsnitril) 3-Pyridinhexamin vom Siedepunkt 140-150°/0,3 mm. Dieses Material zeigte im Gaschromatogramm nur einen einzigen Peak.

## Beispiel 118

Herstellung von Tabletten (Nassgranulierung)

| | Bestandteile | mg/Tablette | | |
|---|---|---|---|---|
| 1. | N-[4-(1H-Imidazol-1-yl)-butyl]-2-(1-methyläthyl)-11-oxo-11H-pyrido[2,1-b]-chinazolin-8-carboxamid | 100 | 250 | 500 |
| 2. | Lactose | 98,5 | 147,5 | 170 |
| 3. | Polyvinylpyrrolidon | 15 | 30 | 40 |
| 4. | Modifizierte Stärke | 15 | 30 | 40 |
| 5. | Kornstärke | 15 | 30 | 40 |
| 6. | Magnesiumstearat | 1,5 | 2,5 | 5 |
| | Tablettengewicht | 245 mg | 490 mg | 795 mg |

Verfahren :

1) Man vermischt die Bestandteile 1, 2, 4 und 5 in einem geeigneten Mixer, granuliert mit Polyvinylpyrrolidon, versetzt mit Wasser/Alkohol, trocknet das Granulat und mahlt das trockene Granulat in einer geeigneten Mühle.

2) Man versetzt mit dem Magnesiumstearat und verpresst auf einer geeigneten Presse.

## Beispiel 119

Herstellung von Tabletten (Nassgranulierung)

| | Bestandteile | mg/Tablette | | |
|---|---|---|---|---|
| 1. | N-[4-(1H-Imidazol-1-yl)-butyl]-2-(1-methyläthyl)-11-oxo-11H-pyrido[2,1-b]-chinazolin-8-carboxamid | 100 | 250 | 500 |
| 2. | Lactose | 147,5 | 100 | 97,5 |
| 3. | Vorgelatinisierte Stärke | 25 | 30 | 60 |
| 4. | Modifizierte Stärke | 25 | 50 | 60 |
| 5. | Kornstärke | 25 | 50 | 60 |
| 6. | Magnesiumstearat | 2,5 | 5 | 7,5 |
| | Tablettengewicht | 325 mg | 485 mg | 785 mg |

Verfahren :

1) Man vermischt die Bestandteile 1, 2, 3, 4 und 5 in einem geeigneten Mixer, granuliert mit Wasser und trocknet in einem geeigneten Ofen über Nacht. Man mahlt mittels einer geeigneten Mühle.

2) Man vermischt mit Bestandteil 6 und verpresst auf einer geeigneten Presse.

Beispiel 120

Herstellung von Kapseln

| | Bestandteile | mg/Kapsel | | |
|---|---|---|---|---|
| 1. | N-[4-(1H-Imidazol-1-yl)-butyl]-2-(1-methyläthyl)-11-oxo-11H-pyrido[2,1-b]-chinazolin-8-carboxamid | 100 | 250 | 500 |
| 2. | Lactose | 99 | 148 | - |
| 3. | Kornstärke | 20 | 30 | 57 |
| 4. | Talk | 5 | 10 | 15 |
| 5. | Magnesiumstearat | 1 | 2 | 3 |
| | Kapselfüllgewicht | 225 mg | 440 mg | 575 mg |

Verfahren :

1) Man vermischt die Bestandteile 1, 2 und 3 in einem geeigneten Mixer und mahlt mittels einer geeigneten Mühle.

2) Man vermischt die erhaltene Mischung mit den Bestandteilen 4 und 5 und füllt mittels einer geeigneten Maschine in Kapseln ab.

**Patentansprüche** (für die Vertraagsstaaten : BE, CH, DE, FR, GB, IT, LI, LU, NL, SE)

1. Eine Verbindung der allgemeinen Formel

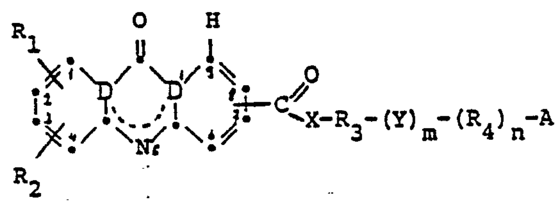

(I)

worin eines von D und D' ein Stickstoffatom und das andere ein Kohlenstoffatom, die gestrichelte Linie zwei zusätzliche Bindungen, $R_1$ und $R_2$ unabhängig von einander Wasserstoff, $(C_{1-7})$-Alkyl, $(C_{1-7})$-Alkoxy, Hydroxy oder Halogen, X —O— oder —N($R_5$)—, $R_5$ Wasserstoff oder $(C_{1-7})$-Alkyl, $R_3$ $(C_{2-7})$-Alkylen, $R_4$ $(C_{1-7})$-Alkylen, Y —O— oder —S—, m 0 oder 1, n 0 oder 1 und A eine unsubstituierte oder durch $(C_{1-7})$-Alkyl, $(C_{1-7})$-Alkoxy, Halogen oder Nitro substituierte, aromatische, 5- oder 6-gliedrige heterocyclische Gruppe bedeuten, mit der Massgabe, dass m und n beide O sind, wenn A über ein heterocyclisches Stickstoffatom gebunden ist,
oder ein pharmazeutisch annehmbares Säureadditionssalz davon.

2. Eine Verbindung gemäss Anspruch 1, worin $R_1$ und $R_2$ in der 2- bzw. 3-Stellung und die Gruppe —CO—X—$R_3$—$(Y)_m$—$(R_4)_n$—A in der 8-Stellung sind.

3. Eine Verbindung gemäss Anspruch 1 oder 2, worin X —N($R_5$)— bedeutet.

4. Eine Verbindung gemäss einem der Ansprüche 1-3, worin A 3-Pyridyl, 4-Pyridyl oder Imidazol-1-yl bedeutet.

5. Eine Verbindung gemäss einem der Ansprüche 1-4, worin $R_3$ $(C_{4-6})$-Alkylen und m und n 0 bedeuten.

6. Eine Verbindung gemäss einem der Ansprüche 1-5, worin $R_1$ $(C_{1-7})$-Alkyl und $R_2$ Wasserstoff bedeuten.

7. Eine Verbindung gemäss einem der Ansprüche 1-4, worin $R_1$ Hydroxy, D ein Kohlenstoffatom, D' ein Stickstoffatom und die gestrichelte Linie zwei zusätzliche Bindungen bedeuten.

8. N-[4-(1H-Imidazol-1-yl) butyl]-2-(1-methyläthyl)-11-oxo-11H-pyrido [2,1-b] chinazolin-8-carboxamid.

9. 2-(1-Methyläthyl)-N-[6-(3-pyridyl) hexyl]-11-oxo-11H-pyrido [2,1-b] chinazolin-8-carboxamid.

10. 2-(1-Methyläthyl)-N-[4-(3-pyridyl) butyl]-11-oxo-11H-pyrido [2,1-b] chinazolin-8-carboxamid.

11. 8-(1-Methyläthyl)-N-[4-(3-pyridyl) butyl]-11-oxo-11H-pyrido [2,1-b] chinazolin-2-carboxamid.

12. 2-Hydroxy-N-[4-(3-pyridyl) butyl]-11-oxo-11H-pyrido [2,1-b] chinazolin-8-carboxamid.

13. 2,3-Dimethyl-N-[4-(5-pyrimidinyl) butyl]-11-oxo-11H-pyrido [2,1-b] chinazolin-8-carboxamid.

14. 2-(1-Methyläthyl)-N-[4-(1H-imidazol-1-yl)-1-methylbutyl]-11-oxo-11H-pyrido [2,1-b] chinazolin-8-carboxamid und 2,3-Dimethyl-N-[4-(3-pyridyl)butyl]-11-oxo-11H-pyrido-[2,1-b]chinazolin-8-carboxamid.

15. 2-(1-Methyläthyl)-N-[3-(3-pyridyl) propyl]-11-oxo-11H-pyrido [2,1-b] chinazolin-8-carboxamid,
2-(1-Methyläthyl)-N-[4-(3-pyridyl) butyl]-11-oxo-11H-pyrido [2,1-b] chinazolin-6-carboxamid,
N-[5-(1H-Imidazol-1-yl) pentyl]-2-(1-methyläthyl)-11-oxo-11H-pyrido [2,1-b] chinazolin-8-carboxamid,
N-[4-(4-Pyridyl) butyl]-2-(1-methyläthyl)-11-oxo-11H-pyrido [2,1-b] chinazolin-8-carboxamid,
2-(1-Methyläthyl)-N-[4-(3-pyridyloxy) butyl]-11-oxo-11H-pyrido [2,1-b] chinazolin-8-carboxamid,
N-[2-(4-Pyridylthio) äthyl]-2-(1-methyläthyl)-11-oxo-11H-pyrido [2,1-b] chinazolin-8-carboxamid,
N-[5-(3-Pyridyl) pentyl]-2-(1-methyläthyl)-11-oxo-11H-pyrido [2,1-b] chinazolin-8-carboxamid,
N-[5-(4-Pyridyl) pentyl]-2-(1-methyläthyl)-11-oxo-11H-pyrido [2,1-b] chinazolin-8-carboxamid,
2-Methoxy-N-[4-(3-pyridyl) butyl]-11-oxo-11H-pyrido [2,1-b] chinazolin-8-carboxamid,
2-(1-Methyläthyl)-N-[4-(5-pyrimidinyl) butyl]-11-oxo-11H-pyrido [2,1-b] chinazolin-8-carboxamid,
N-[4-(2-Methyl-1H-imidazol-1-yl) butyl]-2-(1-methyl-äthyl)-11-oxo-11H-pyrido [2,1-b] chinazolin-8-carboxamid und
N-[4-(3-Pyridyl) butyl]-11-oxo-11H-pyrido [2,1-b] chinazolin-8-carboxamid.

16. Eine Verbindung gemäss einem der Ansprüche 1-15 als pharmazeutischer Wirkstoff.

17. Eine Verbindung gemäss einem der Ansprüche 1-15 als Wirkstoff für die Behandlung von Allergien und thrombotischen Gefässerkrankungen.

18. Ein Verfahren zur Herstellung einer Verbindung gemäss einem der Ansprüche 1-15, dadurch gekennzeichnet, dass man eine Carbonsäure der allgemeinen Formel

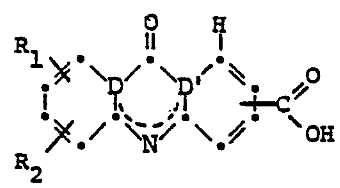

(II)

worin $R_1$, $R_2$, D, D' und die gestrichelte Linie die in Anspruch 1 angegebene Bedeutung besitzen, oder ein reaktives Derivat davon mit einer Verbindung der allgemeinen Formel

$$W—R_3—(Y)_m—(R_4)_n—A \qquad (VI)$$

worin W die Gruppe HX— oder falls man die freie Säure der Formel II verwendet, auch eine Abgangsgruppe bedeutet, und $R_3$, $R_4$, A, X, Y, m und n die in Anspruch 1 angegebene Bedeutung besitzen,
umsetzt und erwünschtenfalls eine erhaltene Verbindung der Formel I in ein pharmazeutisch annehmbares Säureadditionssalz überführt.

19. Ein pharmazeutisches Präparat, enthaltend eine Verbindung gemäss einem der Ansprüche 1-15.

20. Ein pharmazeutisches Präparat gemäss Anspruch 19 für die Behandlung von Allergien und thrombotischen Gefässerkrankungen.

**Patentansprüche** (für den Vertragsstaat AT)

1. Verfahren zur Herstellung einer Verbindung der allgemeinen Formel

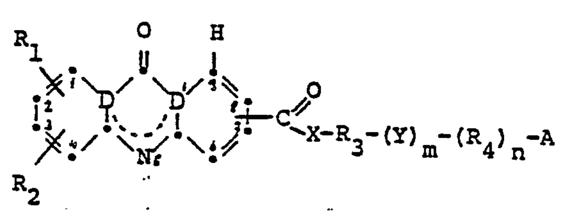

(I)

worin eines von D und D' ein Stickstoffatom und das andere ein Kohlenstoffatom, die gestrichelte Linie zwei zusätzliche Bindungen, $R_1$ und $R_2$ unabhängig von einander Wasserstoff, $(C_{1-7})$-Alkyl, $(C_{1-7})$-Alkoxy, Hydroxy oder Halogen, X —O— oder —$N(R_5)$—, $R_5$ Wasserstoff oder $(C_{1-7})$-Alkyl, $R_3$ $(C_{2-7})$-Alkylen, $R_4$ $(C_{1-7})$-Alkylen, Y —O— oder —S—, m 0 oder 1, n 0 oder 1 und A eine unsubstituierte oder durch $(C_{1-7})$-Alkyl, $(C_{1-7})$-Alkoxy, Halogen oder Nitro substituierte, aromatische, 5- oder 6-gliedrige heterocyclische Gruppe bedeuten, mit der Massgabe, dass m und n beide O sind, wenn A über ein

heterocyclisches Stickstoffatom gebunden ist, oder eines pharmazeutisch annehmbaren Säureadditionssalzes davon, dadurch gekennzeichnet, dass man eine Carbonsäure der allgemeinen Formel

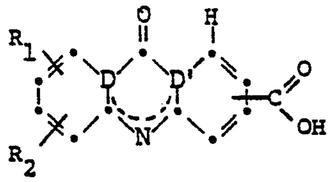

(II)

worin $R_1$, $R_2$, D, D' und die gestrichelte Linie obige Bedeutung besitzen, oder ein reaktives Derivat davon mit einer Verbindung der allgemeinen Formel

$$W—R_3—(Y)_m—(R_4)_n—A \quad (VI)$$

worin W die Gruppe HX— oder, falls man die freie Säure der Formel II verwendet, auch eine Abgangsgruppe bedeutet, und $R_3$, $R_4$, A, X, Y, m und n obige Bedeutung besitzen, umsetzt und erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass man eine Verbindung der allgemeinen Formel

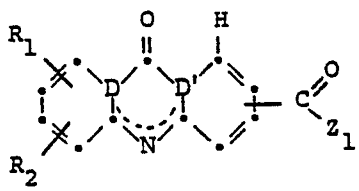

(IIIa)

worin $Z_1$ ein Halogenatom bedeutet, und $R_1$, $R_2$, D, D' und die gestrichelte Linie die in Anspruch 1 angegebene Bedeutung besitzen, mit einer Verbindung der allgemeinen Formel

$$HX—R_3—(Y)_m—(R_4)_n—A \quad (IV)$$

worin $R_3$, $R_4$, A, X, Y, m und n die in Anspruch 1 angegebene Bedeutung besitzen, umsetzt.

3. Verfahren gemäss Anspruch 1 zur Herstellung einer Verbindung der Formel I, worin X —$N(R_5)$— bedeutet, dadurch gekennzeichnet, dass man eine Verbindung der Formel II in Gegenwart von Diphenylphosphorylazid und einem Protonenakzeptor mit einer Verbindung der allgemeinen Formel

$$HN(R_5)—R_3—(Y)_m—(R_4)_n—A \quad (IVa)$$

worin $R_3$, $R_4$, $R_5$, A, Y, m und n die in Anspruch 1 angegebene Bedeutung besitzen, umsetzt.

4. Verfahren gemäss Anspruch 1 zur Herstellung einer Verbindung der Formel I, worin D ein Kohlenstoffatom, D' ein Stickstoffatom, die gestrichelte Linie zwei zusätzliche Bindungen und X —O— bedeuten, dadurch gekennzeichnet, dass man eine Verbindung der allgemeinen Formel

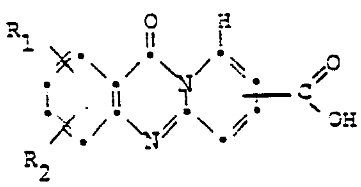

(IIa)

worin $R_1$ und $R_2$ die in Anspruch 1 angegebene Bedeutung besitzen, in Gegenwart einer Base mit einer Verbindung der allgemeinen Formel

$$Z'—R_3—(Y)_m—(R_4)_n—A \quad (V)$$

worin $R_3$, $R_4$, A, Y, m und n die in Anspruch 1 angegebene Bedeutung besitzen, und Z' eine Abgangsgruppe bedeutet, umsetzt.

5. Verfahren gemäss Anspruch 1 zur Herstellung einer Verbindung der Formel I, worin D ein Kohlenstoffatom, D' ein Stickstoffatom, die gestrichelte Linie zwei zusätzliche Bindungen und X —N($R_5$)— bedeuten, dadurch gekennzeichnet, dass man eine Verbindung der allgemeinen Formel

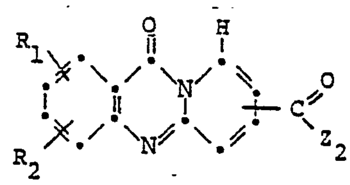

(IIIb)

worin $R_1$ und $R_2$ die in Anspruch 1 angegebene Bedeutung besitzen, und $Z_2$ Cyanomethoxy, Phenoxy oder substituiertes Phenoxy bedeutet, mit einer Verbindung der in Anspruch 3 definierten Formel IVa umsetzt.

6. Verfahren gemäss Anspruch 1, worin $R_1$ und $R_2$ in der 2- bzw. 3-Stellung und die Gruppe —CO—X—$R_3$—$(Y)_m$—$(R_4)_n$—A in der 8-Stellung sind.

7. Verfahren gemäss Anspruch 1 oder 6, worin X —N($R_5$)— bedeutet.

8. Verfahren gemäss Anspruch 1, 6 oder 7, worin A 3-Pyridyl, 4-Pyridyl oder Imidazol-1-yl bedeutet.

9. Verfahren gemäss einem der Ansprüche 1 und 6-8, worin $R_3$ ($C_{4-6}$)-Alkylen und m und n die Zahl 0 bedeuten.

10. Verfahren gemäss einem der Ansprüche 1 und 6-9, worin $R_1$ ($C_{1-7}$)-Alkyl und $R_2$ Wasserstoff bedeuten.

11. Verfahren gemäss einem der Ansprüche 1 und 6-8, worin $R_1$ Hydroxy, D ein Kohlenstoffatom, D' ein Stickstoffatom und die gestrichelte Linie zwei zusätzliche Bindungen bedeuten.

12. Verfahren gemäss Anspruch 1, dadurch gekennzeichnet, dass man N-[4-(1H-Imidazol-1-yl) butyl]-2-(1-methyläthyl)-11-oxo-11H-pyrido [2,1-b] chinazolin-8-carboxamid herstellt.

13. Verfahren gemäss Anspruch 1, dadurch gekennzeichnet, dass man 2-(1-Methyläthyl)-N-[6-(3-pyridyl) hexyl]-11-oxo-11H-pyrido [2,1-b] chinazolin-8-carboxamid herstellt.

14. Verfahren gemäss Anspruch 1, dadurch gekennzeichnet, dass man 2-(1-Methyläthyl)-N-[4-(3-pyridyl) butyl]-11-oxo-11H-pyrido [2,1-b] chinazolin-8-carboxamid herstellt.

15. Verfahren gemäss Anspruch 1, dadurch gekennzeichnet, dass man 8-(1-Methyläthyl)-N-[4-(3-pyridyl) butyl]-11-oxo-11H-pyrido [2,1-b] chinazolin-2-carboxamid herstellt.

16. Verfahren gemäss Anspruch 1, dadurch gekennzeichnet, dass man 2-Hydroxy-N-[4-(3-pyridyl) butyl]-11-oxo-11H-pyrido [2,1-b] chinazolin-8-carboxamid herstellt.

17. Verfahren gemäss Anspruch 1, dadurch gekennzeichnet, dass man 2,3-Dimethyl-N-[4-(5-pyrimidinyl) butyl]-11-oxo-11H-pyrido [2,1-b] chinazolin-8-carboxamid herstellt.

**Claims** (for the Contracting States : BE, CH, DE, FR, GB, IT, LI, LU, NL, SE)

1. A compound of the general formula

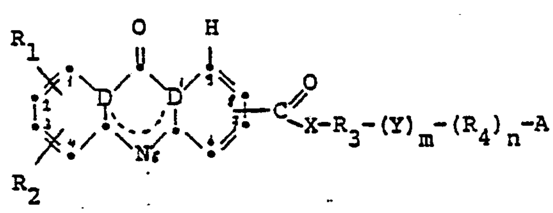

(I)

wherein one of D and D' signifies a nitrogen atom and the other signifies a carbon atom, the dotted line signifies two additional bonds, $R_1$ and $R_2$ each independently signify hydrogen, ($C_{1-7}$)-alkyl, ($C_{1-7}$)-alkoxy, hydroxy or halogen, X signifies —O— or —N($R_5$)—, $R_5$ signifies hydrogen or ($C_{1-7}$)-alkyl, $R_3$ signifies ($C_{2-7}$)-alkylene, $R_4$ signifies ($C_{1-7}$)-alkylene, Y signifies —O— or —S—, m signifies 0 or 1, n signifies 0 or 1 and A signifies an aromatic, 5- or 6-membered heterocyclic group which is unsubstituted or substituted by ($C_{1-7}$)-alkyl, ($C_{1-7}$)-alkoxy, halogen or nitro, with the proviso that m and n are both O when A is linked via a heterocyclic nitrogen atom, or a pharmaceutically acceptable acid addition salt thereof.

2. A compound in accordance with claim 1, wherein $R_1$ and $R_2$ are in the 2- and, respectively, 3-position and the group —CO—X—$R_3$—$(Y)_m$—$(R_4)_n$—A is in the 8-position.

3. A compound in accordance with claim 1 or 2, wherein X signifies —$N(R_5)$—.

4. A compound in accordance with any one of claims 1-3, wherein A signifies 3-pyridyl, 4-pyridyl or imidazol-1-yl.

5. A compound in accordance with any one of claims 1-4, wherein $R_3$ signifies $(C_{4-6})$-alkylene and m and n signify 0.

6. A compound in accordance with any one of claims 1-5, wherein $R_1$ signifies $(C_{1-7})$-alkyl and $R_2$ signifies hydrogen.

7. A compound in accordance with any one of claims 1-4, wherein $R_1$ signifies hydroxy, D signifies a carbon atom, D' signifies a nitrogen atom and the dotted line signifies two additional bonds.

8. N-[4-(1H-Imidaol-1-yl) butyl]-2-(1-methylethyl)-11-oxo-11H-pyrido [2,1-b] quinazoline-8-carboxamide.

9. 2-(1-Methylethyl)-N-[6-(3-pyridyl) hexyl]-11-oxo-11H-pyrido [2,1-b] quinazoline-8-carboxamide.

10. 2-(1-Methylethyl)-N-[4-(3-pyridyl) butyl]-11-oxo-11H-pyrido [2,1-b] quinazoline-8-carboxamide.

11. 8-(1-Methylethyl)-N-[4-(3-pyridyl) butyl]-11-oxo-11H-pyrido [2,1-b] quinazoline-2-carboxamide.

12. 2-Hydroxy-N-[4-(3-pyridyl) butyl]-11-oxo-11H-pyrido [2,1-b] quinazoline-8-carboxamide.

13. 2,3-Dimethyl-N-[4-(5-pyrimidinyl) butyl]-11-oxo-11H-pyrido [2,1-b] quinazoline-8-carboxamide.

14. 2-(1-Methylethyl)-N-[4-(1H-imadazol-1-yl)-1-methylbutyl]-11-oxo-11H-pyrido [2,1-b] quinazoline-8-carboxamide and 2,3-dimethyl-N-[4-(3-pyridyl) butyl]-11-oxo-11H-pyrido [2,1-b] quinazoline-8-carboxamide.

15. 2-(1-Methylethyl)-N-[3-(3-pyridyl) propyl]-11-oxo-11H-pyrido [2,1-b] quinazoline-8-carboxamide,
2-(1-methylethyl)-N-[4-(3-pyridyl) butyl]-11-oxo-11H-pyrido [2,1-b] quinazoline-6-carboxamide,
N-[5-(1H-imidazol-1-yl) pentyl]-2-(1-methylethyl)-11-oxo-11H-pyrido [2,1-b] quinazoline-8-carboxamide,
N-[4-(4-pyridyl) butyl]-2-(1-methylethyl)-11-oxo-11H-pyrido [2,1-b] quinazoline-8-carboxamide,
2-(1-methylethyl)-N-[4-(3-pyridyloxy) butyl]-11-oxo-11H-pyrido [2,1-b] quinazoline-8-carboxamide,
N-[2-(4-pyridylthio) éthyl]-2-(1-methylethyl)-11-oxo-11H-pyrido [2,1-b] quinazoline-8-carboxamide,
N-[5-(3-pyridyl) pentyl]-2-(1-methylethyl)-11-oxo-11H-pyrido [2,1-b] quinazoline-8-carboxamide,
N-[5-(4-pyridyl) pentyl]-2-(1-methylethyl)-11-oxo-11H-pyrido [2,1-b] quinazoline-8-carboxamide,
2-methoxy-N-[4-(3-pyridyl) butyl]-11-oxo-11H-pyrido [2,1-b] quinazoline-8-carboxamide,
2-(1-methylethyl)-N-[4-(5-pyrimidinyl) butyl]-11-oxo-11H-pyrido [2,1-b] quinazoline-8-carboxamide,
N-[4-(2-methyl-1H-imidazol-1-yl) butyl]-2-(1-methylethyl)-11-oxo-11H-pyrido [2,1-b] quinazoline-8-carboxamide and
N-[4-(3-pyridyl) butyl]-11-oxo-11H-pyrido [2,1-b] quinazoline-8-carboxamide.

16. A compound in accordance with any one of claims 1-15 as a pharmaceutically active substance.

17. A compound in accordance with any one of claims 1-15 as an active substance for the treatment of allergies and thrombotic vascular disorders.

18. A process for the manufacture of a compound in accordance with any one of claims 1-15, characterized by reacting a carboxylic acid of the general formula

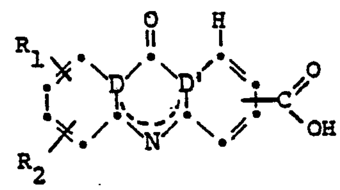

(II)

wherein $R_1$, $R_2$, D, D' and the dotted line have the significance given in claim 1, or a reactive derivative thereof with a compound of the general formula

$$W—R_3—(Y)_m—(R_4)_n—A \quad (VI)$$

wherein W signifies the group HX— or, where the free acid of formula II is used, also a leaving group, and $R_3$, $R_4$, A, X, Y, m and n have the significance given in claim 1,
and, if desired, converting a compound of formula I obtained into a pharmaceutically acceptable acid addition salt.

19. A pharmaceutical preparation containing a compound in accordance with any one of claims 1-15.

20. A pharmaceutical preparation in accordance with claim 19 for the treatment of allergies and thrombotic vascular disorders.

**Claims** (for the Contracting State AT)

1. A process of the manufacture of a compound of the general formula

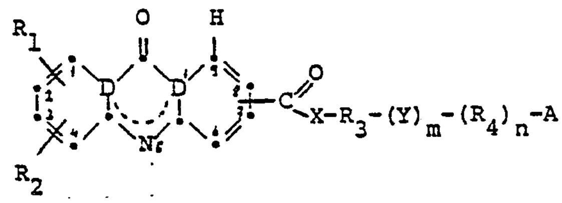

(I)

wherein one of D and D' signifies a nitrogen atom and the other signifies a carbon atom, the dotted line signifies two additional bonds, $R_1$ and $R_2$ each independently signify hydrogen, $(C_{1-7})$-alkyl, $(C_{1-7})$-alkoxy, hydroxy or halogen, X signifies —O— or —$N(R_5)$—, $R_5$ signifies hydrogen or $(C_{1-7})$-alkyl, $R_3$ signifies $(C_{2-7})$-alkylene, $R_4$ signifies $(C_{1-7})$-alkylene, Y signifies —O— or —S—, m signifies 0 or 1, n signifies 0 or 1 and A signifies an aromatic, 5- or 6-membered heterocyclic group which is unsubstituted or substituted by $(C_{1-7})$-alkyl, $(C_{1-7})$-alkoxy, halogen or nitro, with the proviso that m and n are both O when A is linked via a heterocyclic nitrogen atom,
or of a pharmaceutically acceptable acid addition salt thereof, characterized by reacting a carboxylic acid of the general formula

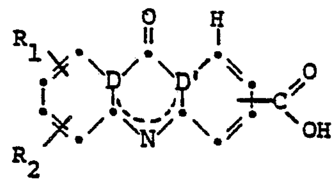

(II)

wherein $R_1$, $R_2$, D, D' and the dotted line have the above significance.
or a reactive derivative thereof with a compound of the general formula

$$W—R_3—(Y)_m—(R_4)_n—A \quad (VI)$$

wherein W signifies the group HX— or, where the free acid of formula II is used, also a leaving group, and $R_3$, $R_4$, A, X, Y, m and n have the above significance,
and, if desired, converting a compound of formula I obtained into a pharmaceutically acceptable acid addition salt.

2. A process in accordance with claim 1, characterized in that a compound of the general formula

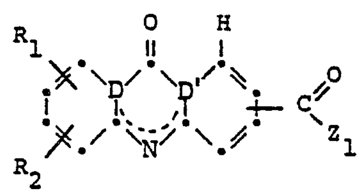

(IIIa)

wherein $Z_1$ signifies a halogen atom and $R_1$, $R_2$, D, D' and the dotted line have the significance given in claim 1,
is reacted with a compound of the general formula

$$HX—R_3—(Y)_m—(R_4)_n—A \quad (IV)$$

wherein $R_3$, $R_4$, A, X, Y, m and n have the significance given in claim 1.

3. A process in accordance with claim 1 for the manufacture of a compound of formula I in which X signifies —$N(R_5)$—, characterized in that a compound of formula II is reacted in the presence of diphenylphosphoryl azide and a proton acceptor with a compound of the general formula

$$HN(R_5)—R_3—(Y)_m—(R_4)_n—A \quad (IVa)$$

wherein $R_3$, $R_4$, $R_5$, A, Y, m and n have the significance given in claim 1.

4. A process in accordance with claim 1 for the manufacture of a compound of formula I in which D signifies a carbon atom, D' signifies a nitrogen atom, the dotted line signifies two additional bonds and X signifies —O—, characterized in that a compound of the general formula

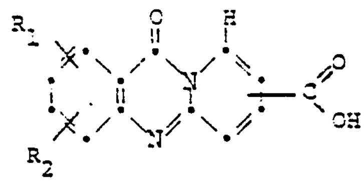

(IIa)

wherein $R_1$ and $R_2$ have the significance given in claim 1, is reacted in the presence of a base with a compound of the general formula

$$Z'—R_3—(Y)_m—(R_4)_n—A \qquad (V)$$

wherein $R_3$, $R_4$, A, Y, m and n have the significance given in claim 1 and Z' signifies a leaving group.

5. A process in accordance with claim 1 for the manufacture of a compound of formula I in which D signifies a carbon atom, D' signifies a nitrogen atom, the dotted line signifies two additional bonds and X signifies $—N(R_5)—$, characterized in that a compound of the general formula

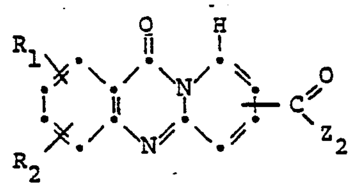

(IIIb)

wherein $R_1$ and $R_2$ have the significance given in claim 1 and $Z_2$ signifies cyanomethoxy, phenoxy or substituted phenoxy, is reacted with a compound of formula IVa defined in claim 3.

6. A process in accordance with claim, 1 wherein $R_1$ and $R_2$ are present in the 2- and, respectively, 3-position and the group $—CO—X—R_3—(Y)_m—(R_4)_n—A$ is in 8-position.

7. A process in accordance with claim 1 or 6, wherein X signifies $—N(R_5)—$.

8. A process in accordance with claim 1, 6 or 7, wherein A signifies 3-pyridyl, 4-pyridyl or imidazol-1-yl.

9. A process in accordance with any one of claims 1 and 6-8, wherein $R_3$ signifies $(C_{4-6})$-alkylene and m and n signify the number 0.

10. A process in accordance with any one of claims 1 and 6-9, wherein $R_1$ signifies $(C_{1-7})$-alkyl and $R_2$ signifies hydrogen.

11. A process in accordance with any one of claims 1 and 6-8, wherein $R_1$ signifies hydroxy, D signifies a carbon atom. D' signifies a nitrogen atom and the dotted line signifies two additional bonds.

12. A process in accordance with claim 1, characterized in that N-[4-(1H-imidazol-1-yl) butyl]-2-(1-methylethyl)-11-oxo-11H-pyrido [2,1-b] quinazoline-8-carboxamide is manufactured.

13. A process in accordance with claim 1, characterized in that 2-(1-methylethyl)-N-[6-(3-pyridyl) hexyl]-11-oxo-11H-pyrido [2,1-b] quinazoline-8-carboxamide is manufactured.

14. A process in accordance with claim 1, characterized in that 2-(1-methylethyl)-N-[4-(3-pyridyl) butyl]-11-oxo-11H-pyrido [2,1-b] quinazoline-8-carboxamide is manufactured.

15. A process in accordance with claim 1, characterized in that 8-(1-methylethyl)-N-[4-(3-pyridyl) butyl]-11-oxo-11H-pyrido [2,1-b] quinazoline-2-carboxamide is manufactured.

16. A process in accordance with claim 1, characterized in that 2-hydroxy-N-[4-(3-pyridyl) butyl]-11-oxo-11H-pyrido [2,1-b] quinazoline-8-carboxamide is manufactured.

17. A process in accordance with claim 1, characterized in that 2,3-dimethyl-N-[4-(5-pyrimidinyl) butyl]-11-oxo-11H-pyrido [2,1-b] quinazoline-8-carboxamide is manufactured.

**Revendications** (pour les Etats contractants : BE, CH, DE, FR, GB, IT, LI, LU, NL, SE)

1. Un composé de formule générale

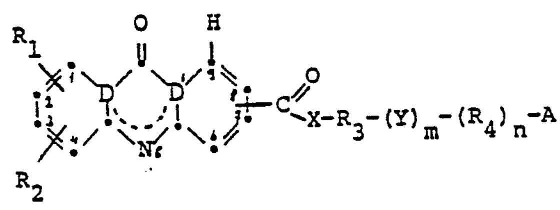

(I)

dans laquelle l'un des symboles D et D' représente un atome d'azote et l'autre un atome de carbone, le trait discontinu représente deux liaisons supplémentaires, $R_1$ et $R_2$ représentent chacun, indépendamment l'un de l'autre, l'hydrogène, un groupe alkyle en $C_1$-$C_7$, alcoxy en $C_1$-$C_7$, hydroxy ou un halogène, X représente —O— ou —N($R_5$)—, $R_5$ représente l'hydrogène ou un groupe alkyle en $C_1$-$C_7$, $R_3$ représente un groupe alkylène en $C_2$-$C_7$, $R_4$ un groupe alkylène en $C_1$-$C_7$, Y représente —O— ou —S—, m est égal à 0 ou 1, n est égal à 0 ou 1 et A représente un groupe aromatique hétérocyclique à 5 ou 6 chaînons non substitué ou substitué par des groupes alkyle en $C_1$-$C_7$, alcoxy en $C_1$-$C_7$, des halogènes ou des groupes nitro, avec la restriction que m et n doivent tous deux être égaux à 0 lorsque A est relié par l'intermédiaire d'un atome d'azote hétérocyclique, ou un sel acceptable pour l'usage pharmaceutique, formé par addition avec un acide d'un tel composé.

2. Un composé selon la revendication 1, dans lequel $R_1$ et $R_2$ sont respectivement en positions 2 et 3 et le groupe —CO—X—$R_3$—$(Y)_m$—$(R_4)_n$—A est en position 8.

3. Un composé selon la revendication 1 ou 2, dans lequel X représente —N($R_5$)—.

4. Un composé selon l'une des revendications 1 à 3, dans lequel A représente un groupe 3-pyridyle, 4-pyridyle ou imidazole-1-yle.

5. Un composé selon l'une des revendications 1 à 4, dans lequel $R_3$ représente un groupe alkylène en $C_4$-$C_6$ et m et n sont égaux à 0.

6. Un composé selon l'une des revendications 1 à 5, dans lequel $R_1$ représente un groupe alkyle en $C_1$-$C_7$ et $R_2$ l'hydrogène.

7. Un composé selon l'une des revendications 1 à 4, dans lequel $R_1$ représente un groupe hydroxy, D un atome de carbone, D' un atome d'azote et le trait discontinu deux liaisons supplémentaires.

8. Le N-[4-(1H-imidazole-1-yl)-butyl]-2-(1-méthyléthyl)-11-oxo-11H-pyrido [2,1-b] quinazoline-8-carboxamide.

9. Le 2-(1-méthyléthyl)-N-[6-(3-pyridyl)-hexyl]-11-oxo-11H-pyrido [2,1-b] quinazoline-8-carboxamide.

10. Le 2-(1-méthyléthyl)-N-[4-(3-pyridyl)-butyl]-11-oxo-11H-pyrido [2,1-b] quinazoline-8-carboxamide.

11. Le 8-(1-méthyléthyl)-N-[4-(3-pyridyl)-butyl]-11-oxo-11H-pyrido [2,1-b] quinazoline-2-carboxamide.

12. Le 2-hydroxy-N-[4-(3-pyridyl)-butyl]-11-oxo-11H-pyrido [2,1-b] quinazoline-8-carboxamide.

13. Le 2,3-diméthyl-N-[4-(5-pyrimidinyl)-butyl]-11-oxo-11H-pyrido [2,1-b] quinazoline-8-carboxamide.

14. Le 2-(1-méthyléthyl)-N-[4-(1H-imidazole-1-yl)-1-méthylbutyl]-11-oxo-11H-pyrido [2,1-b] quinazoline-8-carboxamide et le 2,3-diméthyl-N-[4-(3-pyridyl)-butyl]-11-oxo-11H-pyrido [2,1-b] quinazoline-8-carboxamide.

15. Le 2-(1-méthyléthyl)-N-[3-(3-pyridyl)-propyl]-11-oxo-11H-pyrido [2,1-b] quinazoline-8-carboxamide,

le 2-(1-méthyléthyl)-N-[4-(3-pyridyl)-butyl]-11-oxo-11H-pyrido [2,1-b] quinazoline-6-carboxamide,

le N-[5-(1H-imidazole-1-yl)-pentyl]-2-(1-méthyléthyl)-11-oxo-11H-pyrido [2,1-b] quinazoline-8-carboxamide,

le N-[4-(4-pyridyl)-butyl]-2-(1-méthyléthyl)-11-oxo-11H-pyrido [2,1-b] quinazoline-8-carboxamide,

le 2-(1-méthyléthyl)-N-[4-(3-pyridyloxy)-butyl]-11-oxo-11H-pyrido [2,1-b] quinazoline-8-carboxamide,

le N-[2-(4-pyridylthio)-éthyl]-2-(1-méthyléthyl)-11-oxo-11H-pyrido [2,1-b] quinazoline-8-carboxamide,

le N-[5-(3-pyridyl)-pentyl]-2-(1-méthyléthyl)-11-oxo-11H-pyrido [2,1-b] quinazoline-8-carboxamide,

le N-[5-(4-pyridyl)-pentyl]-2-(1-méthyléthyl)-11-oxo-11H-pyrido [2,1-b] quinazoline-8-carboxamide,

le 2-méthoxy-N-[4-(3-pyridyl)-butyl]-11-oxo-11H-pyrido [2,1-b] quinazoline-8-carboxamide,

le 2-(1-méthyléthyl)-N-[4-(5-pyrimidinyl)-butyl]-11-oxo-11H-pyrido [2,1-b] quinazoline-8-carboxamide,

le N-[4-(2-méthyl-1H-imidazole-1-yl)-butyl]-2-(1-méthyléthyl)-11-oxo-11H-pyrido [2,1-b] quinazoline-8-carboxamide, et

le N-[4-(3-pyridyl)-butyl]-11-oxo-11H-pyrido [2,1-b] quinazoline-8-carboxamide.

16. Un composé selon l'une des revendications 1 à 15, en tant que substance active pharmaceutique.

17. Un composé selon l'une des revendications 1 à 15, en tant que substance active pour le traitement des allergies et des maladies vasculaires thrombotiques.

18. Un procédé pour la préparation d'un composé selon l'une des revendications 1 à 15, caractérisé en ce que l'on fait réagir un acide carboxylique de formule générale

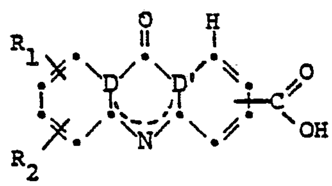

(II)

dans laquelle $R_1$, $R_2$, D, D' et le trait discontinu ont les significations indiquées dans la revendication 1, ou un dérivé réactif d'un tel acide, avec un composé de formule générale

$$W—R_3—(Y)_m—(R_4)_n—A \qquad (VI)$$

dans laquelle W représente le groupe HX— ou bien encore, dans le cas où on utilise l'acide libre de

formule II, un groupe éliminable, et $R_3$, $R_4$, A, X, Y, m et n ont les significations indiquées dans la revendication 1, et, si on le désire, on convertit un composé obtenu répondant à la formule I en un sel acceptable pour l'usage pharmaceutique par addition avec un acide.

19. Une composition pharmaceutique contenant un composé selon l'une des revendications 1 à 15.

20. Une composition pharmaceutique selon la revendication 19, pour le traitement des allergies et des maladies vasculaires thrombotiques.

**Revendications** (pour l'Etat contractant AT)

1. Procédé de préparation d'un composé de formule générale

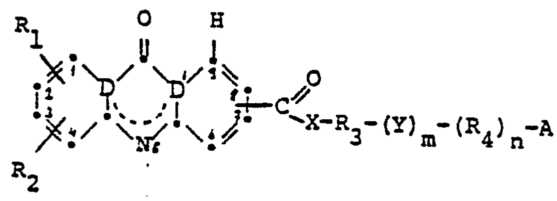

(I)

dans laquelle l'un des symboles D et D' représente un atome d'azote et l'autre un atome de carbone, le trait discontinu représente deux liaisons supplémentaires, $R_1$ et $R_2$ représentent chacun, indépendamment l'un de l'autre, l'hydrogène, un groupe alkyle en $C_1$-$C_7$, alcoxy en $C_1$-$C_7$, hydroxy ou un halogène, X représente —O— ou —$N(R_5)$—, $R_5$ représente l'hydrogène ou un groupe alkyle en $C_1$-$C_7$, $R_3$ représente un groupe alkylène en $C_2$-$C_7$, $R_4$ représente un groupe alkylène en $C_1$-$C_7$, Y représente —O— ou —S—, m est égal à 0 ou 1, n est égal à 0 ou 1, et A représente un groupe aromatique hétérocyclique à 5 ou 6 chaînons non substitué ou substitué par des groupes alkyle en $C_1$-$C_7$, alcoxy en $C_1$-$C_7$, des halogènes ou des groupes nitro, avec la restriction que m et n doivent tous deux être égaux à 0 lorsque A est relié par l'intermédiaire d'un atome d'azote hétérocyclique, ou d'un sel acceptable pour l'usage pharmaceutique, formé par addition avec un acide d'un tel composé, caractérisé en ce que l'on fait réagir un acide carboxylique de formule générale

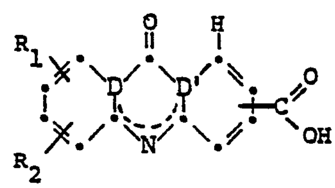

(II)

dans laquelle $R_1$, $R_2$, D, D' et le trait discontinu ont les significations indiquées ci-dessus, ou un dérivé réactif d'un tel composé, avec un composé de formule générale

$$W—R_3—(Y)_m—(R_4)_n—A \quad (VI)$$

dans laquelle W représente le groupe HX—, ou bien encore, lorsqu'on utilise l'acide libre de formule II, un groupe éliminable, et $R_3$, $R_4$, A, X, Y, m et n ont les significations indiquées ci-dessus, et, si on le désire, on convertit un composé obtenu, répondant à la formule I, en un sel acceptable pour l'usage pharmaceutique par addition avec un acide.

2. Procédé selon la revendication 1, caractérisé en ce que l'on fait réagir un composé de formule générale

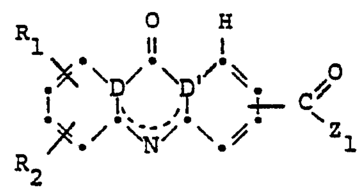

(IIIa)

dans laquelle $Z_1$ représente un atome d'halogène et $R_1$, $R_2$, D, D' et le trait discontinu ont les significations indiquées dans la revendication 1, avec un composé de formule générale

$$HX—R_3—(Y)_m—(R_4)_n—A \quad (IV)$$

dans laquelle $R_3$, $R_4$, A, X, Y, m et n ont les significations indiquées dans la revendication 1.

3. Procédé selon la revendication 1, pour préparer un composé de formule I dans laquelle X représente —N($R_5$)—, caractérisé en ce que l'on fait réagir un composé de formule II, en présence du diphénylphosphorylazide et d'un accepteur de protons, avec un composé de formule générale

$$HN(R_5)—R_3—(Y)_m—(R_4)_n—A \quad (IVa)$$

dans laquelle $R_3$, $R_4$, $R_5$, A, Y, m et n ont les significations indiquées dans la revendication 1.

4. Procédé selon la revendication 1, pour préparer un composé de formule I dans laquelle D représente un atome de carbone, D' un atome d'azote, le trait discontinu deux liaisons supplémentaires et X représente —O—, caractérisé en ce que l'on fait réagir un composé de formule générale

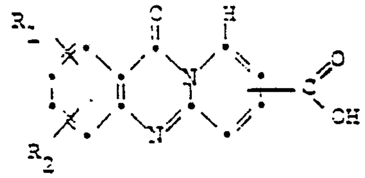

(IIa)

dans laquelle $R_1$ et $R_2$ ont les significations indiquées dans la revendication 1, en présence d'une base, avec un composé de formule générale

$$Z'—R_3—(Y)_m—(R_4)_n—A \quad (V)$$

dans laquelle $R_3$, $R_4$, A, Y, m et n ont les significations indiquées dans la revendication 1 et Z' représente un groupe éliminable.

5. Procédé selon la revendication 1, pour préparer un composé de formule I dans laquelle D représente un atome de carbone, D' un atome d'azote, le trait discontinu deux liaisons supplémentaires et X représente —N($R_5$)—, caractérisé en ce que l'on fait réagir un composé de formule générale

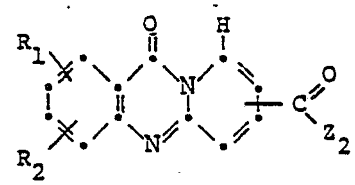

(IIIb)

dans laquelle $R_1$ et $R_2$ ont les significations indiquées dans la revendication 1, et $Z_2$ représente un groupe cyanométhoxy, phénoxy ou phénoxy substitué, avec un composé de formule IVa définie dans la revendication 3.

6. Procédé selon la revendication 1, dans lequel $R_1$ et $R_2$ sont respectivement en positions 2 et 3 et le groupe —CO—X—$R_3$—$(Y)_m$—$(R_4)_n$—A est en position 8.

7. Procédé selon la revendication 1 ou 6, dans lequel X représente —N($R_5$)—.

8. Procédé selon la revendication 1, 6 ou 7, dans lequel A représente un groupe 3-pyridyle, 4-pyridyle ou imidazole-1-yle.

9. Procédé selon l'une des revendications 1 et 6 à 8, dans lequel $R_3$ représente un groupe alkylène en $C_4$-$C_6$ et m et n sont égaux à 0.

10. Procédé selon l'une des revendications 1 et 6 à 9, dans lequel $R_1$ représente un groupe alkyle en $C_1$-$C_7$ et $R_2$ l'hydrogène.

11. Procédé selon l'une des revendications 1 et 6 à 8, dans lequel $R_1$ représente un groupe hydroxy, D un atome de carbone, D' un atome d'azote et le trait discontinu deux liaisons supplémentaires.

12. Procédé selon la revendication 1, caractérisé en ce que l'on prépare le N-[4-(1H-imidazole-1-yl)-butyl]-2-(1-méthyléthyl)-11-oxo-11H-pyrido [2,1-b] quinazoline-8-carboxamide.

13. Procédé selon la revendication 1, caractérisé en ce que l'on prépare le 2-(1-méthyléthyl)-N-[6-(3-pyridyl)-hexyl]-11-oxo-11H-pyrido [2,1-b] quinazoline-8-carboxamide.

14. Procédé selon la revendication 1, caractérisé en ce que l'on prépare le 2-(1-méthyléthyl)-N-[4-(3-pyridyl)-butyl]-11-oxo-11H-pyrido [2,1-b] quinazoline-8-carboxamide.

15. Procédé selon la revendication 1, caractérisé en ce que l'on prépare le 8-(1-méthyléthyl)-N-[4-(3-pyridyl)-butyl]-11-oxo-11H-pyrido [2,1-b] quinazoline-2-carboxamide.

16. Procédé selon la revendication 1, caractérisé en ce que l'on prépare le 2-hydroxy-N-[4-(3-pyridyl)-butyl]-11-oxo-11H-pyrido [2,1-b] quinazoline-8-carboxamide.

17. Procédé selon la revendication 1, caractérisé en ce que l'on prépare le 2,3-diméthyl-N-[4-(5-pyrimidinyl)-butyl]-11-oxo-11H-pyrido [2,1-b] quinazoline-8-carboxamide.